# EUROPÄISCHE PATENTSCHRIFT

(11) **EP 1 638 957 B1**
(45) Veröffentlichungstag und Bekanntmachung des Hinweises auf die Patenterteilung: **29.09.2010**
(21) Anmeldenummer: 04739660.1
(22) Anmeldetag: 07.06.2004
(51) Int. Cl.: C07D 403/10, C07D 491/10, C07D 495/10, C07D 513/04, C07D 405/10, C07D 493/10, C07D 231/36, C07D 487/04, C07D 498/04, C07D 491/04, C07D 231/16, C07D 231/18, A01N 43/56

(54) **N-HETEROCYCLYL-PHENYLSUBSTITUIERTE CYCLISCHE KETOENOLE**
N-HETEROCYCLYL PHENYL-SUBSTITUTED CYCLIC KETOENOLS
CETOENOLS CYCLIQUES N-HETEROCYCLYLE-PHENYLE SUBSTITUES

(30) Priorität: 12.06.2003 DE 10326386
(43) Veröffentlichungstag der Anmeldung: 29.03.2006
(73) Patentinhaber: Bayer CropScience AG, 40789 Monheim (DE)
(72) Erfinder: FISCHER, Reiner, 40789 Monheim (DE); ULLMANN, Astrid, 50677 Köln (DE); BRETSCHNEIDER, Thomas, 53797 Lohmar (DE); LEHR, Stefan, 65835 Liederbach (DE); KUNZ, Klaus, 40625 Düsseldorf (DE); KONZE, Jörg, 51147 Köln (DE); MALSAM, Olga, 51503 Rösrath (DE); DREWES, Mark, Wilhelm, 40764 Langenfeld (DE); FEUCHT, Dieter, 65760 Eschborn (DE); KUCK, Karl-Heinz, 40764 Langenfeld (DE); WACHENDORFF-NEUMANN, Ulrike, 56566 Neuwied (DE); MORADI, Wahed, Ahmed, 40789 Monheim (DE); BOJACK, Guido, 65207 Wiesbaden (DE); AULER, Thomas, 42799 Leichlingen (DE); HILLS, Martin, 65510 Idstein (DE); KEHNE, Heinz, 65719 Hofheim (DE)
(86) Internationale Anmeldenummer: PCT/EP2004/006127
(87) Internationale Veröffentlichungsnummer: WO 2004/111042

(56) Entgegenhaltungen:
- WO-A-01/09092
- WO-A-01/17973
- WO-A-99/43649
- WO-A-99/55673
- DE-A- 10 139 465
- US-A- 3 644 409
- US-B1- 6 458 965
- WILLIAMS P D ET AL: "Nonpeptide oxytocin antagonists: analogs of L-371,257 with improved potency" BIOORGANIC & MEDICINAL CHEMISTRY LETTERS, OXFORD, GB, Bd. 9, Nr. 9, 3. Mai 1999 (1999-05-03), Seiten 1311-1316, XP004163964 ISSN: 0960-894X
- NANNINI G ET AL: "NEW ANALGESIC-ANTI-INFLAMMATORY DRUGS 1-OXO-2-SUBSTITUTED ISOINDOLINE DERIVATIVES" ARZNEIMITTEL FORSCHUNG. DRUG RESEARCH, EDITIO CANTOR. AULENDORF, DE, Bd. 23, Nr. 8, 1973, Seiten 1090-1100, XP002953827 ISSN: 0004-4172
- DATABASE BEILSTEIN BEILSTEIN INSTITUTE FOR ORGANIC CHEMISTRY, FRANKFURT-MAIN, DE; XP002296804 Database accession no. BRN 347923 & BORSCHE; BAHR: JUSTUS LIEBIGS ANNALEN DER CHEMIE., Bd. 402, 1913, Seite 108,

## Beschreibung

Die vorliegende Erfindung betrifft neue N-Heterocyclyl-phenylsubstituierte cyclische Ketoenole, mehrere Verfahren zu ihrer Herstellung und ihre Verwendung als Schädlingsbekämpfungsmittel und/oder Herbizide und/oder Microbizide.

Die Erfindung betrifft außerdem neue selektiv-herbizide Wirkstoffkombinationen, die N-Heterocyclyl-phenylsubstituierte cyclische Ketoenole einerseits und zumindest eine die Kulturpflanzenverträglichkeit verbesserte Verbindung andererseits enthalten und mit besonders gutem Erfolg zur selektiven Unkrautbekämpfung mit verschiedenen Nutzpflanzenkulturen verwendet werden können.

Von 3-Acyl-pyrrolidin-2,4-dionen sind pharmazeutische Eigenschaften vorbeschrieben (S. Suzuki et al. Chem Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Arylpyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01 997, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/16748, WO 99/24437, WO 99/43649, WO 99/48869, WO 99/55673, WO 01/17972, WO 01/23354, WO 01/74770, WO 03/062244, DE-A-10 231 333 und DE-A-10 239 479).

Es ist bekannt, dass bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-A³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 95/26 345, WO 96/20 196, WO 96/25 395, WO 96/35 664, WO 97/01 535, WO 97/02 243, WO 97/36 868, WO 98/05638, WO 98/25928, WO 99/16748, WO 99/43649, WO 99/48869, WO 99/55673, WO 01/17972, WO 01/23354, WO 01/74770, WO 03/062244 und DE-A-10 239 479 bekannt. Auch 3-Aryl-Δ³-dihydrothiphen-on-Derivate sind bekannt (WO 95/26 345, 96/25 395, WO 97/01535, WO 97/02 243, WO 97/36 868, WO 98/05638, WO 98/25928, WO 99/16748, WO 99/43649, WO 99/48869, WO 99/55673, WO 01/17972, WO 01123354, WO 01/74770, und WO 03/062244).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137, WO 96/25 395, WO 96/35 664, WO 97/01535, WO 97/02 243, WO 97/16 436, WO 97/19 941, WÖ 97/36 868, WO 98/05638, WO 99/43649, WO 99/48869, WO 99/55673, WO 01/17972. WO 01/74770 und WO 03/062244 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785, WO 96/02 539, WO 96/35 664, WO 97/01 535, WO 97/02 243, WO 97/02 243, WO 97/36 868, WO 99/05638, WO 99/43649, WO 99/48869, WO 99/55673, WO 01/17972, WO 01/74770 und WO 03/062244 beschrieben.

Es ist bekannt, dass bestimmte substituierte 2-Arylcyclopentandione herbizide und akarizide Eigenschaften besitzen (vgl. z.B. US-4 283 348; 4 338 122; 4 436 666; 4 526 723; 4 551 547; 4 632 698; WO 96/01 798; WO 96/03 366, WO 97/14 667 sowie WO 98/39281, WO 99/43649, WO 99/48869, WO 99/55673, WO 01/17972, WO 01/74770 und WO 03/062244). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66, (1973), 584 und der Offenlegungsschrift DE-A 2 361084 bekannt, mit Angabe von herbiziden und akariziden Wirkungen.

Es ist bekannt, dass bestimmte substituierte 2-Arylcyclohexandione herbizide und akarizide Eigenschaften besitzen (US-4 175 135, 4 209 432, 4 256 657, 4 256 658, 4 256 659, 4 257 858, 4 283 348, 4 303 669, 4 351 666, 4 409 153, 4 436 666, 4 526 723, 4 613 617, 4 659 372, DE-A 2 813 341, sowie Wheeler, T.N., J. Org. Chem. 44, 4906 (1979)), WO 99/43649, WO 99/48869, WO 99/55673. WO 01/17972, WO 01/74770 und WO 03/062244).

Es ist bekannt, dass bestimmte substituierte 4-Aryl-pyrazolidin-3,5-dione akarizide, insektizide und herbizide Eigenschaften besitzen (vgl. z.B. WO 92/16 5 10, EP-A-508 126, WO 96/11 574, WO 96/21652, WO 99/43649, WO 99/47525, WO 99/48869, WO 99/55673 WO 01/17 351, WO 01/17 352, WO 01/17 353, WO 01/17 972, WO 01/17 973, WO 03/028446 und WO 03/062244.

WO01/09092 beschreibt biphenylsubstituierte cyclische Ketoenole. DE-A-10139465 (WO03/013249) beschreibt selektive herbizide Mittel auf Basis von substituierten cyclischen Ketoenolen und Safenern.

Die Wirksamkeit und/oder Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) gefunden 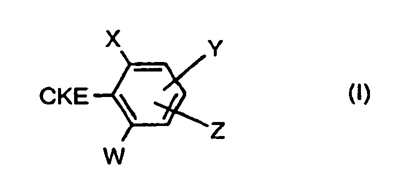 in welcher
- W: steht hervorgehoben für Wasserstoff, Methyl oder Ethyl,
- X: steht hervorgehoben für Chlor, Methyl oder Ethyl,
- Y: steht hervorgehoben für Wasserstoff,
- Z: steht hervorgehoben für den Rest in der 4- oder 5-Position 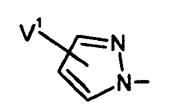
- V¹: steht hervorgehoben für Chlor oder Methoxy,
- CKE: steht hervorgehoben für eine der Gruppen 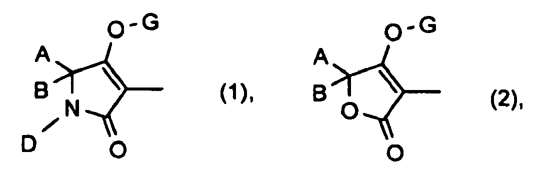 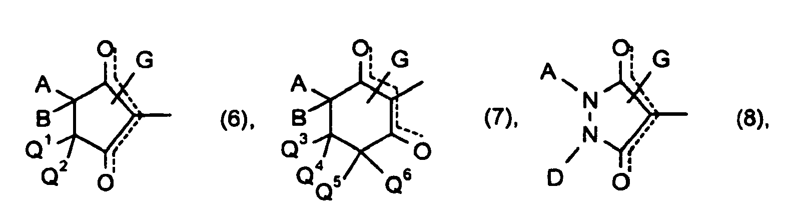
- A: steht hervorgehoben für Wasserstoff, C₁-C₄-Alkyl oder Cyclopropyl,
- B: steht hervorgehoben für Wasserstoff oder Methyl oder
- A, B: und das Kohlenstoffatom an das sie gebunden sind, stehen hervorgehoben für gesättigtes C₅-C₆-Cycloalkyl, in welchem gegebenenfalls ein Ringglied durch Sauerstoff ersetzt ist und welches gegebenenfalls einfach durch Methyl oder Methoxy substituiert ist, mit der Maßgabe, dass dann Q³ hervorgehoben für Wasserstoff steht,
- D: steht hervorgehoben für Wasserstoff,
oder
- A und D: stehen gemeinsam hervorgehoben für C₃-C₅-Alkandiyl, worin gegebenenfalls ein Kohlenstoffatom durch Sauerstoff ersetzt ist
- Q¹: steht hervorgehoben für Wasserstoff,
- Q²: steht hervorgehoben für Wasserstoff,
- Q³: steht hervorgehoben für Methyl,
- Q⁴: steht hervorgehoben für Methyl oder
- Q³ und Q⁴: stehen hervorgehoben gemeinsam mit dem Kohlenstoff, an den sie gebunden sind, für einen gesättigten C₅-C₆-Ring, mit der Maßgabe, dass dann A hervorgehoben für Wasserstoff steht,
- Q⁵: steht hervorgehoben für Wasserstoff,
- Q⁶: steht hervorgehoben für Wasserstoff,
- G: steht hervorgehoben für Wasserstoff (a) oder für eine der Gruppen 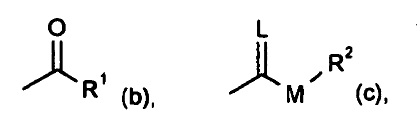 in welchen
L für Sauerstoff steht und
M für Sauerstoff oder Schwefel steht.
- R¹: steht hervorgehoben für C₁-C₆-Alkyl oder C₁-C₂-Alkoxy-C₁-alkyl,
- R²: steht hervorgehoben für C ₁-C₈-Alkyl oder Benzyl.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1), (2) und (6) bis (8) der Gruppe CKE ergeben sich folgende hauptsächliche Strukturen (I-1), (I-2) und (I-6) bis (I-8): 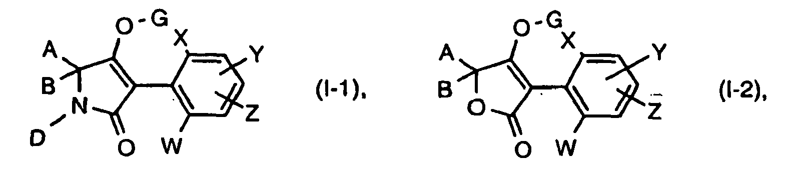 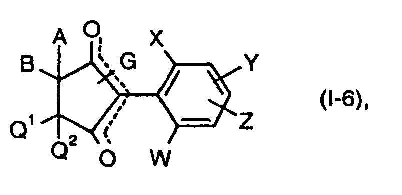 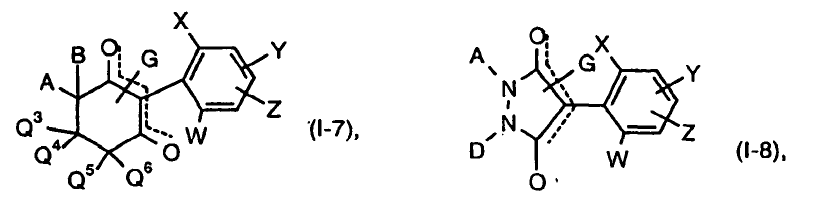 worin
A, B, D, G, Q¹, Q², Q³, Q⁴, Q⁵, Q⁶, W, X, Y und Z die oben angegebene Bedeutung haben.

Unter Einbeziehung der verschiedenen Bedeutungen (a), (b), (c), der Gruppe G ergeben sich folgende hauptsächliche Strukturen (I-1-a) bis (I-1-c), wenn CKE für die Gruppe (1) steht, 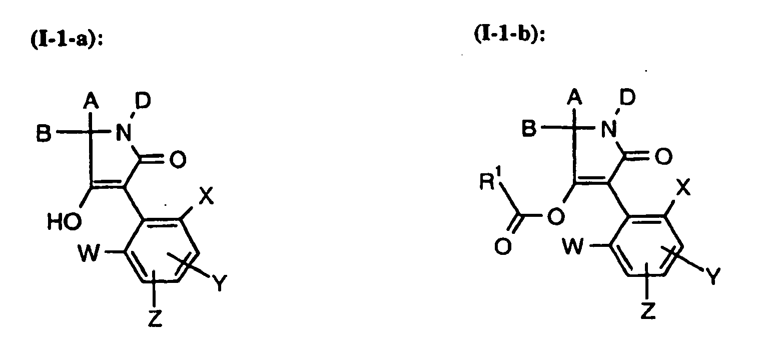 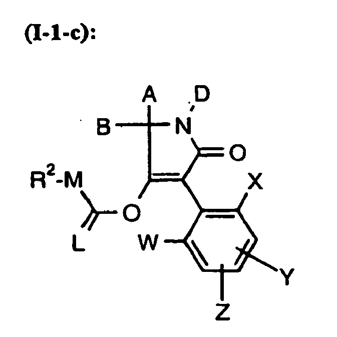 worin
A, B, D, E, L, M, W, X, Y, Z, R¹, R², die oben angegebenen Bedeutungen besitzen.

Unter Einbeziehung der verschiedenen Bedeutungen (a), (b), (c), der Gruppe G ergeben sich folgende hauptsächliche Strukturen (I-2-a) bis (I-2-c), wenn CKE für die Gruppe (2) steht, 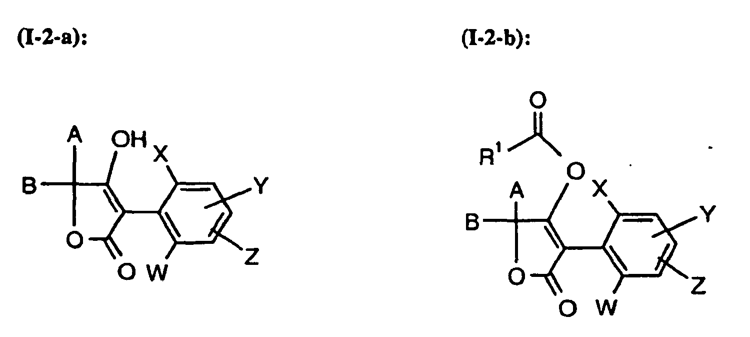 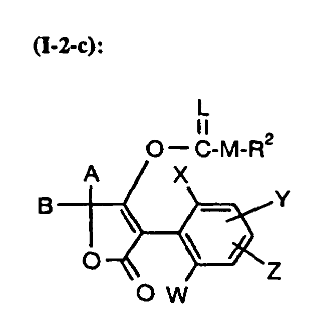 worin
A, B, E, L, M, W, X, Y, Z, R¹, R², die oben angegebene Bedeutung haben.

Die Verbindungen der Formel (I-6) können in Abhängigkeit von der Stellung des Substituenten G in den zwei isomeren Formen der Formeln (I-6-A) und (I-6-B) vorliegen, 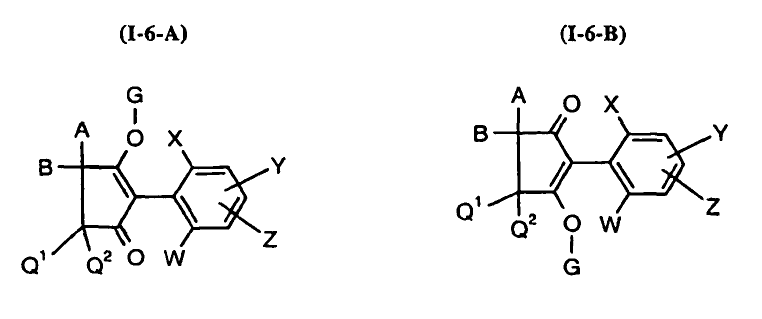 was durch die gestrichelte Linie in der Formel (I) zum Ausdruck gebracht werden soll.

Die Verbindungen der Formeln (I-6-A) und (I-6-B) können sowohl als Gemische als auch in Form ihrer reinen Isomeren vorliegen. Gemische der Verbindungen der Formeln (I-6-A) und (I-6-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), der Gruppe G ergeben sich folgende hauptsächlichen Strukturen (I-6-a) bis (I-6-c): 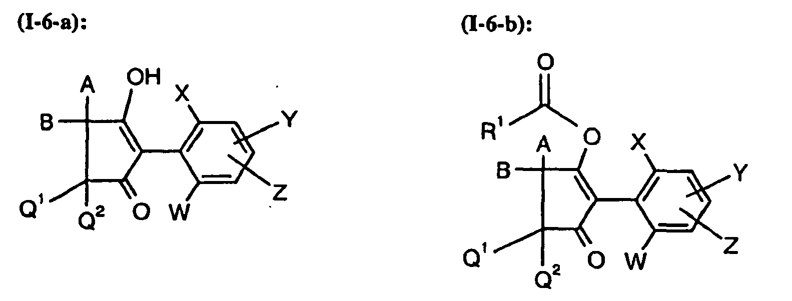 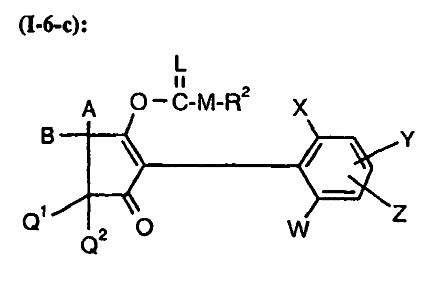 worin
A, B, Q¹, Q², E, L, M, W, X, Y, Z, R¹, R², die oben angegebenen Bedeutungen besitzen.

Die Verbindungen der Formel (I-7) können in Abhängigkeit von der Stellung des Substituenten G in den zwei isomeren Formen der Formeln (I-7-A) bzw. (1-7-B) vorliegen, was durch die gestrichelte Linie in der Formel (1-7) zum Ausdruck gebracht werden soll:

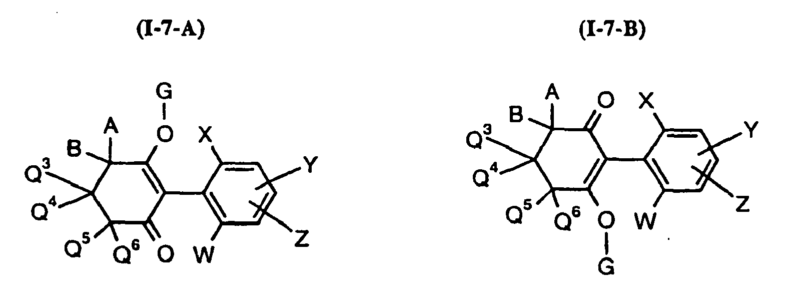

Die Verbindungen der Formeln (I-7-A) bzw. (I-7-B) können sowohl als Gemische als auch in Form ihrer reinen Isomeren vorliegen. Gemische der Verbindungen der Formeln (I-7-A) und (I-7-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt ein, dass die betreffenden Verbindung gegebenenfalls als Isomerengemisch oder in der jeweils anderen isomeren Form vorliegen kann.

Unter Einbeziehung der verschiedenen Bedeutungen (a), (b), (c), der Gruppe G ergeben sich folgende hauptsächlichen Strukturen (I-7-a) bis (I-7-c): 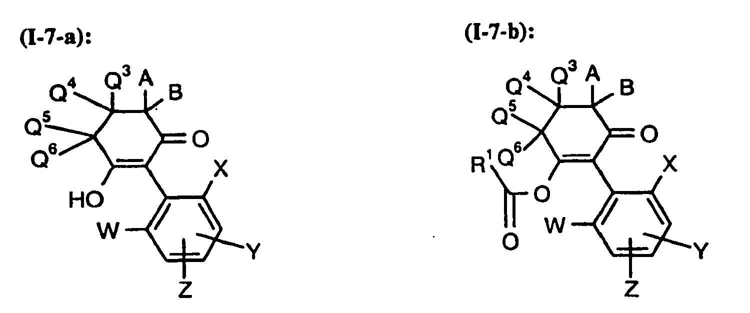 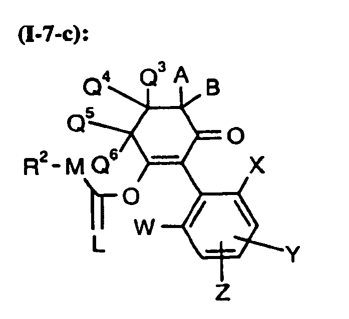 worin
A, B, E, L, M, Q³, Q⁴, Q⁵, Q⁶, W, X, Y, Z, R¹, R², die oben angegebenen Bedeutungen besitzen.

Die Verbindungen der Formel (I-8) können in Abhängigkeit von der Stellung des Substituenten G in den zwei isomeren Formeln (I-8-A) und (I-8-B) vorliegen, 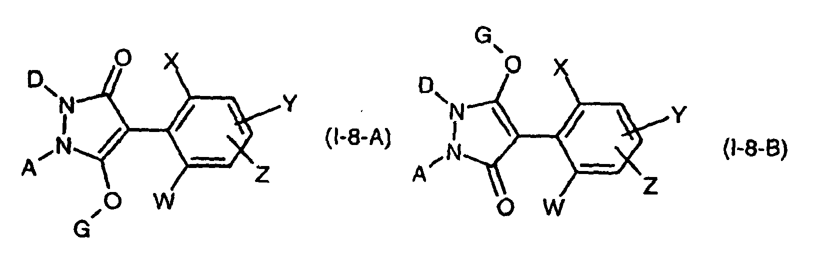 was durch die gestrichelte Linie in der Formel (I-8) zum Ausdruck gebracht werden soll.

Die Verbindungen der Formeln (I-8-A) und (I-8-B) können sowohl als Gemische als auch in Form ihrer reinen Isomeren vorliegen. Gemische der Verbindungen der Formel (I-8-A) und (I-8-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), der Gruppe G ergeben sich folgende hauptsächliche Strukturen (I-8-a) (bis (I-8-c), wenn Het für die Gruppe (8) steht, 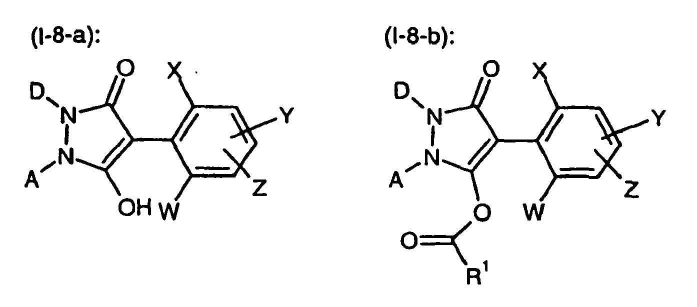 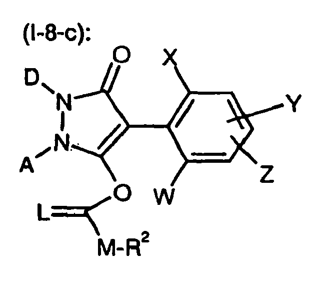 worin
A, D, E, L, M, W, X, Y, Z, R¹, R², die oben angegebenen Bedeutungen besitzen.

Weiterhin wurde gefunden, dass man die neuen Verbindungen der Formel (I) nach einem der im folgenden beschriebenen Verfahren erhält:
(A) Man erhält substituierte 3-(N-Heterocyclyl)-phenylpyrrolidin-2,4-dione bzw. deren Enole der Formel (I-1-a) 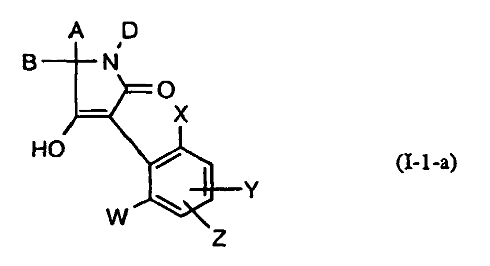 in welcher
   A, B, D, W, X, Y und Z die oben angegebenen Bedeutungen haben,
   wenn man
   N-Acylaminosäureester der Formel (II) 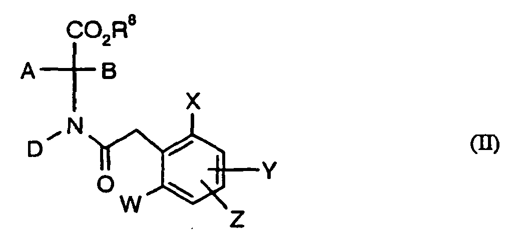 in welcher
   A, B, D, W, X, Y und Z die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, dass man substituierte 3-(N-Heterocyclyl)-phenyl-4-hydroxy-Δ³-dihydrofuranon-Derivate der Formel (I-2-a) 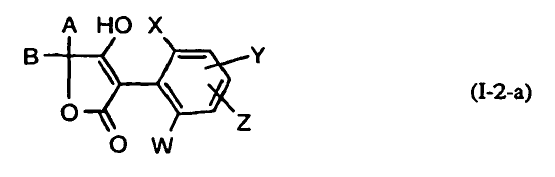 in welcher
   A, B, W, X, Y und Z die oben angegebenen Bedeutungen haben,
   erhält, wenn man
   Carbonsäureester der Formel (III) 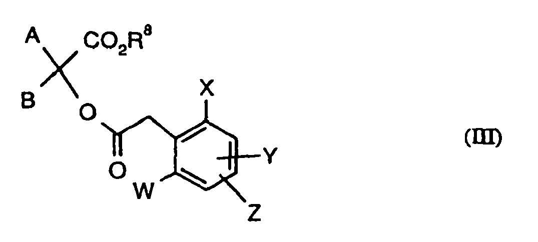 in welcher
   A, B, W, X , Y, Z und R⁸ die oben angegebenen Bedeutungen haben,
   in Gegenwart eines Verdünnungsmittels und in Gegenwart einer Base intramolekular kondensiert.
   Weiterhin wurde gefunden,
(F) dass man Verbindungen der Formel (I-6-a) 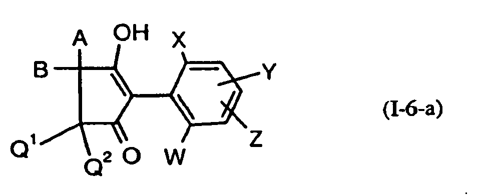 in welcher
   A, B,Q¹, Q², W, X, Y und Z die oben angegebene Bedeutung haben,
   erhält, wenn man
   Ketocarbonsäureester der Formel (VIII) 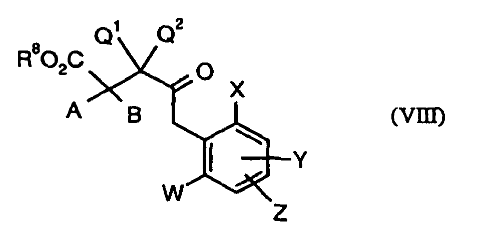 in welcher
   - A, B, Q1, Q², W, X, Y und Z: die oben angegebene Bedeutung haben, und
   - R⁸: für Alkyl (insbesondere C₁-C₈-Alkyl) steht,
   gegebenenfalls in Gegenwart eines Verdünnungsmittels und in Gegenwart einer Base intramolekular cyclisiert.
   Außerdem wurde gefunden,
(G) dass man Verbindungen der Formel (I-7-a) 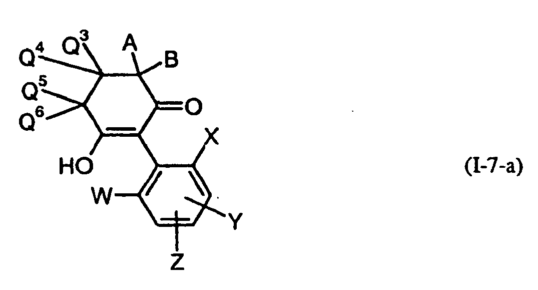 in welcher
   A, B, Q³, Q⁴, Q⁵, Q⁶, W, X, Y und Z die oben angegebene Bedeutung haben,
   erhält, wenn man
   6-Aryl-5-keto-hexansäureester der Formel (IX) 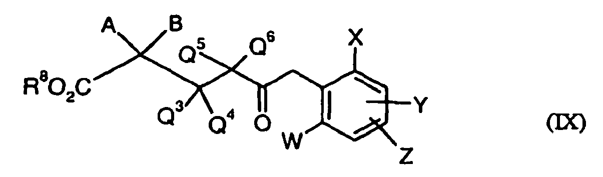 in welcher
   - A, B, Q³, Q⁴, Q⁵, Q⁶, W, X, Y und Z: oben angegebene Bedeutung haben und
   - R⁸: für Alkyl (bevorzugt C₁-C₆-Alkyl) steht,
   in Gegenwart eines Verdünnungsmittels und in Gegenwart einer Base intramolekular kondensiert.
(H) Weiterhin wurde gefunden, dass man die Verbindungen der Formel (I-8-a) 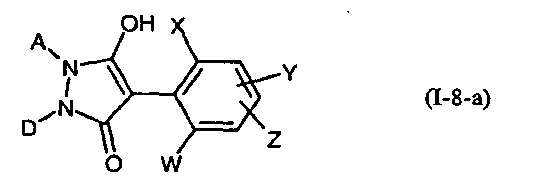 in welcher
   A, D, W, X, Y und Z die oben angegebenen Bedeutungen haben,
   erhält, wenn man
   Verbindungen der Formel (X) 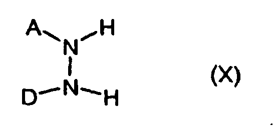 in welcher
   - A und D: die oben angegebene Bedeutung haben,

   α) mit Verbindungen der Formel (VI) 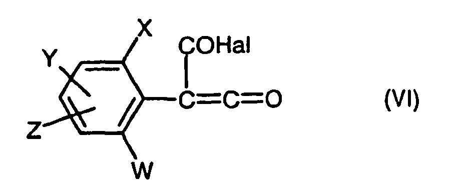 in welcher
      Hal, W, X, Y und Z die oben angegebenen Bedeutungen haben,
      gegebenenfalls in Gegenwart eines Verdünnungsmittels und gegebenenfalls in Gegenwart eines Säureakzeptors umsetzt, oder
   β) mit Verbindungen der Formel (XI) 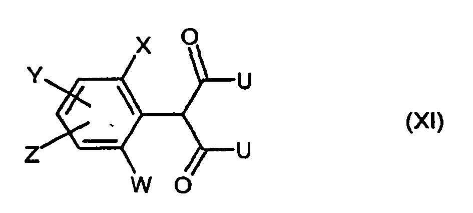 in welcher
      W, X, Y und Z die oben angegebene Bedeutung haben,
      und U für NH₂ oder O-R⁸ steht,
      wobei R⁸ die oben angegebene Bedeutung hat,
      gegebenenfalls in Gegenwart eines Verdünnungsmittels und gegebenenfalls in Gegenwart einer Base umsetzt, oder
   γ) Verbindungen der Formel (XII) 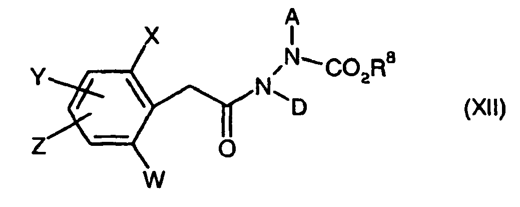 in welcher
      - A, D, W, X, Y, Z und R⁸: die oben angegebene Bedeutung haben,
      gegebenenfalls in Gegenwart eines Verdünnungsmittels und gegebenenfalls in Gegenwart einer Base umsetzt.
      Außerdem wurde gefunden
      (I) dass man die Verbindungen der oben gezeigten Formeln (I-1-b) bis (I-8-b), in welchen A, B, D, Q¹, Q², Q³, Q⁴, Q⁵, Q⁶, R¹, W, X, Y und Z die oben angegebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
         (α) mit Säurehalogeniden der Formel (XIII) 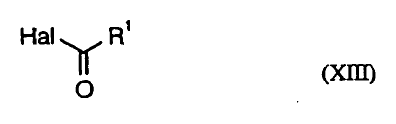 in welcher
            - R¹: die oben angegebene Bedeutung hat und
            - Hal: für Halogen (insbesondere Chlor oder Brom) steht
            oder
         (β) mit Carbonsäureanhydriden der Formel (XIV)

            R¹-CO-O-CO-R¹ (XIV)

            in welcher
            - R¹: die oben angegebene Bedeutung hat,
            gegebenenfalls in Gegenwart eines Verdünnungsmittels und gegebenenfalls in Gegenwart eines Säurebindemittels umsetzt;
(J) dass man die Verbindungen der oben gezeigten Formeln (I-1-c) bis (I-8-c), in welchen A, B, D, Q¹, Q², Q³, Q⁴, Q⁵, Q⁶, R², M, W, X, Y und Z die oben angegebenen Bedeutungen haben und L für Sauerstoff steht, erhält, wenn man Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
   mit Chlorameisensäureestem oder Chlorameisensäurethioestern der Formel (XV)

   R²-M-CO-Cl (XV)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(K) dass man Verbindungen der oben gezeigten Formeln (I-1-c) bis (1-8-c), in welchen A, B, D, Q¹, Q², Q³, Q⁴, Q⁵, Q⁶, R², M, W, X, Y und Z die oben angegebenen Bedeutungen haben und L für Schwefel steht, erhält, wenn man Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
   mit Chlormonothioameisensäureestern oder Chlordithioameisensäureestern der Formel (XVI) 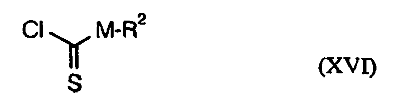 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt
(P) dass man Verbindungen der oben gezeigten Formeln (I-1) bis (I-8), in welchen A, B, D, Q¹, Q², Q³ Q⁴, Q⁵, Q⁶, W, X, Y und Z die oben angegebene Bedeutung haben, erhält, wenn man Verbindungen der Formel (I-1'bis (1-8'), - 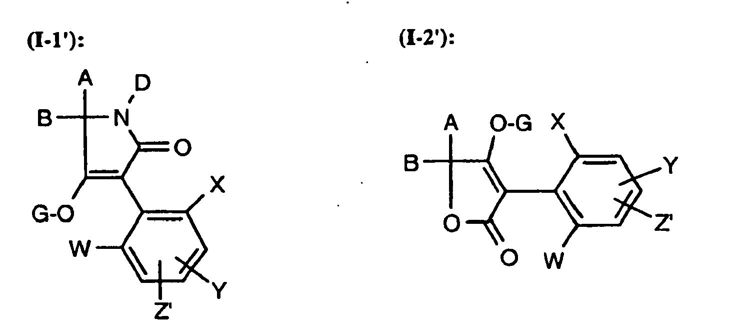 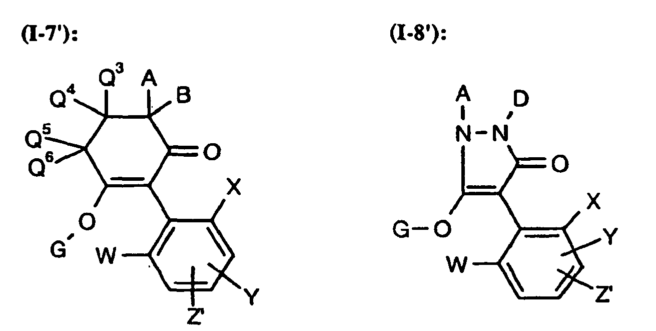 in welchen
   - A, B, D, G, Q¹, Q², Q³, Q⁴, Q⁵, Q⁶, W, X und Y: die oben angegebene Bedeutung haben und
   - Z': für Chlor, Brom, Jod, bevorzugt für Brom steht,
   mit NH-Heterocyclen der Formel (XXIII)

   H-Z (XXIII)

   in welcher
   - Z: die oben angegebene Bedeutung hat,
   in Gegenwart eines Lösungsmittels, einer Base und eines Katalysators umsetzt, wobei als Katalysator insbesondere Kupfer-I-Salze in Frage kommen.

Weiterhin wurde gefunden, dass die neuen Verbindungen der Formel (I) eine sehr gute Wirksamkeit als Schädlingsbekämpfungsmittel, vorzugsweise als Insektizide und/oder Akarizide und/oder Herbizide und/oder Fungizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
(a') mindestens ein substituiertes, cyclisches Ketoenol der Formel (I), in welcher CKE, W, X, Y und Z die oben angegebene Bedeutung haben
   und
(b') zumindest eine die Kulturpflanzen-Verträglichkeit verbessernde Verbindung aus der folgenden Gruppe von Verbindungen: Cloquinocet-mexyl oder Mefenpyr-diethyl

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß hervorgehoben werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im Einzelnen seien außer den bei den Herstellungsbeispielen genannten Verbindungen die folgenden Verbindungen der Formel (I-1-a) genannt:

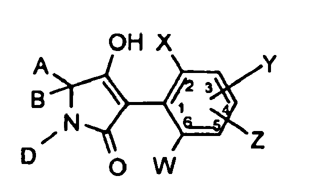

**Tabelle 1:**

| | | |
|---|---|---|
| W = CH₃, X = CH₃₂ Y = H, 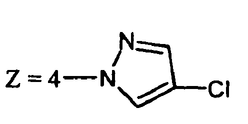 | | |

| **A** | **B** | **D** |
|---|---|---|
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| s-C₄H₉ | CH₃ | H |
| t-C₄H₉ | CH₃ | H |
| C₂H₅ | C₂H₅ | H |
| C₃H₇ | C₃H₇ | H |
| 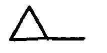 | CH₃ | H |
| 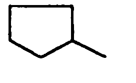 | CH₃ | H |
| 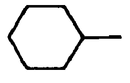 | CH₃ | H |
| -(CH2)2- | | H |
| -(CH2)4- | | H |
| -(CH₂)₅- | | H |
| -(CH2)6- | | H |
| -(CH2)7- | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHOC₄H₉-(CH₂)₂- | | H |
| -(CH₂)₂-C(CH₃)₂-(CH₂)₂- | | H |
| -CH₂-(CHCH₃)₂-(CH₂)₂- | | H |
| 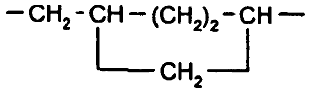 | | H |
| 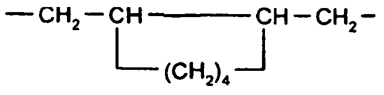 | | H |
| 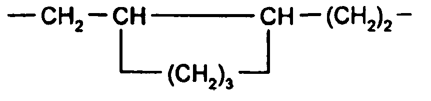 | | H |
| 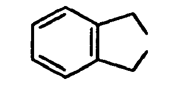 | | H |
| 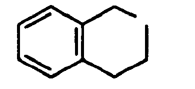 | | H |
| | | |
| **A** | **D** | **B** |
| -(CH₂)₃- | | H |
| -(CH2)4- | | H |
| -CH₂-CHCH₃-CH₂- | | H |
| -CH₂-CH₂-CHCH₃- | | H |
| -CH₂-CHCH₃-CHCH₃- | | H |
| -CH₂-S-CH₂- | | H |
| -CH₂-S-(CH₂)₂- | | H |
| -(CH₂)₂-S-CH₂- | | H |
| 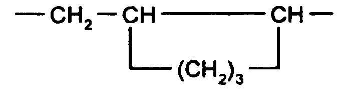 | | H |
| H | CH₃ | H |
| H | C₂H₅ | H |
| H | C₃H₇ | H |
| H | i-C₃H₇ | H |
| H | 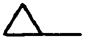 | H |
| H | 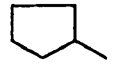 | H |
| H | 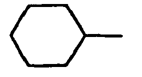 | H |
| CH3 | CH3 | H |
| CH₃ | C₂H₅ | H |
| CH₃ | C₃H₇ | H |
| CH₃ | i-C₃H₇ | H |
| CH₃ | 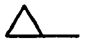 | H |
| CH₃ | 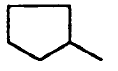 | H |
| CH₃ | 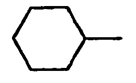 | H |
| C₂H₅ | CH₃ | H |
| C₂H₅ | C₂H₅ | H |

**Tabelle 2: A, B und D wie in Tabelle 1 angegeben**

| |
|---|
| W = CH₃, X = C₂H₅, Y = H, 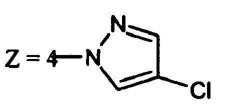 |

**Tabelle 3: A, B und D wie in Tabelle 1 angegeben**

| | |
|---|---|
| W =CH₃, X= Cl, Y=H, | 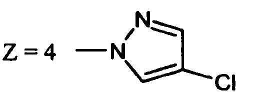 |

**Tabelle 4: A, B und D wie in Tabelle 1 angegeben**

| | |
|---|---|
| W = C₂H₅, X = C₂H₅, Y = H, | 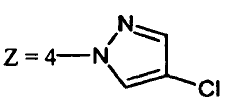 |

**Tabelle 5: A, B und D wie in Tabelle 1 angegeben**

| | |
|---|---|
| W = C₂H₅, X = Cl, Y = H, | 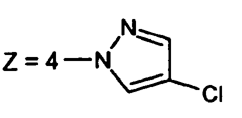 |

**Tabelle 6: A, B und D wie in Tabelle 1 angegeben**

| | |
|---|---|
| W=H,X=CH₃, Y=H, | 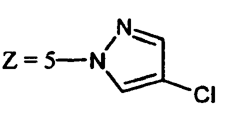 |

**Tabelle 7: A, B und D wie in Tabelle 1 angegeben**

| | |
|---|---|
| W = H, X = Cl, Y = H, | 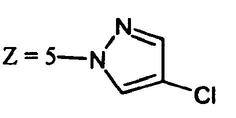 |

Im Einzelnen seien außer den bei den Herstellungsbeispielen genannten Verbindungen die folgenden Verbindungen der Formel (I-2-a) genannt:

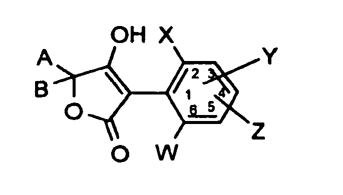

**Tabelle 8:**

| | |
|---|---|
| W=CH₃,X=CH₃,Y=H, 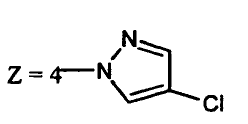 | |

| **A** | **B** |
|---|---|
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| C₃H₇ | C₃H₇ |
| 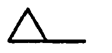 | CH₃ |
| 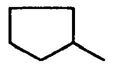 | CH₃ |
| 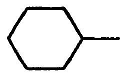 | CH₃ |
| -(CH₂)₂- | |
| -(CH₂)₄- | |
| -(CH₂)₅- | |
| -(CH₂)₆- | |
| -(CH2)7- | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -(CH₂)₂-CHCH₃-(CH₂)₂- | |
| (CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHOC₄H₉-(CH₂)₂- | |
| -(CH₂)₂-C(CH₃)₂-(CH₂)₂- | |
| -CH₂-(CHCH₃)₂-(CH₂)₂- | |
| 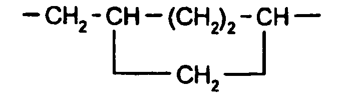 | |
| 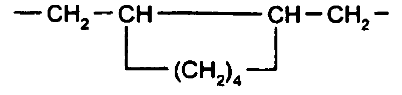 | |
| 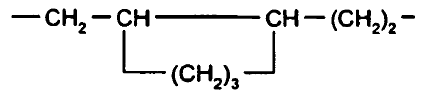 | |
| 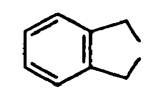 | |
| 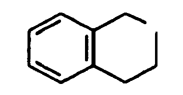 | |

**Tabelle 9: A und B wie in Tabelle 8 angegeben**

| | |
|---|---|
| W = CH₃, X = C₂H₅, Y = H, | 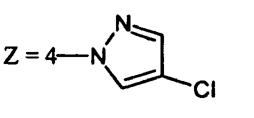 |

**Tabelle 10: A und B wie in Tabelle 8 angegeben**

| | |
|---|---|
| W=CH₃, X=Cl, Y=H, | 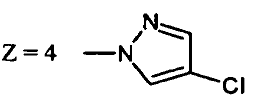 |

**Tabelle 11: A und B wie in Tabelle 8 angegeben**

| | |
|---|---|
| W = C₂H₅, X = C₂H₅, Y = H, | 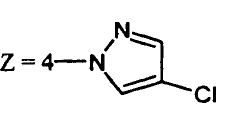 |

**Tabelle 12: A und B wie in Tabelle 8 angegeben**

| | |
|---|---|
| W = C₂H₅, X = Cl, Y = H, | 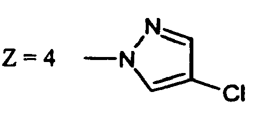 |

**Tabelle 13: A und B wie in Tabelle 8 angegeben**

| | |
|---|---|
| W=H,X=CH₃, Y=H, | 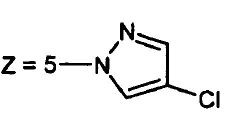 |

**Tabelle 14: A und B wie in Tabelle 8 angegeben**

| | |
|---|---|
| W= H, X = Cl, Y = H, | 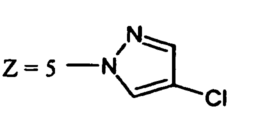 |

Im einzelnen seien außer bei den Herstellungsbeispielen genannten Verbindungen die folgenden Verbindungen der Formel (I-8-a) genannt:

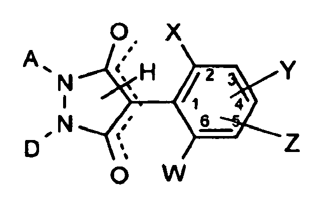

**Tabelle 15: W, X, Y und Z wie in Tabelle 1 angegeben.**

| **A** | **D** |
|---|---|
| CH₃ | CH₃ |
| CH₃ | -(CH₂)₂OH- |
| CH₃ | -(CH₂)₂OCH₃- |
| CH₃ | -(CH₂)₂-O-(CH₂)₂-OCH₃- |
| -(CH₂)₂-O-CH₃- | -(CH₂)₂-O-CH₃- |
| -(CH₂)₂-O-(CH₂)₂-OCH₃- | -(CH₂)₂-O-(CH₂)₂-OCH₃- |
| -(CH₂)₃- | |
| -(CH₂)₄- | |
| -(CH₂)₂-O-(CH₂)₂- | |
| 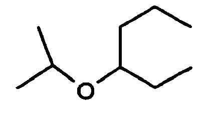 | |
| 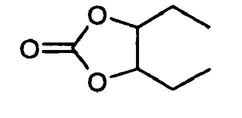 | |
| 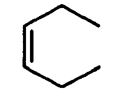 | |
| 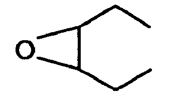 | |
| 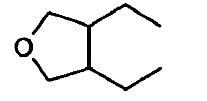 | |
| 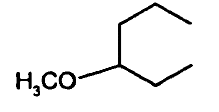 | |
| 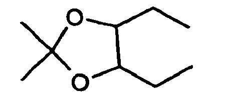 | |
| 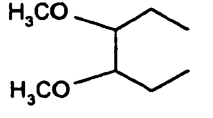 | |
| 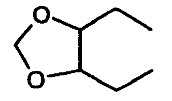 | |
| 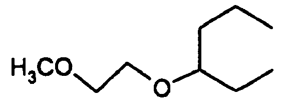 | |

**Tabelle 16: A und D wie in Tabelle 15 angegeben**

| |
|---|
| W, X, Y und Z wie in Tabelle 2 angegeben. |

**Tabelle 17: A und D wie in Tabelle 15 angegeben**

| |
|---|
| W, X, Y und Z wie in Tabelle 3 angegeben. |

**Tabelle 18: A und D wie in Tabelle 15 angegeben**

| |
|---|
| W, X, Y und Z wie in Tabelle 4 angegeben. |

**Tabelle 19: A und D wie in Tabelle 15 angegeben**

| |
|---|
| W, X, Y und Z wie in Tabelle 5 angegeben. |

**Tabelle 20: A und D wie in Tabelle 15 angegeben**

| |
|---|
| W, X, Y und Z wie in Tabelle 6 angegeben. |

**Tabelle 21: A und D wie in Tabelle 15 angegeben**

| |
|---|
| W, X, Y und Z wie in Tabelle 7 angegeben. |

| **Beispiel-** **Nr.** | **R²⁹** | **R³²** | **R³³** | **(Positionen)** **(X⁴)ₙ** | **(Positionen)** **(X⁵)ₙ** |
|---|---|---|---|---|---|
| IIe-10 | H | H | C₃H₇-i | (2) OCH₃ | - |
| | | | | (5) CH₃ | |
| IIe-11 | H | H | 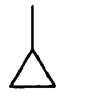 | (2) OCH₃ | - |
| | | | | (5) CH₃ | |
| IIe-12 | H | CH₃ | CH₃ | (2) OCH₃ | - |
| | | | | (5) CH₃ | |

Als die die Kulturpflanzen-Verträglichkeit verbessernde Verbindung [Komponente (b')] sind Cloquintocet-mexyl, Mefenpyr-diethyl, Cloquintocet-mexyl und Mefenpyr-diethyl besonders hervorgehoben seien.

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

**Tabelle Beispiele für die erfindungsgemäßen Kombinationen**

| **Wirkstoffe der Formel (I)** | **Safener** |
|---|---|
| I-1 | Cloquintocet-mexyl |
| I-1 | Cloquintocet-mexyl |
| I-2 | Cloquintocet-mexyl |
| I-2 | Mefenpyr-diethyl |
| 1-6 | Cloquintocet-mexyl |
| 1-7 | Cloquintocet-mexyl |
| I-7 | Mefenpyr-diethyl |
| I-8 | Mefenpyr-diethyl |

Es wurde nun überraschend gefunden, dass die oben definierten Wirkstoffkombinationen aus N-Heterocyclyl-phenyl substituierten cyclischen Ketoenole der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von N-Heterocyclyl-phenyl substituierten cyclischen Ketoenolen auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Verwendet man beispielsweise gemäß Verfahren (A) N-[6-Methyl-3-(N-4-chlorpyrazolyl)-phenylacetyl]-1-amino-cyclohexan-carbonsäureethylester als Ausgangsstoff, so kann der Verlauf des erfindungsgemäßen Verfahrens durch folgendes Reaktionsschema wiedergegeben werden:

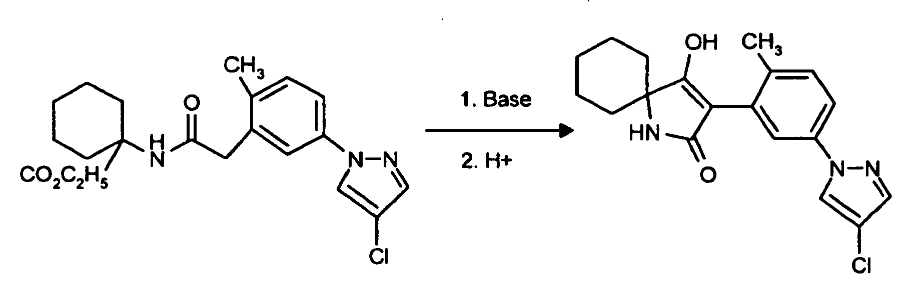

Verwendet man beispielsweise gemäß Verfahren (B) O-[2-Chlor-5-(N-4-chlorpyrazolyl)-phenylacetyl]-2-hydroxyisobuttersäureethylester, so kann der Verlauf des erfindungsgemäßen Verfahrens durch folgendes Reaktionsschema wiedergegeben werden:

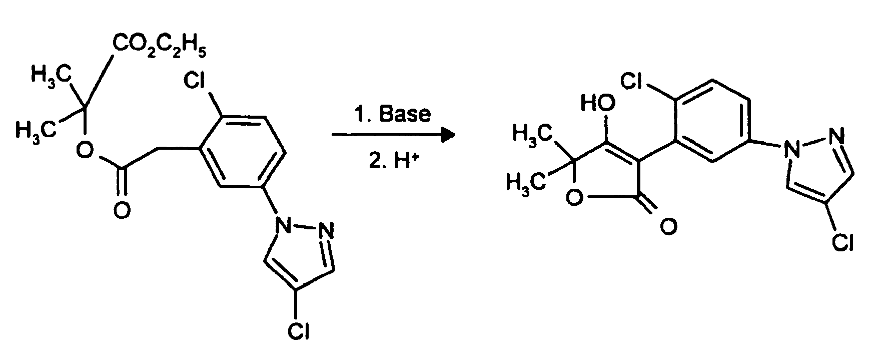

Verwendet man beispielsweise gemäß Verfahren (F) 5-[2,6-Dimethyl-(N-4-chlorpyrazolylphenyl)]-2,3-tetramethylen-4-oxo-valeriansäureethylester, so kann der Verlauf des erfindungsgemäßen Verfahrens durch folgendes Reaktionsschema wiedergegeben werden:

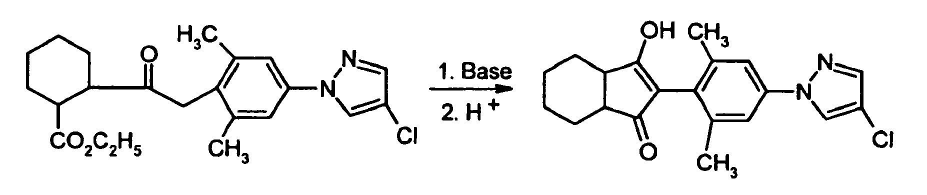

Verwendet man beispielsweise gemäß Verfahren (G) 5-[2,6-Dimethyl-4-(N-4-chlorpyrazolyl)-phenyl]-2,2-dimethyl-5-oxo-hexansäure-ethylester, so kann der Verlauf des erfindungsgemäßen Verfahrens durch folgendes Reaktionsschema wiedergegeben werden:

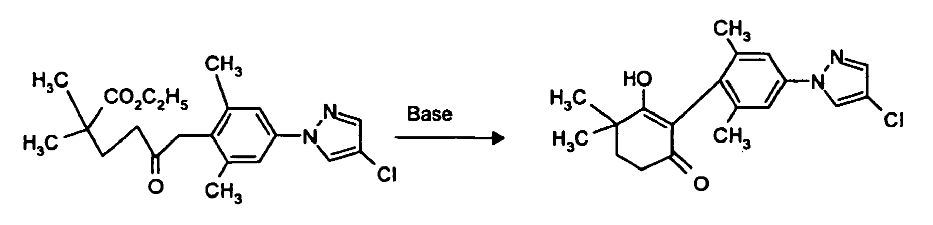

Verwendet man beispielsweise gemäß Verfahren (Hα) Hexahydropyridazin und (Chlorcarbonyl)-2-[2,6-dimethyl-4-(N-4-chlorpyrazolyl)-phenyl]-keten als Ausgangsverbindungen, so kann der Reaktionsverlauf des erfindungsgemäßen Verfahrens durch folgendes Reaktionsschema wiedergegeben werden:

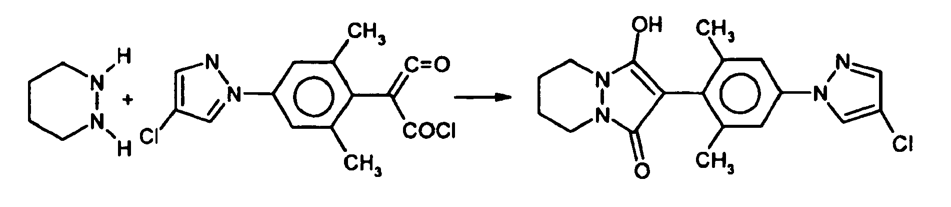

Verwendet man beispielsweise gemäß Verfahren (Hβ) Hexahydropyridazin und 2,6-Dimethyl-4-(N-4-chlorpyrazolyl)-phenylmalonsäuredimethylester als Ausgangsprodukte, so kann der Verlauf des erfindungsgemäßen Verfahrens durch folgendes Reaktionsschema wiedergegeben werden:

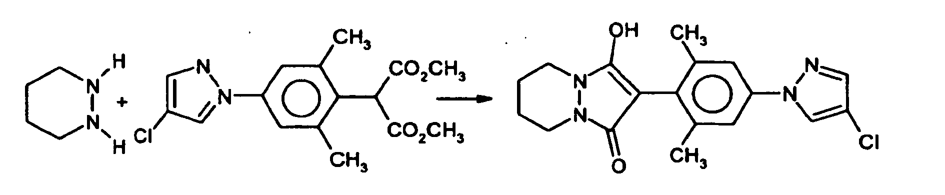

Verwendet man beispielsweise gemäß Verfahren (Hγ) 1-Ethoxycarbonyl-2-[2,6-dimethyl-4-(N-4-chlorpyrazolyl)-phenylacetyl]-hexahydropyridazin als Ausgangsprodukt, so kann der Reaktionsverlauf durch folgendes Schema wiedergegeben werden:

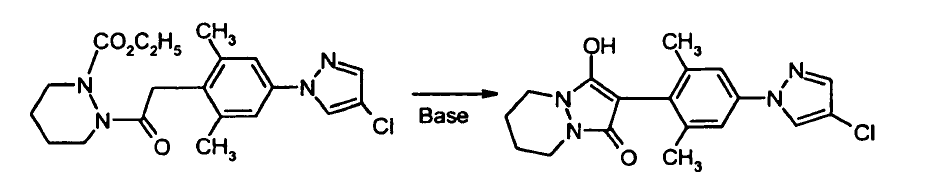

Verwendet man beispielsweise gemäß Verfahren (Iα) 3-[2,6-Dimethyl-4-(N-4-chlorpyrazolyl-phenyl)-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

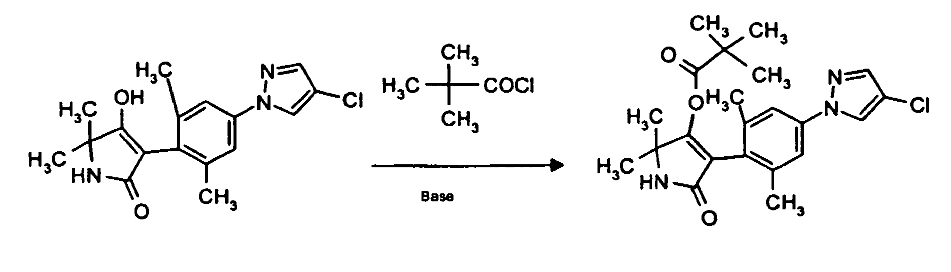

Verwendet man beispielsweise gemäß Verfahren (I) (Variante β) 3-[2,6-Dimethyl-4-(N-4-chlor-pyrazolyl-)-phenyl-)-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

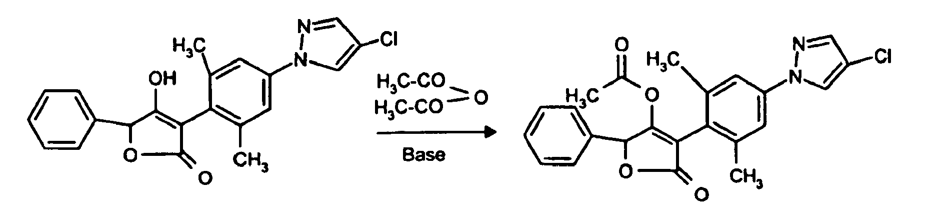

Verwendet man beispielsweise gemäß Verfahren (J) 8-[2,6-Dimethyl-4-(N-4-chlorpyrazolyl)-phenyl]-1,6-diaza-bicydo-(4,3,0^{1,6})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden:

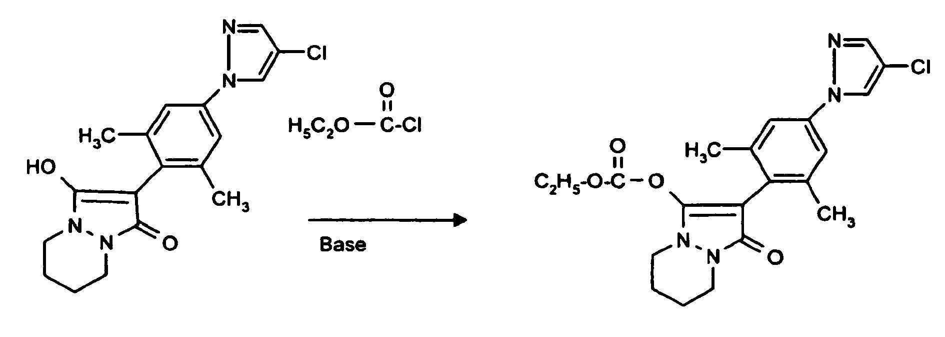

Verwendet man beispielsweise gemäß Verfahren (K), 3-[2,6-Dimethyl-4-(N-4-chlorpyrazolyl)-phenyl]-4-hydroxy-5-methyl-6-(3-pyridyl)-pyron und Chlormonothioameisensäuremethylester als Ausgangsprodukte, so kann der Reaktionsverlauf folgendermaßen wiedergegeben werden:

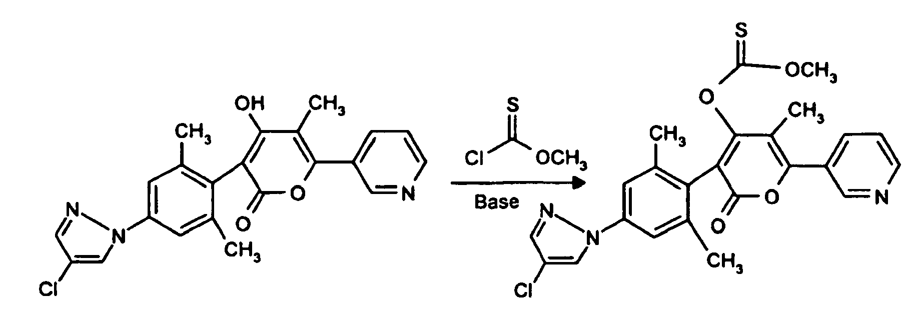

Verwendet man beispielsweise gemäß Verfahren (P) 3-[2,6-Dimethyl-4-brom)-phenyl]-4,4-(pentamethylen)-pyrrolidin-2,4-dion und 4-Chlorpyrazol als Ausgangsprodukte, so kann der Reaktionsverlauf durch folgendes Schema wiedergegeben werden:

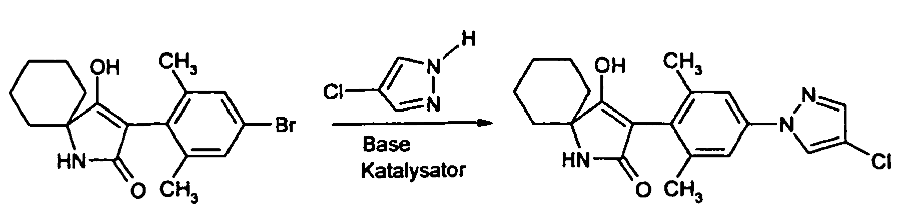

Die beim erfindungsgemäßen Verfahren (a) als Ausgangsstoffe benötigten Verbindungen der Formel (II) 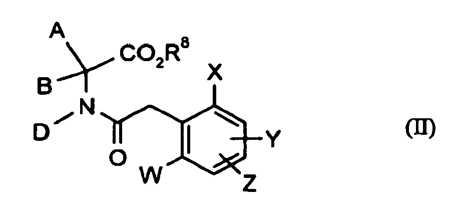 in welcher
- A, B, D, W, X, Y, Z und R⁸: die oben angegebenen Bedeutungen haben,
sind neu.

Man erhält die Acylaminosäureester der Formel (II) beispielsweise, wenn man Aminosäurederivate der Formel (XXIV) 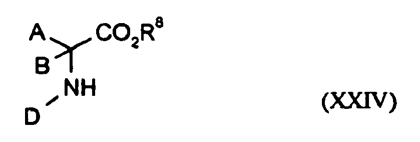 in welcher
A, B, R⁸ und D die oben angegebenen Bedeutungen haben,
mit substituierten Phenylessigsäurederivaten Formel (XXV) 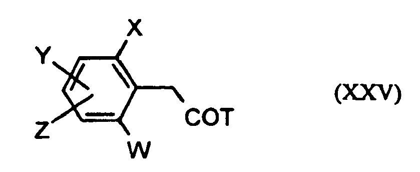 in welcher
- W, X, Y und Z: die oben angegebenen Bedeutungen haben und
- T: für eine durch Carbonsäureaktivierungsreagenzien wie Carbonyldiimidazol, Carbonyldiimide (wie z.B. Dicyclohexylcarbondiimid), Phosphorylierungsreagenzen (wie z.B. POCl₃, BOP-Cl), Halogenierungsmittel z.B. Thionylchlorid, Oxalyl- chlorid, Phosgen, Sulfonsäurechloride (z.B. Toluolsulfonsäurechlorid) oder Chlor- ameisensäureester eingeführte Abgangsgruppe steht,
acyliert (Chem. Reviews 52, 237-416 (1953); Bhattacharya, Indian J. Chem. 6, 341-5, 1968) oder wenn man Acylaminosäuren der Formel (XXVI) 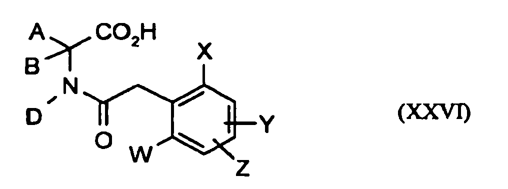 in welcher
A, B, D, W, X, Y und Z die oben angegebenen Bedeutungen haben,
verestert (Chem. Ind. (London) 1568 (1968)).

Die Verbindungen der Formel (XXVI) 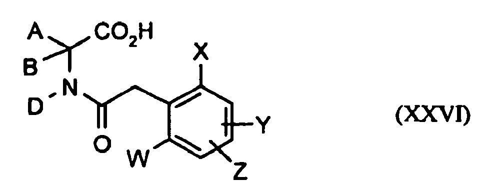 in welcher
- A, B, D, W, X, Y und Z: die oben angegebenen Bedeutungen haben,
sind neu.

Man erhält die Verbindungen der Formel (XXVI), wenn man Aminosäuren der Formel (XXVII) 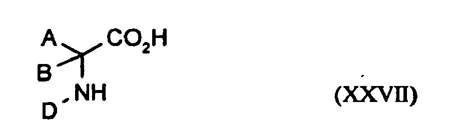 in welcher
- A, B und D: die oben angegebenen Bedeutungen haben,
mit substituierten Phenylessigsäurederivaten der Formel (XXV) 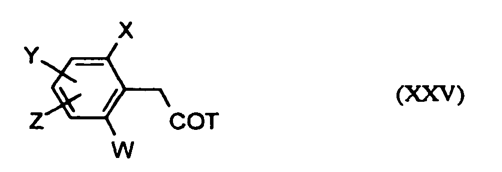 in welcher
- W, X, Y und Z: die oben angegebenen Bedeutungen haben und
- T: die oben angegebene Bedeutung hat,
beispielsweise nach Schotten-Baumann acyliert (Organikum, VEB Deutscher Verlag der Wissenschaften, Berlin 1977, S. 505).

Die Verbindungen der Formel (XXV) sind neu. Sie lassen sich nach im Prinzip bekannten Verfahren und wie aus den Herstellungsbeispielen ersichtlich darstellen (s. z.B. H. Henecka, Houben-Weyl, Methoden der Organischen Chemie, Bd. 8, S. 467-469 (1952)).

Man erhält die Verbindungen der Formel (XXV) beispielsweise, indem man substituierte Phenylessigsäuren der Formel (XXVIII) 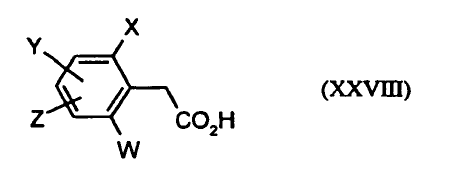 in welcher
W, X ,Y und Z die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid), Phosphorylierungsreagenzien wie (z.B. POCl₃, BOP-Cl), Carbonyldiimidazol, Carbonyldiimide (z.B. Dicyclohexylcarbonyldiimid) gegebenenfalls in Gegenwart eines Verdünnungsmittels (z.B. gegebenenfalls chlorierten aliphatischen oder aromatischen Kohlenwasserstoffen wie Toluol oder Methylenchlorid oder Ethern, z.B. Tetrahydrofuran, Dioxan, Methyl-tert.-butylether) bei Temperaturen von -20°C bis 150°C, bevorzugt von -10°C bis 100°C, umsetzt.

Die Verbindungen der Formel (XXIV) und (XXVII) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXVII), in der A und B einen Ring bilden, sind im Allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 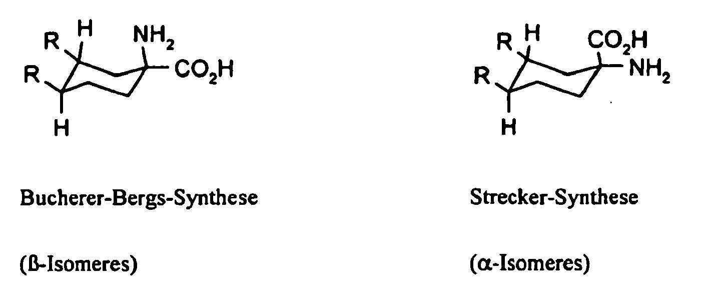 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 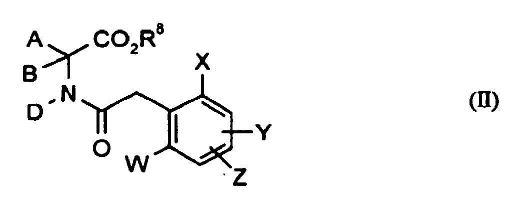 in welcher
- A, B, D, W, X, Y, Z und R⁸: die oben angegebenen Bedeutungen haben,
herstellen, wenn man Aminonitrile der Formel (XXIX) 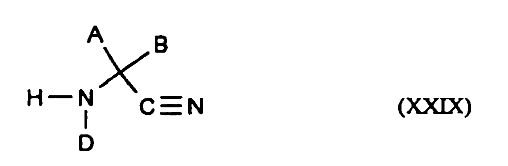 in welcher
- A, B und D: die oben angegebenen Bedeutungen haben,
mit substituierten Phenylessigsäurederivaten der Formel (XXV) 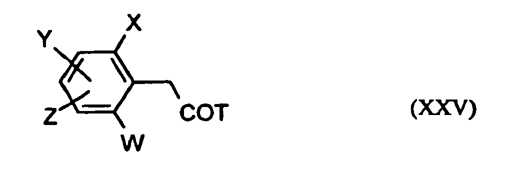 in welcher
- T, W, X, Y und Z: die oben angegebenen Bedeutungen haben,
zu Verbindungen der Formel (XXX) 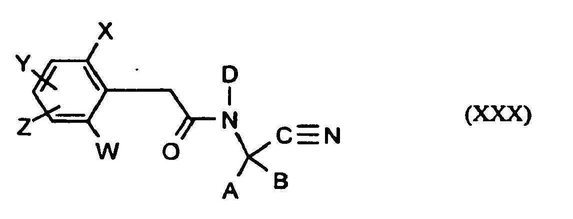 in welcher
- A, B, D, W, X, Y und Z: die oben angegebenen Bedeutungen haben,
umsetzt,
und diese anschließend einer sauren Alkoholyse unterwirft.

Die Verbindungen der Formel (XXX) sind ebenfalls neu.

Die bei dem erfindungsgemäßen Verfahren (B) als Ausgangstofie benötigten Verbindungen der Formel (III) 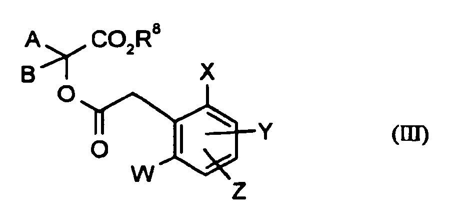 in welcher
- A, B, W, X, Y, Z und R⁸: die oben angegebenen Bedeutungen haben,
sind neu.

Sie lassen sich nach im Prinzip bekannten Methoden herstellen.

So erhält man die Verbindungen der Formel (III) beispielsweise, wenn man 2-Hydroxycarbonsäureester der Formel (XXXI-A) 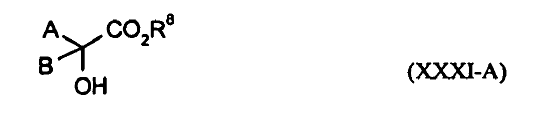 in welcher
- A, B und R⁸: die oben angegebenen Bedeutungen haben,
mit substituierten Phenylessigsäurederivaten der Formel (XXV) 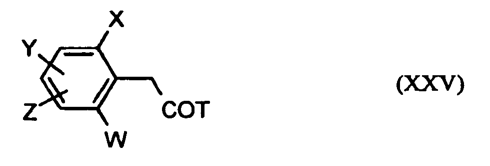 in welcher
T, W, X, Y und Z die oben angegebenen Bedeutungen haben,
acyliert (Chem. Reviews 52, 237-416 (1953)).

Weiterhin erhält man Verbindungen der Formel (III), wenn man substituierte Phenylessigsäuren der Formel (XXVIII) 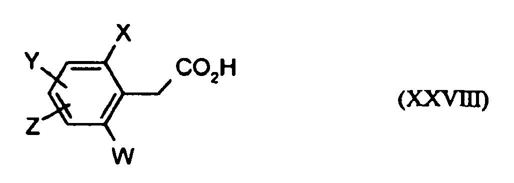 in welcher
W, X, Y und Z die oben angegebenen Bedeutungen haben,
mit α-Halogencarbonsäureestern der Formel (XXXI-B) 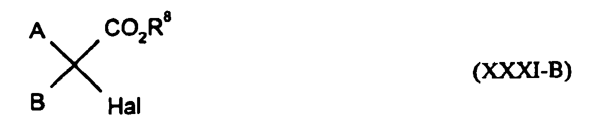 in welcher
- A, B und R⁸: die oben angegebenen Bedeutungen haben und
- Hal: für Chlor oder Brom steht,
alkyliert.

Die Verbindungen der Formel (XXVIII) sind neu.

Die Verbindungen der Formel (XXXI-B) sind käuflich.

Beispielsweise erhält man die Verbindungen der Formel (XXVIII), 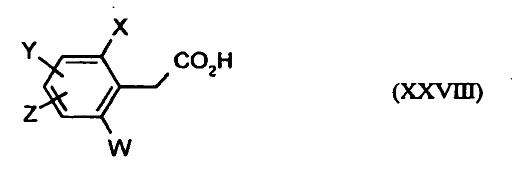 in welcher
W, X, Y und Z die oben angegebenen Bedeutungen haben,
wenn man Phenylessigsäureester der Formel (XXXII) 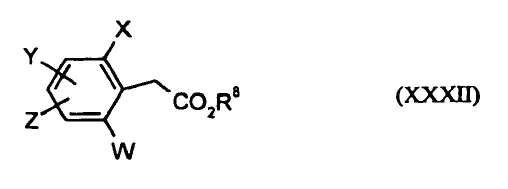 in welcher
- W, X, Y, Z und R⁸: die oben angegebene Bedeutung haben,
in Gegenwart von Säuren oder Basen, in Gegenwart eines Lösungsmittels unter allgemein bekannten Standardbedingungen verseift.

Die Verbindungen der Formel (XXXII) sind neu.

Die Verbindungen der Formel (XXXII) 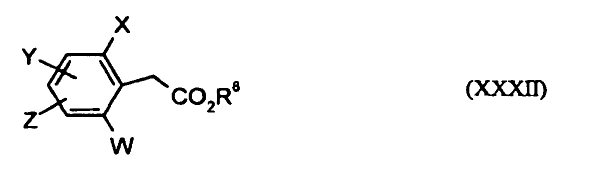 in welcher
- W, X, Y, Z und R⁸: die oben angegebene Bedeutung haben,
erhält man beispielsweise nach dem in den Beispielen beschriebenen Verfahren (Q),
wenn man Phenylessigsäureester der Formel (XXXII-a) 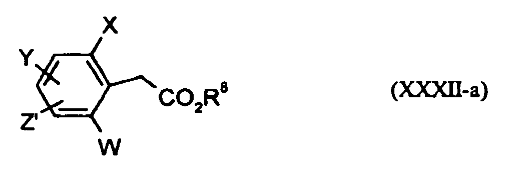 in welcher
- R⁸, W, X und Y: die oben angegebene Bedeutung haben, und
- Z': für Halogen (insbesondere für Brom) steht,
in Gegenwart eines HN-haltigen Heterocyclus der Formel (XXIII), in welcher Z die oben angegebene Bedeutung hat, in Gegenwart einer Base und gegebenenfalls in Gegenwart eines Katalysators (bevorzugt Kupfersalze wie z.B. Kupfer(I)iodid) umsetzt (S. Buchwald et. al. JACS 123, 7727, 2001).

Die Phenylessigsäureester der Formel (XXXII-a) sind teilweise aus den Anmeldungen WO 96/35 664 und WO 97/02 243 bekannt oder lassen sich nach den dort beschriebenen Verfahren herstellen.

Die bei den obigen Verfahren (H-α) als Ausgangsstoffe benötigten Halogencarbonylketene der Formel (VI) sind neu. Sie lassen sich nach im Prinzip bekannten Methoden in einfacher Weise herstellen (vgl. beispielsweise Org. Prep. Proced. Int., 7, (4), 155-158, 1975 und DE 1 945 703). So erhält man z.B. die Verbindungen der Formel (VI) 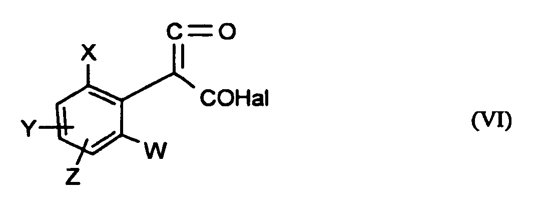 in welcher
- W, X, Y und Z: die oben angegebenen Bedeutungen haben und
- Hal: für Chlor oder Brom steht,
wenn man
substituierte Phenylmalonsäuren der Formel (XXXIV) 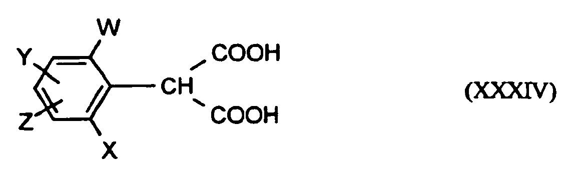 in welcher
W, X, Y und Z die oben angegebenen Bedeutungen haben, mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methyl-Sterylformamid oder Triphenylphosphin und gegebenenfalls in Gegenwart von Basen wie z.B. Pyridin oder Triethylamin, umsetzt.

Die substituierten Phenylmalonsäuren der Formel (XXXIV) sind neu. Sie lassen sich in einfacher Weise nach bekannten Verfahren herstellen (vgl. z.B. Organikum, VEB Deutscher Verlag der Wissenschaften, Berlin 1977, S. 517 ff, EP-A-528 156, WO 96/35 664, WO 97/02 243, WO 97/01535, WO 97/36868 und WO 98/05638).

So erhält man Phenylmalonsäuren der Formel (XXXIV) 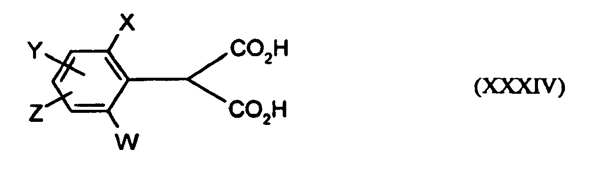 in welcher
W, X ,Y und Z die oben angegebenen Bedeutungen haben,
wenn man Phenylmalonsäurederivate der Formel (XI) mit U = OR⁸ 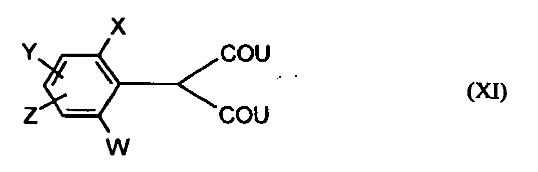 in welcher
- U, W, X, Y und Z: die oben angegebene Bedeutung haben,
zunächst in Gegenwart einer Base und einem Lösungsmittel verseift und anschließend vorsichtig ansäuert (EP-A-528 156, WO 96/35 664, WO 97/02 243).

Die Malonsäureester der Formel (XI) mit U = OR⁸ 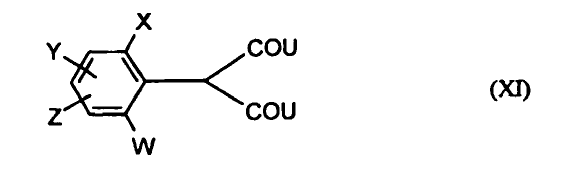 in welcher
U, W, X, Y und Z die oben angegebene Bedeutung haben,
sind neu.

Sie lassen sich nach allgemein bekannten Methoden der Organischen Chemie darstellen (vgl. z.B. Tetrahedron Lett. 27, 2763 (1986), Organikum VEB Deutscher Verlag der Wissenschaften, Berlin 1977, S. 587 ff., WO 96/35664, WO 97/02243, WO 97/01535, WO 97/36868, WO 98/05638 und WO 99/47525).

Die bei dem obigen Verfahren (F) als Ausgangsstoffe benötigten Verbindungen der Formel (VIII) 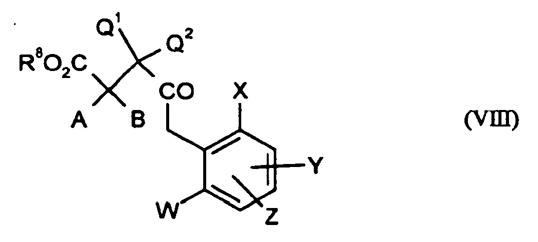 in welcher
- A, B, Q¹, Q², W, X, Y, Z und R⁸: die oben angegebene Bedeutung haben,
sind neu.

Sie lassen sich nach im Prinzip bekannten Methoden herstellen.

Man erhält die 5-Aryl-4-ketocarbonsäureester der Formel (VIII) beispielsweise, wenn man 5-Aryl-4-ketocarbonsäuren der Formel (XXXV) 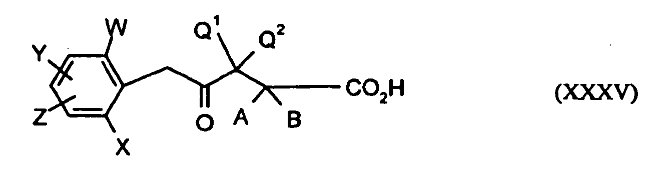 in welcher
- W, X, Y, Z, A, B, Q¹ und Q²: die oben angegebene Bedeutung haben,
verestert (vgl. z.B. Organikum, 15. Auflage, Berlin, 1977, Seite 499) oder alkyliert (siehe Herstellungsbeispiel).

Die 5-Aryl-4-ketocarbonsäuren der Formel (XXXV) 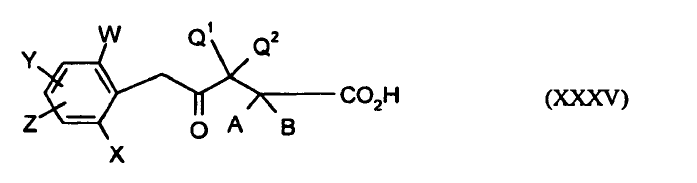 in welcher
A, B, Q¹, Q², W, X, Y und Z die oben angegebene Bedeutung haben,
sind neu, lassen sich aber nach im Prinzip bekannten Methoden herstellen (WO 96/01 798, WO 97/14667, WO 98/39281).

Man erhält die 5-Aryl-4-ketocarbonsäuren der Formel (XXXV) beispielsweise, wenn man 2-Phenyl-3-oxo-adipinsäureester der Formel (XXXVI) 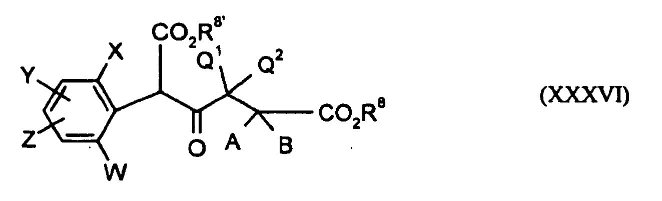 in welcher
- A, B, Q¹, Q², W, X, Y und Z: die oben angegebene Bedeutung haben und
- R⁸ und R⁸': für Alkyl (insbesondere C₁-C₈-Alkyl) stehen und
bei Einsatz der Verbindung der Formel (XXXVIII) R⁸ für Wasserstoff steht,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XXXVI) 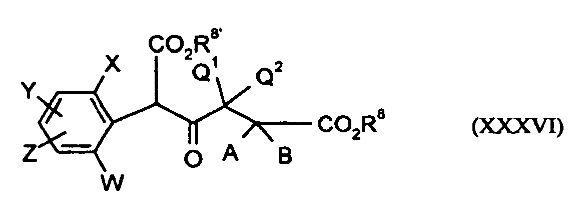 in welcher
- A, B, Q¹, Q², W, X, Y, Z, R⁸, R⁸': bei Einsatz der Verbindung der Formel sind neu.
die oben angegebene Bedeutung haben und in denen (XXXVIII) R⁸ auch für Wasserstoff steht,

Man erhält die Verbindungen der Formel (XXXVI) beispielsweise, wenn man Dicarbonsäurehalbesterchloride der Formel (XXXVII), 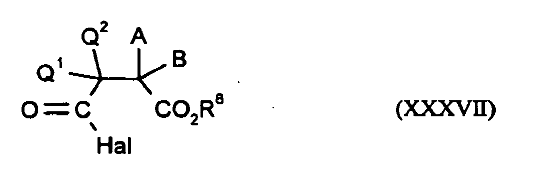 in welcher
- A, B, Q¹, Q² und R⁸: die oben angegebene Bedeutung haben und
- Hal: für Chlor oder Brom steht,
oder Carbonsäureanhydride der Formel (XXXVIII) 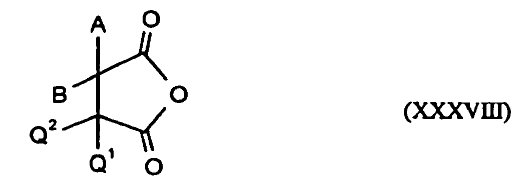 in welcher
A, B, Q¹ und Q² die oben angegebene Bedeutung haben,
mit einem Phenylessigsäureester der Formel (XXXII) 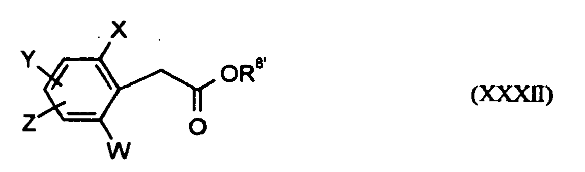 in welcher
- W, X, Y, Z und R⁸': die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Die Verbindungen der Formeln (XXXVII) und (XXXVIII) sind teilweise bekannte Verbindungen der Organischen Chemie und/oder lassen sich nach im Prinzip bekannten Methoden in einfacher Weise herstellen.

Die bei dem obigen Verfahren (G) als Ausgangsstoffe benötigten Verbindungen der Formel (IX) 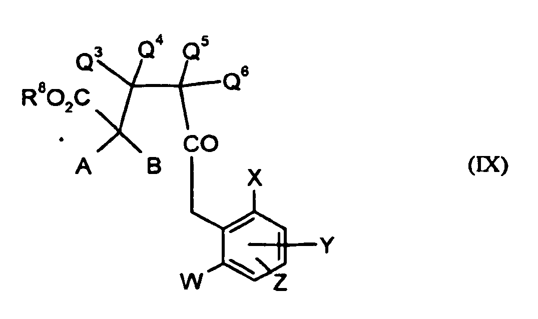 in welcher
- A, B, Q³, Q⁴, Q⁵, Q⁶, W, X, Y, Z und R⁸: die oben angegebene Bedeutung haben, sind neu.

Sie lassen sich nach im Prinzip bekannten Methoden herstellen.

Man erhält die 6-Aryl-5-ketocarbonsäureester der Formel (IX) beispielsweise, wenn man 6-Aryl-5-ketocarbonsäuren der Formel (XXXIX) 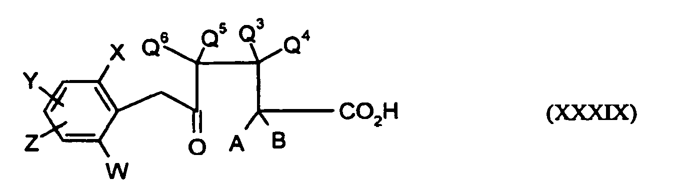 in welcher
A, B, Q³, Q⁴, Q⁵, Q⁶, W, X, Y und Z die oben angegebene Bedeutung haben,
verestert, (vgl. z.B. Organikum, 15. Auflage, Berlin, 1977, Seite 499).

Die 6-Aryl-5-ketocarbonsäuren der Formeln (XXXIX) 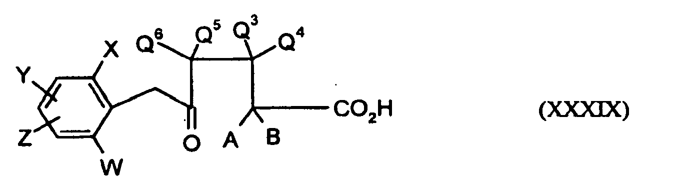 in welcher
A, B, Q³, Q⁴, Q⁵, Q⁶, W, X, Y und Z die oben angegebene Bedeutung haben,
sind neu. Sie lassen sich nach im Prinzip bekannten Methoden herstellen (WO 99/43649, WO 99/48869), beispielsweise wenn man
substituierte 2-Phenyl-3-oxo-heptandisäureester der Formel (XL) 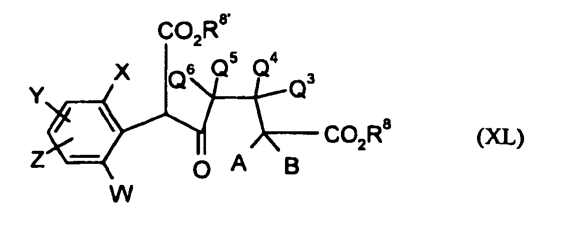 in welcher
- A, B, Q³, Q⁴, Q⁵, Q⁶, W, X, Y und Z: die oben angegebene Bedeutung haben und
- R⁸ und R^{8'}: für Alkyl (bevorzugt C₁-C₆-Alkyl), stehen, und
bei Einsatz der Verbindung der Formel (XLII) R⁸ für Wasserstoff steht,
gegebenenfalls in Gegenwart eines Verdünnungsmittels und gegebenenfalls in Gegenwart einer Base oder Säure verseift und decarboxyliert (vgl. z.B. Organikum, 15. Auflage, Berlin, 1977, Seite 519 bis 521).

Die Verbindungen der Formel (XL) 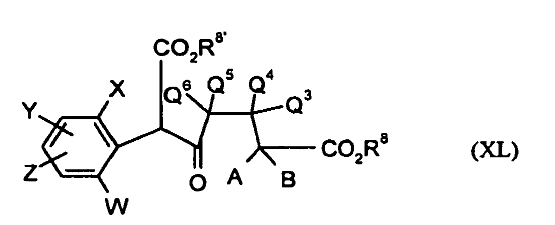 in welcher
A, B, Q³, Q⁴, Q⁵, Q⁶, W, X, Y, Z, R⁸ und R⁸' die oben angegebene Bedeutung haben,
sind neu und erhältlich,
wenn man Dicarbonsäureester der Formel (XLI), 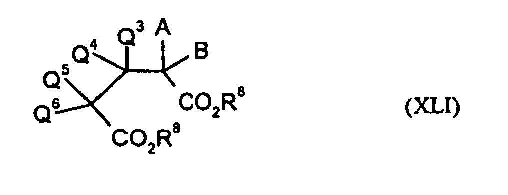 in welcher
- A, B, Q³, Q⁴, Q⁵, Q⁶ und R⁸: die oben angegebene Bedeutung haben,
oder Carbonsäureanhydride der Formel (XLII) 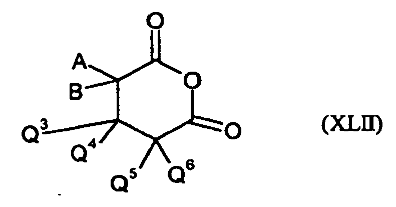 in welcher
- A, B, Q³, Q⁴, Q⁵, Q⁶: die oben angegebene Bedeutung haben
mit einem substituierten Phenylessigsäureester der Formel (XXXII) 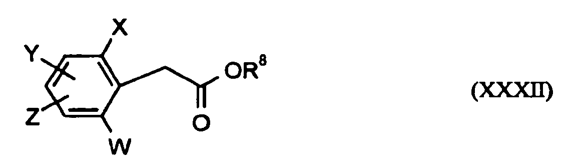 in welcher
- W, X, Y, Z und R⁸: die oben angegebene Bedeutung haben,
in Gegenwart eines Verdünnungsmittels und in Gegenwart einer Base kondensiert.

Die Verbindungen der Formel (XLI) und (XLII) sind teilweise bekannt und/oder lassen sich nach bekannten Verfahren darstellen.

Die für das erfindungsgemäße Verfahren (H-α) und (H-β) als Ausgangsstoffe benötigten Hydrazine der Formel (X)

A-NH-NH-D (X)

in welcher
- A und D: die oben angegebenen Bedeutungen haben,
sind teilweise bekannt und/oder nach literaturbekannten Methoden herstellbar (vgl. beispielsweise Liebigs Ann. Chem. 585, 6 (1954); Reaktionen der organischen Synthese, C. Fern, Seite 212, 513; Georg Thieme Verlag Stuttgart, 1978; Liebigs Ann. Chem. 443, 242 (1925); Chem. Ber. 98, 2551 (1965), EP-A-508 126, WO 92/16510, WO 99/47 525, WO 01/17 972).

Die für das erfindungsgemäße Verfahren (H-γ) benötigten Verbindungen der Formel (XII) 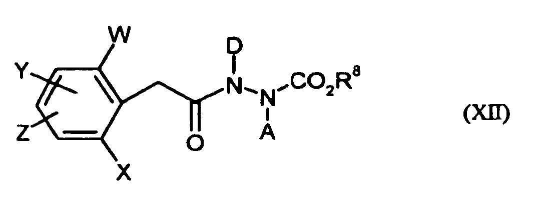 in welcher
- A, D, W, X, Y, Z und R⁸: die oben angegebene Bedeutung haben,
sind neu.

Man erhält die Acylcarbazate der Formel (XII) beispielsweise, wenn man Carbazate der Formel. (XLIII) 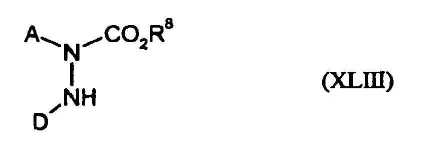 in welcher
- A, R⁸ und D: die oben angegebenen Bedeutungen haben,
mit substituierten Phenylessigsäurederivaten der Formel (XXV) 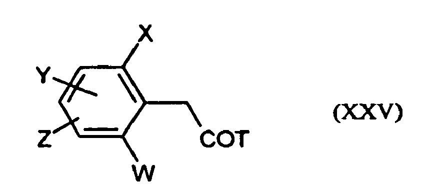 in welcher
- T, W, X, Y und Z: die oben angegebenen Bedeutungen haben
acyliert (Chem. Reviews 52, 237-416 (1953); Bhattacharya, Indian J. Chem. 6, 341-5, 1968):

Die Carbazate der Formel (XLIII) sind teilweise käufliche und teilweise bekannte Verbindungen oder lassen sich nach im Prinzip bekannten Verfahren der organischen Chemie herstellen.

Die bei dem obigen Verfahren (P) als Ausgangsstoffe benötigten Verbindungen der Formeln (I-1') bis (I-8'), in welchen A, B, D, G, Q¹, Q², Q³, Q⁴, Q⁵, Q⁶, W, X und Y die oben angegebene Bedeutung haben und Z' für Chlor und Brom, bevorzugt für Brom steht, sind teilweise aus dem eingangs zitierten Patentanmeldungen bekannt (z.B. WO 96/35 664, WO 97/02 243) oder lassen sich gemäß den dort beschriebenen Verfahren herstellen.

Die Verbindungen der Formel (XXIII)

H - Z (XXIII)

in welcher
- Z: die oben angegebene Bedeutung hat,
sind teilweise käuflich oder lassen sich nach allgemeinen und im Prinzip bekannten Verfahren herstellen.

Die zur Durchführung der erfindungsgemäßen Verfahren (I), (J), (K), außerdem als Ausgangsstoffe benötigten Säurehalogenide der Formel (XIII), Carbonsäureanhydride der Formel (XIV), Chlorameisensäureester oder Chlorameisensäurethioester der Formel (XV), Chlormonothioameisensäureester oder Chlordithioameisensäureester der Formel (XVI), sind allgemein bekannte Verbindungen der Organischen bzw. Anorganischen Chemie.

Die Verbindungen der Formeln (XIII) bis (XXII), (XXIV), (XXVII), (XXIX), (XXXI-A), (XXXI-B), (XXXVII), (XXXVIII), (XLI) und (XLII) sind darüber hinaus aus den eingangs zitierten Patentanmeldungen bekannt und/oder lassen sich nach den dort angegebenen Methoden herstellen.

Das Verfahren (A) ist dadurch gekennzeichnet, dass man Verbindungen der Formel (II), in welcher A, B, D, W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid, Dimethylacetamid und N-Methylpyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und - hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formel (II) und die deprotonierenden Basen im Allgemeinen in etwa doppeläquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass Verbindungen der Formel (III), in welcher A, B, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.butylat einsetzbar.

Die Reaktionstemperaturen können bei der Durchführung des erfindungsgemäßen Verfahrens (B) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (B) wird im Allgemeinen unter Normaldruck durchgeführt.

Bei der Durchführung des erfindungsgemäßen Verfahrens (B) setzt man die Reaktionskomponenten der Formel (III) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (F) ist dadurch gekennzeichnet, dass man Verbindungen der Formel (VIII), in welcher A, B, Q¹, Q², W, X, Y, Z und R⁸ die oben angegebene Bedeutung haben, in Gegenwart einer Base einer intramolekularen Kondensation unterwirft.

Als Verdünnungsmittel können bei dem erfindungsgemäßen Verfahren (F) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (F) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (F) innerhalb eines größeren Bereiches variiert werden. Im Allgemeinen arbeitet man bei Temperaturen zwischen -75°C und 250°C, vorzugsweise zwischen -50°C und 150°C.

Das erfindungsgemäße Verfahren (F) wird im Allgemeinen unter Normaldruck durchgerührt.

Bei der Durchführung des erfindungsgemäßen Verfahrens (F) setzt man die Reaktionskomponenten der Formel (VIII) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (G) ist dadurch gekennzeichnet, dass man Verbindungen der Formel (IX), in welcher A, B, Q³, Q⁴, Q⁵, Q⁶, W, X, Y, Z und R⁸ die oben angegebene Bedeutung haben, in Gegenwart von Basen einer intramolekularen Kondensation unterwirft.

Als Verdünnungsmittel können bei dem erfindungsgemäßen Verfahren (G) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methylpyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (G) alle üblichen Protonenakzeptoren eingesetzt werden.

Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methytrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (G) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (G) wird im Allgemeinen unter Normaldruck durchgeführt.

Bei der Durchführung des erfindungsgemäßen Verfahrens (G) setzt man die Reaktionskomponenten der Formel (IX) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das erfindungsgemäße Verfahren (H-α) ist dadurch gekennzeichnet, dass man Hydrazine der Formel (X) oder Salze dieser Verbindungen mit Ketensäurehalogeniden der Formel (VI) in Gegenwart eines Verdünnungsmittels und gegebenenfalls in Gegenwart eines Säureakzeptors umsetzt.

Als Verdünnungsmittel können bei dem erfindungsgemäßen Verfahren (H-α) alle inerten organischen Solventien eingesetzt werden. Vorzugsweise verwendbar sind gegebenenfalls chlorierte Kohlenwasserstoffe, wie beispielsweise Mesitylen, Chlorbenzol und Dichlorbenzol, Toluol, Xylol, ferner Ether, wie Dibutylether, Glykoldimethylether, Diglykoldimethylether und Diphenylethan, außerdem polare Lösungsmittel, wie Dimethylsulfoxid, Sulfolan, Dimethylformamid oder N-Methyl-pyrrolidon.

Als Säureakzeptoren können bei der Durchführung der erfindungsgemäßen Verfahrensvariante (H-α) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante (H-α) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (H-α) wird zweckmäßigerweise unter Normaldruck durchgeführt.

Bei der Durchführung des erfindungsgemäßen Verfahrens (H-α) setzt man die Reaktionskomponenten der Formeln (VI) und (X), in welchen A, D, W, X, Y und Z die oben angegebenen Bedeutungen haben und Hal für Halogen steht, und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das Verfahren (H-β) ist dadurch gekennzeichnet, dass man Hydrazine der Formel (X) oder Salze dieser Verbindung, in welcher A und D die oben angegebenen Bedeutungen haben, mit Malonestern oder Malonsäureamiden der Formel (XI), in welcher U, W, X, Y, Z und R⁸ die oben angegebene Bedeutung haben, in Gegenwart einer Base einer Kondensation unterwirft.

Als Verdünnungsmittel können bei dem erfindungsgemäßen Verfahren (H-β) alle inerten organischen Solventien eingesetzt werden. Vorzugsweise verwendbar sind gegebenenfalls halogenierte Kohlenwasserstoffe, wie Toluol, Xylol, Mesitylen, Chlorbenzol und Dichlorbenzol, ferner Ether, wie Dibutylether, Tetrahydrofuran, Dioxan, Diphenylether, Glykoldimethylether und Diglykoldimethylelhel, außerdem polare Lösungsmitteln, wie Dimethylsulfoxid, Sulfolan, Dimethylformamid, Dimethylacetamid und N-Methylpyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (H-β) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Katium-tert.-butylat einsetzbar.

Verwendbar sind auch tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindugnsgemäßen Verfahrens (H-β) innerhalb eines größeren Bereiches variiert werden. Im Allgemeinen arbeitet man bei Temperaturen zwischen 0°C und 280°C, vorzugsweise zwischen 50°C und 180°C.

Das erfindungsgemäße Verfahren (H-β) wird im Allgemeinen unter Normaldruck durchgeführt.

Bei der Durchführung des erfindungsgemäßen Verfahrens (H-β) setzt man die Reaktionskomponenten der Formeln (XI) und (X) im Allgemeinen in etwa äquimolaren Mengen ein. Es ist jedoch auch möglich, die eine oder andere Komponente in einem größeren Überschuss (bis zu 3 mol) zu verwenden.

Das Verfahren (H-γ) ist dadurch gekennzeichnet, dass man Verbindungen der Formel (XII), in welcher A, D, W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (H-γ) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol" Butanol, Iso-Butanol und tert.-Butanol.

Als Base (Deprotonierungsmittel) können bei der Durchführung des erfindungsgemäßen Verfahrens (H-γ) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und - hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (H-γ) innerhalb eines größeren Bereiches variiert werden. Im Allgemeinen arbeitet man bei Temperaturen zwischen -20°C und 250°C, vorzugsweise zwischen 50°C und 150°C.

Das erfindungsgemäße Verfahren (H-γ) wird im Allgemeinen unter Normaldruck durchgeführt.

Bei der Durchführung des erfindungsgemäßen Verfahrens (H-γ) setzt man die Reaktionskomponenten der Formel (XII) und die deprotonierenden Basen im Allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (I-α) ist dadurch gekennzeichnet, dass man Verbindungen der Formeln (I-1-a) bis (I-8-a) jeweils mit Carbonsäurehalogeniden der Formel (XIII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (I-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (I-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkalimetall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (I-α) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (I-α) werden die Ausgangsstoffe der Formeln (I-1-a) bis (I-8-a) und das Carbonsäurehalogenid der Formel (XIII) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (I-β) ist dadurch gekennzeichnet, dass man Verbindungen der Formeln (I-1-a) bis (1-8-a) mit Carbonsäureanhydriden der Formel (XIV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (I-β) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (I-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (I-β) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (I-β) werden die Ausgangsstoffe der Formeln (I-1-a) bis (I-8-a) und das Carbonsäureanhydrid der Formel (XIV) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (J) ist dadurch gekennzeichnet, dass man Verbindungen der Formeln (I-1-a) bis (I-8-a) jeweils mit Chlorameisensäureestern oder Chlorameisensäurethiolestern der Formel (XV) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (J) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethylanilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (J) alle gegenüber den Chlorameisensäureestern bzw. Chlorameisensäurethiolestern inerten Solventien eingesetzt werden. Vorzugsweise verwendbar sind Kohlenwasserstofie, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (J) innerhalb eines größeren Bereiches variiert werden. Arbeitet man in Gegenwart eines Verdünnungsmittels und eines Säurebindemittels, so liegen die Reaktionstemperaturen im Allgemeinen zwischen -20°C und +100°C, vorzugsweise zwischen 0°C und 50°C.

Das erfindungsgemäße Verfahren (J) wird im Allgemeinen unter Normaldruck durchgeführt.

Bei der Durchführung des erfindungsgemäßen Verfahrens (J) werden die Ausgangsstoffe der Formeln (I-1-a) bis (I-8-a) und der entsprechende Chlorameisensäureester bzw. Chlorameisensäurethiolester der Formel (XIII) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (K) ist dadurch gekennzeichnet, dass man Verbindungen der Formeln (I-1-a) bis (I-8-a) jeweils mit Verbindungen der Formel (XVI) in Gegenwart eines Verdünnungsmittels und gegebenenfalls in Gegenwart eines Säurebindemittels umsetzt.

Beim Herstellungsverfahren (K) setzt man pro Mol Ausgangsverbindung der Formeln (I-1-a) bis (I-8-a) ca. 1 Mol Chlormonothioameisensäureester bzw. Chlordithioameisensäureester der Formel (XVI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-8-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das Verfahren (P) ist dadurch gekennzeichnet, dass man Verbindungen der Formel (I-1') bis (I-8'), in welchen A, B, D, Q¹, Q², Q³, Q⁴, Q⁵, Q⁶, W, X, Y, R¹, R², R³, R⁴, R⁵, R⁶ und R⁷ die oben genannten Bedeutungen haben und Z' für Halogen besonders bevorzugt für Brom steht, mit HN-Heterocyclen der Formel (XXIII) in welcher Z die oben angegebene Bedeutung hat, in Gegenwart eines Kupfersalzes und in Gegenwart einer Base einer Kupplungsreaktion unterwirft (J. Am. Chem. Soc. 2001, 123, 7729-29; WO 02-85 838; Synlett 2002, 3, 423-30).

Als Lösungsmitteln kommen bei dem erfindungsgemäßen Verfahren (P) beispielsweise gegebenenfalls halogenierte aliphatische, alicyclische oder aromatische Kohlenwasserstoffe, wie beispielsweise Benzin, Benzol, Toluol, Xylol, Petrolether, Hexan, Cyclohexan, Chlorbenzol, Dichlorbenzol, Ether, wie Diethylether, Diisopropylether, Dioxan, Tetrahydrofuran oder Ethylenglykoldimethyl- oder -diethylether; Amide wie beispielsweise N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Mischungen solcher Lösungsmittel in Frage. Bevorzugt wird N,N-Dimethylformamid eingesetzt.

Als Basen können bei den erfindungsgemäßen Verfahren (P) Alkali- und/oder Erdalkalimetallcarbonate, -alkoholate, -phosphate, -fluoride und/oder -hydroxide eingesetzt werden, wobei insbesondere Kaliumcarbonat, Natriumcarbonat, Cäsiumcarbonat, Cäsiumhydrogencarbonat, Natriummethanolat, Kalium-tert.-butylat, Kaliumamylat, Cäsiumfluorid, Kaliumphosphat und Bariumhydroxid bevorzugt sind. Besonders bevorzugt werden Kaliumcarbonat, Natriumcarbonat, Cäsiumcarbonat und/oder Cäsiumhydrogencarbonat. Ganz besonders bevorzugt wird Kaliumcarbonat.

Die Basen können auch in Gegenwart von Phasentransferkatalysatoren wie z.B. Triethylbenzylammoniumchlorid, Tetrabutylammoniumbromid oder TDA 1 (= Tris-(methoxyethoxyethyl)-amin) eingesetzt werden.

Als Kupfersalze werden bei dem erfindungsgemäßen Verfahen (P) Kupfer-I-salze eingesetzt wie z.B. CuJ.

Weiterhin kann das Verfahren (P) auch in Gegenwart von zusätzlichen Hilfsbasen wie Diaminen wie z.B. Ethylendiamin, Propylendiamin, 1,2-Diaminocyclohexan durchgeführt werden.

Die Reaktionstemperaturen können bei der Durchführung des Verfahrens (P) innerhalb eines größeren Bereiches variiert werden. Im Allgemeinen arbeitet man bei Temperaturen von 0 bis 250°C, bevorzugt bei 30 bis 200°C; ganz besonders bevorzugt bei 50 bis 150°C.

Das erfindungsgemäße Verfahren (P) wird im Allgemeinen unter Normaldruck durchgeführt.

Bei der Durchführung des erfindungsgemäßen Verfahrens (P) setzt man die Reaktionskomponenten der Formeln (I-1') bis (I-8') und (XXIII) im Allgemeinen in äquimolaren Mengen ein. Es ist jedoch auch möglich, die eine oder andere Komponente in einen größeren Überschuss (bis zu 3 Mol) zu verwenden. Die Basen werden im Allgemeinen im Molverhältnis 1:1 bis 10:1 bevorzugt 2:1 bis 5:1 eingesetzt. Die Kupfersalze werden im Allgemeinen im Molverhältnis von 0,01:1 bis 1:1, bevorzugt 0,05:1 bis 0,5:1 eingesetzt.

Das Verfahren Q ist dadurch gekennzeichnet, dass man Verbindungen der Formel (XXXII-a), in welcher W, X, Y und R⁸ die oben angegebene Bedeutung haben und Z' für Halogen, besonders bevorzugt für Brom steht, mit HN-haltigen Heterocyclen der Formel (XXIII), in welcher Z die oben angegebene Bedeutung hat, in Gegenwart einer Base und in Gegenwart eines Kupfersalzes einer Kupplungsreaktion unterwirft (J. Am. Chem. Soc. 2001, 123, 7727-29; WO 02-85 838, Synlett 2002, 3, 427-30).

Als Lösungsmittel kommen bei dem erfindungsgemäßen Verfahren (Q) gegebenenfalls halogenierte aliphatische, alicyclische oder aromatische Kohlenwasserstoffe, wie beispielsweise Benzin, Benzol, Toluol, Xylol, Petrolether, Hexan, Cyclohexan, Chlorbenzol, Dichlorbenzol, Ehter, wie Diethylether, Diisopropylether, Dioxan, Tetrahydrofuran oder Ethylenglykoldimethyl- oder -diethylether; Amide wie beispielsweise N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Mischungen solcher Lösungsmittel in Frage. Bevorzugt wird N,N-Dimethylformamid eingesetzt.

Als Basen können bei den erfindungsgemäßen Verfahren (Q) Alkali- und/oder Erdalkalimetallcarbonate, -alkoholate, -phosphate, -fluoride und/oder -hydroxide eingesetzt werden, wobei insbesondere Kaliumcarbonat, Natriumcarbonat, Cäsiumcarbonat, Cäsiumhydrogencarbonat, Natriummethanolat, Kalium-tert.-butylat, Kaliumamylat, Cäsiumfluorid, Kaliumphosphat und Bariumhydroxid bevorzugt sind. Besonders bevorzugt werden Kaliumcarbonat, Natriumcarbonat, Cäsiumcarbonat und/oder Cäsiumhydrogencarbonat. Ganz besonders bevorzugt wird Kaliumcarbonat.

Die Basen können auch in Gegenwart von Phasentransferkatalysatoren wie z.B. Triethylbenzylammoniumchlorid, Tetrabutylammoniumbromid oder TDA 1 (= Tris-(methoxyethoxyethyl)-amin)eingesetzt werden.

Als Kupfersalze werden bei dem erfindungsgemäßen Verfahren (Q) Kupfer-I-salze eingesetzt wie z.B. CuJ.

Weiterhin kann das Verfahren (Q) auch in Gegenwart von zusätzlichen Hilfsbasen wie Diaminen wie z.B. Ethylendiamin, Propylendiamin, 1,2-Diaminocyclohexan durchgeführt werden.

Die Reaktionstemperaturen können bei der Durchführung des Verfahrens (Q) innerhalb eines größeren Bereiches variiert werden. Im Allgemeinen arbeitet man bei Temperaturen von 0 bis 250°C, bevorzugt bei 30 bis 200°C; ganz besonders bevorzugt von 50 bis 150°C.

Das erfindungsgemäße Verfahren (Q) wird im Allgemeinen unter Normaldruck durchgeführt.

Bei der Durchführung des erfindungsgemäßen Verfahrens (Q) setzt man die Reaktionskomponenten der Formeln (XXXII-a) und (XXIII) im Allgemeinen in äquimolaren Mengen ein. Es ist jedoch auch möglich, die eine oder andere Komponente in einem größeren Überschuss (bis zu 3 Mol) zu verwenden. Die Basen werden im Allgemeinen im Molverhältnis 1:1 bis 10:1, bevorzugt 2:1 bis 5:1 eingesetzt. Die Kupfersalze werden im Allgemeinen im Molverhältnis von 0.001 bis 1:1, bevorzugt 0.05:1 bis 0.5:1 eingesetzt.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica. Aus der Ordnung der Dermaptera z.B. Forficula auricularia.

Aus der Ordnung der Isoptera z.B. Reticulitermes spp.

Aus der Ordnung der Phthiraptera z.B. Pediculus humanus corporis, Haematopinus spp., Linognathus spp., Trichodectes spp., Damalinia spp.

Aus der Ordnung der Thysanoptera z.B. Hercinothrips femoralis, Thrips tabaci, Thrips palmi, Frankliniella accidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp.

Aus der Ordnung der Siphonaptera z.B. Xenopsylla cheopis, Ceratophyllus spp.

Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Die erfindungsgemäßen Verbindungen bzw. Wirkstoffkombinationen können gegebenenfalls in bestimmten Konzentrationen bzw. Aufwandmengen auch als Herbizide verwendet werden. Die Verbindungen lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt, werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen bzw. Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Spritzen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe bzw. Wirkstoffkombinationen können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorirn; Dimethirimol; Dimethomorph; Dimoxystrobin; Diniconamle; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover, Flumorph; Fluoromide; Fluoxastrobin; Fluquinconazole; Flurpromidol; Flusilazole; Flusulfamide; Flutolanil; Flutriafol; Folpet; Fosetyl-Al; Fosetyl-sodium; Fuberidazole; Furalaxyl; Furametpyr, Furcarbanil; Furmecyclox; Guazatine; Hexachlorobenzene; Hexaconazole; Hymexazol; Imazalil; Imibenconazole; Iminoctadine triacetate; Iminoctadine tris(albesilate); Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazrolecarboxamide; 2-chloro-N-(2,3-dihydro-1,1,3-trimethyl-1H-inden-4-yl)-3-pyridincarboxamide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
sowie Kupfersalze und -zubereitungen, wie Bordeaux mixture; Copper hydroxide; Copper naphthenate; Copper oxychloride; Copper sulfate; Cufraneb; Cuprous oxide; Mancopper, Oxine-copper.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Benclothiaz, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimefluthrin, Dimethoate, Dimethylvinphos, Dinobuton, Dinocap, Dinotefuran, Diofenolan, Disulfoton, Docusat-sodium, Dofenapyn, DOWCO-439,
Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-Cyhalothrin, Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin, Japonilure,
Kadethrin, Kempolyederviren, Kinoprene,
Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metofluthrin, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602, OK-9701, OK-9802, Omethoate, Oxamyl, Oxydemeton-methyl,
Paecilomyces fumosoroseus, Parathion-methyl, Parathion (-ethyl), Pennethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Potassium oleate, Prallethrin, Profenofos, Profluthrin, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos,
Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (IR-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Trichoderma atroviride, Triflumuron, Trimethacarb,
Vamidothion, Vaniliprole, Verbutin, Verticillium lecanii,
WL-108477, WL-40027,
YI-5201, YI-5301, YI-5302,
XMC, Xylylcarb,
ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff bzw. Wirkstoffkombinationen durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäße verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfeld® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft mit den erfindungsgemäßen Verbindungen bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Wirkstoffkombinationen wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp.. Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe bzw. Wirkstoffkombinationen eignen sich auch zur Bekämpfung von Arthropoden, die landwirtschaftliche Nutztiere, wie z.B. Rinder, Schafe, Ziegen, Pferde, Schweine, Esel, Karnele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe bzw. Wirkstoffkombinationen geschieht im Veterinärsektor in bekannter Weise durch enterale Verabreichung in Form von beispielsweise Tabletten, Kapseln, Tränken, Drenchen, Granulaten, Pasten, Boli, des feedthrough-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändem, Halftern, Markierungsvorrichtungen usw.

Bei der Anwendung für Vieh, Geflügel, Haustiere etc. kann man die Wirkstoffe bzw. Wirkstoffkombinationen als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen bzw. Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

Hautflügler wie Sirex juvencus, Urocerus gigas, Urocerus gigas taignus, Urocerus augur.

Termiten wie Katotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel. Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe bzw. Wirkstoffkombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln. Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im Allgemeinen ist es jedoch ausreichend 0,0001 bis 20 Gew.%, vorzugsweise 0,001 bis 10 Gew.%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.%, vorzugsweise 50 bis 68 Gew.%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylearbamat, N-Octyl-isothiazolin-3-on und 4,5-Dichlor-N-octylisothiazolin-3-on, sein.

Zugleich können die erfindungsgemäßen Verbindungen bzw. Wirkstoffkombinationen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(tri-alkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie
   Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb; oder herkömmliche Antifouling-Wirkstoffe wie
   4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen Insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe bzw. Wirkstoffkombinationen eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus. Aus der Ordnung der Dermaptera z.B. Forficula auricularia. Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe bzw. Wirkstoffkombinationen können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe bzw. Wirkstoffkombinationen können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe bzw. Wirkstoffkombinationen ist jedoch keineswegs auf diese Gattungen beschränkt, sondern erstreckt sich in gleicher Weise auch auf andere Pflanzen.

Die erfindungsgemäßen Wirkstoffe bzw. Wirkstoffkombinationen eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen bzw. Wirkstoffkombinationen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf als auch im Nachauflauf-Verfahren.

Die erfindungsgemäßen Wirkstoffe bzw. Wirkstoffkombinationen können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe bzw. Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesosulfurone, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe bzw. Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe bzw. Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) Salzen 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreistäufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr, Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]ethyl]-3-methyl- 2-[(methylsulfonyl)amino]-butanamide; 1-(1-naphthatenyt)-1H-pyrrole-2,5-dione; 2,3,5,6-tetrachloro-4-(methylsulfonyl)pyridine; 2-amino-4-methyl-N-phenyl-5-thiazolecarboxamide;2-chloro-N-(2,3-dihydro-1,1,3-trimethyl-1H-inden-4-yl)-3-pyridincarboxam ide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Benclothiaz, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimeflurhrin, Dimethoate, Dimethylvinphos, Dinobuton, Dinocap, Dinotefuran, Diofenolan, Disulfoton, Docusat-sodium, Dofenapyn, DOWCO-439,
Eflusilanate, Emamectin, Emamectin-benzoate, Empenthrin (IR-isomer), Endosulfan, Entomopthora spp., EPN, Esfenvalerate, Ethiofencarb, Ethiprole, Ethion, Ethoprophos, Etofenprox, Etoxazole, Etrimfos,
Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-cyhalothrin, Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin,
Japonilure,
Kadethrin, Kernpolyederviren, Kinoprene,
Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metofluthrin, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602, OK-9701, OK-9802, Omethoate, Oxamyl, Oxydemeton-methyl,
Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Potassium oleate, Prallethrin, Profenofos, Profluthrin, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos,
Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Trichoderma atroviride, Triflumuron, Trimethacarb,
Vamidothion, Vaniliprole, Verbutin, Verticillium lecanii,
WL-108477, WL-40027,
YI-5201, YI-5301, YI-5302,
XMC, Xylylcarb,
ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes,

Microsporon-Spezies wie Microsporon canis und audouinii. Die Aufzählung dieser Pilze stellt keinesfalls eine Beschränkung des erfaßbaren mykotischen Spektrums dar, sondern hat nur erläuternden Charakter.

Die Wirkstoffe können als solche, in Form ihrer Formulierungen oder den daraus bereiteten Anwendungsförmen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die Herstellung und die Verwendung der erfindungsgemäßen Wirkstoffe geht aus den nachfolgenden Beispielen hervor.

### Herstellungsbeispiele

### Beispiel I-1-a-1

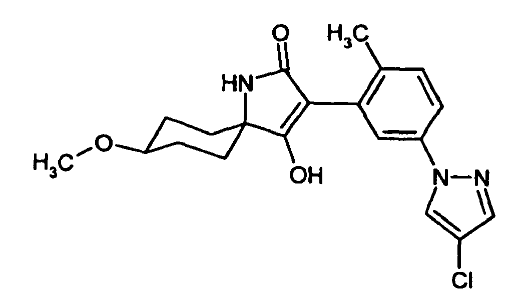

Zu 1,95 g (0,042 Mol) Kalium-tert.-butylat in 6 ml wasserfreiem Dimethylformamid (DMF) gibt man bei 40 bis 50°C 3,15 g der Verbindung gemäß Beispiel II-1 in 7 ml wasserfreiem DMF und rührt 1 Stunde bei 60°C.

Das Reaktionsgemisch wird in Eiswasser eingerührt und bei 0 bis 10°C mit konzentrierter Salzsäure auf pH 4 angesäuert. Der Niederschlag wird mit Eiswasser gewaschen und getrocknet. Es erfolgt säulenchromatographische Reinigung an Kieselgel (Dichlormethan:Methanol, 20: 1).

Ausbeute: 190 mg (6 % der Theorie). Fp.: 265°C.

In Analogie zu Beispiel (I-1-a-1) und gemäß den allgemeinen Angaben zur Herstellung erhält man folgende Verbindungen der Formel (I-1-a)

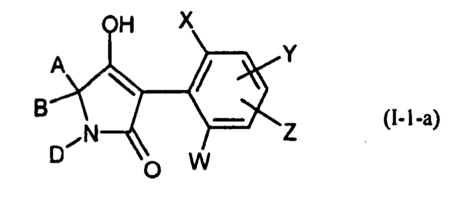

| **Bsp: Nr.** | **W** | **X** | **Y** | **Z** | **D** | **A** | **B** | **Fp.°C** | **Isomer** |
|---|---|---|---|---|---|---|---|---|---|
| I-1-a-2 | CH₃ | CH₃ | H | 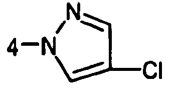 | H | -(CH₂)₂-CHOCH₃-(CH₂)₂- | | 277 | β |
| I-1-a-3 | H | CH₃ | H | 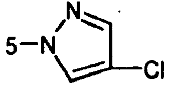 | H | -(CH₂)₅- | | 286 | - |
| I-1-a-4 | CH₃ | CH₃ | H | 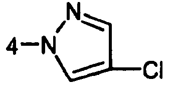 | H | CH₃ | CH₃ | 308 | - |
| I-1-a-5 | CH₃ | CH₃ | H | 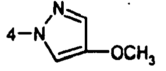 | H | -(CH₂)₂-CHOCH₃-(CH₂)₂- | | 291 | β |
| I-1-a-6 | CH₃ | C₂H₅ | H | 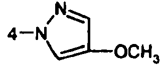 | H | -(CH₂)₂-CHOCH₃-(CH₂)₂- | | 284 | β |
| I-1-a-7 | CH₃ | C₂H₅ | H | 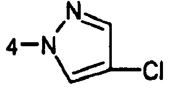 | H | -(CH₂)₂-CHOCH₃-(CH₂)₂- | | 318 | β |
| I-1-a-8 | C₂H₅ | C₂H₅ | H | 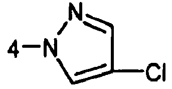 | H | -(CH₂)₂-CHOCH₃-(CH₂)₂- | | 278 | β |
| I-1-a-9 | CH₃ | CH₃ | Cl | 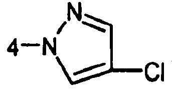 | H | CH₃ | CH₃ | 305 | - |
| I-1-a-10 | H | C₂H₅ | H | 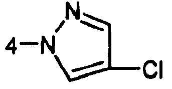 | H | -(CH₂)₂-CHOCH₃-(CH₂)₂- | | 167 | β |
| I-1-a-11 | C₂H₅ | Cl | H | 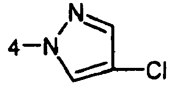 | H | CH3 | CH₃ | 294 | |
| I-1-a-12 | C₂H₅ | Cl | H | 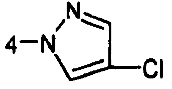 | H | -(CH₂)₂-CHOCH₃-(CH₂)₂- | | 272 | β |
| I-1-a-13 | CH₃ | C₂H₅ | H | 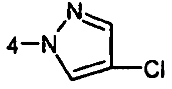 | H | .(CH₂)₂-O-(CH₂)₂- | | 269 | - |
| I-1-a-14 | CH₃ | C₂H₅ | H | 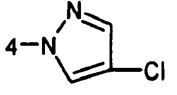 | H | CH₃ | CH₃ | 306 | - |
| I-1-a-15 | H | Cl | H | 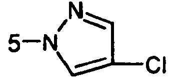 | H | -(CH₂)₂-CHOCH₃-(CH₂)₂- | | *7.57-7.77 (m, 3H, Ar-H), 7.87,8.18 (2 s, 2H Pyr-H) | β |
| I-1-a-16 | CH₃ | C₂H₅ | H | 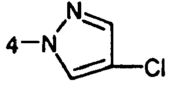 | H | 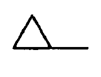 | CH₃ | 171-172 | - |

| | | | | | | | | | |
|---|---|---|---|---|---|---|---|---|---|
| * ¹H-NMR (400 MHz, d₆-DMSO): Verschiebung δ in ppm. | | | | | | | | | |

### Beispiel I-1-b-1

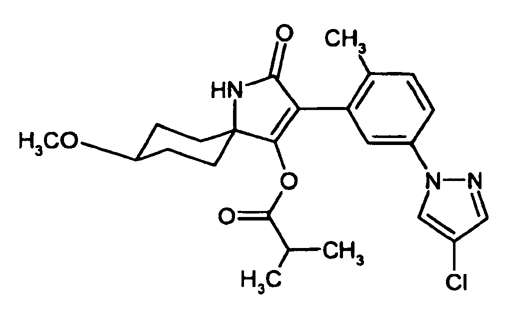

Es werden unter Argon 0,6 g der Verbindung gemäß Beispiel I-1-a-1 in 30 ml wasserfreiem Essigsäureethylester und 0,1 g Triethylamin (1,5 mmol) = 0,21 ml vorgelegt. Man katalysiert mit 10 mg Steglich-Base und versetzt unter Rückfluss mit 0,16 g (0,0015 Mol) Isobuttersäurechlorid in 2 ml wasserfreiem Dichlormethan. Es erfolgt Reaktionsverfolgung durch Dünnschichtchromatographie. Das Lösungsmittel wird abgedampft und der Rückstand an Kieselgel mit Dichlormethan/Essigsäureethylester 3:1 als Laufmittel chromatographiert.

Ausbeute: 0,25 g (34 % der Theorie), Fp. 217°C.

In Analogie zu Beispiel (I-1-b-1) und gemäß den allgemeinen Angaben zur Herstellung erhält man folgende Verbindungen der Formel (I-1-b)

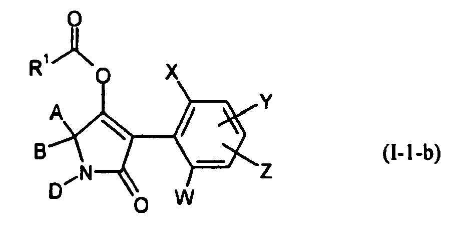

| **Bsp: Nr.** | **W** | **X** | **Y** | **Z** | **D** | **A** | **B** | **R¹** | **Fp.°C** | **Isomer** |
|---|---|---|---|---|---|---|---|---|---|---|
| I-1-b-2 | CH₃ | CH₃ | H | 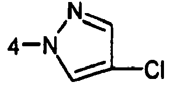 | H | -(CH₂)₂-CHOCH₃-(CH₂)₂- | | i-C₃H₇ | 247 | β |
| I-1-b-3 | CH₃ | CH₃ | H | 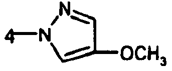 | H | -(CH₂)₂-CHOCH₃-(CH₂)₂- | | H₃C-O-CH₂- | 237 | β |
| 1-1-b-4 | CH₃ | C₂H₅ | H | 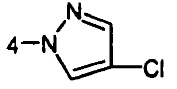 | H | -(CH₂)₂-CHOCH₃-(CH₂)_{2'} | | i-C₃H₇ | 221 | β |
| I-1-b-5 | H | C₂H₅ | H | 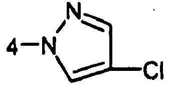 | H | -(CH₂)₂-CHOCH₃-(CH₂)₂- | | i-C₃H₇ | *3.21 (m, 1H, CH OCH₃), 1.05 (d, 6H, CH(CH₃)₂) | β |
| I-1-b-6 | CH₃ | C₂H₅ | H | 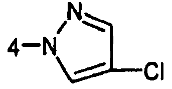 | H | CH₃ | CH₃ | i-C₃H₇ | 140-141 | - |

| | | | | | | | | | | |
|---|---|---|---|---|---|---|---|---|---|---|
| *¹H-NMR (400 MHz, CDCl₃): Verschiebung δ in ppm. | | | | | | | | | | |

### Beispiel I-1-c-1

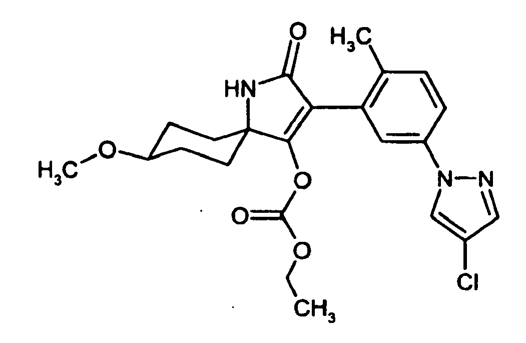

Zu 0,48 g der Verbindung gemäß Beispiel I-1-a-1 in 10 ml wasserfreiem Dichlormethan gibt man 0, 14 ml (1 mmol) Triethylamin bei 10 bis 20°C und 0,1 ml (1 mmol) Chlorameisensäureethylester in 5 ml wasserfreiem Dichlormethan.

Man rührt bei Raumtemperatur unter dünnschichtchromatographischer Kontrolle.

Das Lösungsmittel wird abdestilliert, der Rückstand in Dichlormethan aufgenommen, 2 mal mit 5 ml 0,5 N NaOH-Lösung gewaschen und getrocknet. Das Lösungsmittel wird abdestilliert. Anschließend erfolgt säulenchromatographische Reinigung an Kieselgel (Dichlormethan:Essigsäureethylester 3:1).

Ausbeute: 0,3 g (65 % der Theorie), Fp. 240°C.

In Analogie zu Beispiel (I-1-c-1) und gemäß den allgemeinen Angaben zur Herstellung erhält man folgende Verbindungen der Formel (I-1-c)

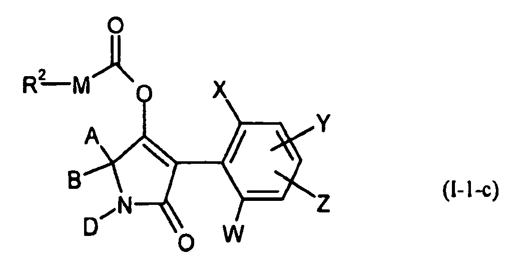

| **Bsp: Nr.** | **W** | **X** | **Y** | **Z** | **D** | **A** | **B** | **M** | **R²** | **Fp.°C** | **Isomer** |
|---|---|---|---|---|---|---|---|---|---|---|---|
| I-1-c-2 | CH₃ | CH₃ | H | 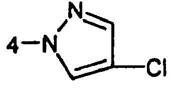 | H | CH₃ | CH₃ | O | C₂H₅ | 159 | - |
| I-1-c-3 | CH₃ | CH₃ | H | 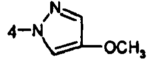 | H | -(CH₂)₂-CHOCH₃-(CH₂)₂- | | O | C₂H₅ | 209-212 | β |
| I-1-c-4 | H | C₂H₅ | H | 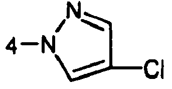 | H | -(CH₂)₂-CHOCH₃-(CH₂)₂- | | O | C₂H₅ | 192-197 | β |
| I-1-c-5 | CH₃ | CH₃ | H | 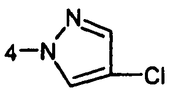 | H | -(CH₂)₂-CHOCH₃-(CH₂)₂- | | O | C₂H₅ | *1.13 (t, 3H, CH₂-CH₃) 2.28 (s, 6H, Ar-CH₃) 7.62, 7.88 (2s, 2H, Pyr-H) | β |
| I-1-c-6 | CH₃ | C₂H₅ | H | 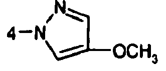 | H | (CH₂)₂-CHOCH₃-(CH₂)_{2'} | | O | C₂H₅ | 220 | β |
| I-1-c-7 | CH₃ | C₂H₅ | H | 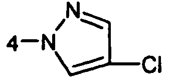 | H | -(CH₂)₂-CHOCH₃-(CH₂)₂- | | O | C₂H₅ | 197 | β |
| I-1-c-8 | C₂H₅ | Cl | H | 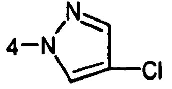 | H | CH₃ | CH₃ | O | C₂H₅ | 178 | |
| I-1-c-9 | C₂H₅ | Cl | H | 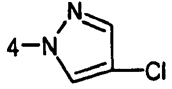 | H | -(CH₂)₂-CHOCH₃-(CH₂)₂- | | O | C₂H₅ | 196 | β |
| I-1-c-10 | C₂H₅ | C₂H₅ | H | 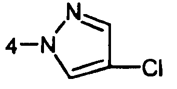 | H | -(CH₂)₂-CHOCH₃-(CH₂)₂- | | O | C₂H₅ | 190 | β |
| I-1-c-11 | CH₃ | C₂H₅ | H | 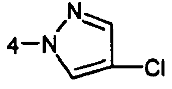 | H | CH₃ | CH₃ | O | C₂H₅ | 149-151 | - |
| I-1-c-12 | CH₃ | C₂H₅ | H | 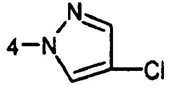 | H | 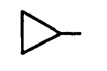 | CH₃ | O | C₂H₅ | - | - |

| | | | | | | | | | | | |
|---|---|---|---|---|---|---|---|---|---|---|---|
| * ¹H-NMR (400 MHz, CDCl₃): Verschiebung δ in ppm. | | | | | | | | | | | |

### Beispiel II-16

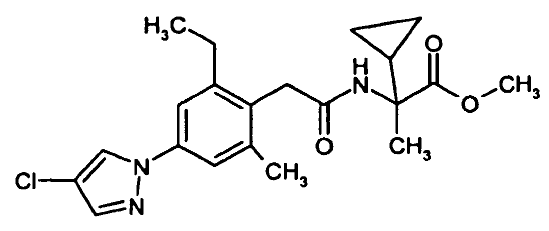

Zu 1 ml Schwefelsäure gibt man 1,29 g der Verbindung gemäß Beispiel XXX-1 in 10 ml Dichlormethan. Es wird 2 h bei 35°C gerührt. Man gibt 6 ml Methanol zu. Es wird 6 h bei 60°C gerührt. Es wird mit. Dichlormethan extrahiert, über Magnesiumsulfat getrocknet und das Lösungsmittel abdestilliert. Es erfolgt säulenchromatographische Reinigung an Kieselgel (Essigsäureethylester → n-Heptan : 1/4 → 1/1).

Ausbeute: 1 g (69 % der Theorie), Fp. 136-137C.

### Beispiel II-1

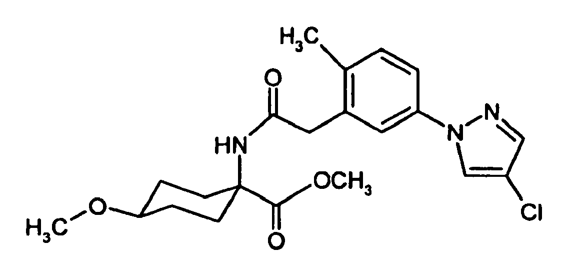

Zu 3,04 g 1-Amino-4-methoxy-cyclohexan-carbonsäuremethylester x HCl in 40 ml wasserfreiem Tetrahydrofuran (THF) gibt man 4 ml Triethylamin und rührt 5 min. Dann gibt man 2,51 g 2-Methyl-5-[1-(4-chlor)-pyrazolyl]-phenylessigsäure zu und rührt 15 min bei Raumtemperatur. Anschließend werden 2,2 ml Triethylamin zugegeben und sofort 0,56 ml Phosphoroxychlorid so zugetropft, dass die Lösung mäßig siedet. Man rührt 30 min unter Rückfluss.

Die Reaktionslösung gießt man in 200 ml Eiswasser, stellt mit 3,5 ml Triethylamin alkalisch, extrahiert mit Dichlormethan und trocknet. Anschließend wird das Lösungsmittel abdestilliert und es erfolgt säulenchromatographische Reinigung an Kieselgel (Dichlormethan:Essigsäureethylester 3:1).

Ausbeute: 3,1 g (75 % der Theorie). Fp.: 153°C.

In Analogie zu Beispiel (II-1) und gemäß den allgemeinen Angaben zur Herstellung erhält man folgende Verbindungen der Formel (II)

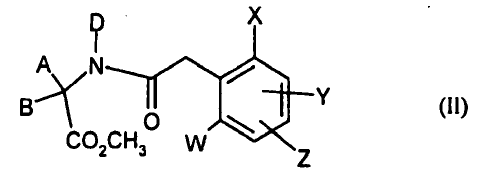

| **Bsp.-Nr.** | **W** | **X** | **Y** | **Z** | **D** | **A** | **B** | **R⁸** | **Fp.°C** | **Isomer** |
|---|---|---|---|---|---|---|---|---|---|---|
| II-2 | CH₃ | CH₃ | H | 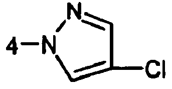 | H | -(CH₂)₂-CHOCH₃-(CH₂)₂- | | | 176 | β |
| II-3 | H | CH₃ | H | 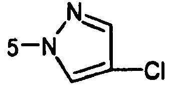 | H | -(CH₂)₅- | | | 146 | - |
| II-4 | CH₃ | CH₃ | H | 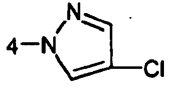 | H | CH₃ | CH₃ | | 194 | - |
| II-5 | CH₃ | CH₃ | H | 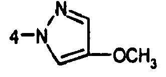 | H | -(CH₂)₂-CHOCH₃-(CH₂)₂- | | CH₃ | 314 | β |
| II-6 | CH₃ | C₂H₅ | H | 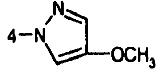 | H | -(CH₂)₂-CHOCH₃-(CH₂)₂- | | CH₃ | 128 | β |
| II-7 | CH₃ | C₂H₅ | H | 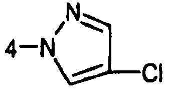 | H | -(CH₂)₂-CHOCH₃-(CH₂)₂- | | CH₃ | 101 | β |
| II-8 | C₂H₅ | C₂H₅ | H | 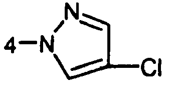 | H | -(CH₂)₂-CHOCH₃-(CH₂)₂- | | CH₃ | 135 | β |
| II-10 | H | C₂H₅ | H | 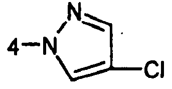 | H | -(CH₂)₂-CHOCH₃-(CH₂)₂- | | CH₃ | 134 | β |
| II-11 | C₂H₅ | Cl | H | 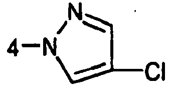 | H | CH₃ | CH₃ | CH₃ | 179 | - |
| II-12 | C₂H₅ | Cl | H | 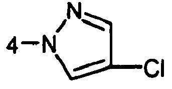 | H | -(CH₂)₂-CHOCH₃-(CH₂)₂- | | CH₃ | 163 | β |
| II-13 | CH₃ | C₂H₅ | H | 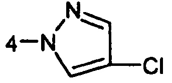 | H | -(CH₂)₂-O(CH₂)₂- | | CH₃ | 172 | - |
| II-14 | CH₃ | C₂H₅ | H | 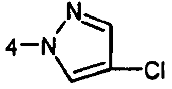 | H | CH₃ | CH₃ | CH₃ | 171 | - |
| II-15 | H | Cl | H | 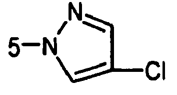 | H | -(CH₂)₂-CHOCH₃-(CH₂)₂- | | CH₃ | 91 | β |
| II-9 | CH₃ | C₂H₅ | H | 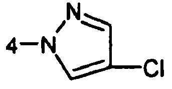 | H | 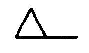 | CH₃ | CH₃ | 136-137 | - |

### Beispiel XXX-1

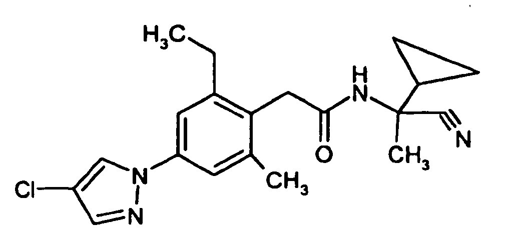

0,38 g 2-Amino-2-cyclopropyl-propionitril und 0,48 ml Triethylamin werden in 20 ml Tetrahydrofuran vorgelegt. Bei 0°C wird 0,97 g 4-(4-Chlor-pyrazolyl)-2-ethyl-6-methylphenylessigsäurechlorid in 20 ml Tetrahydrofuran über 1 h zugetropft. Bei Raumtemperatur wird 6 h nachgerührt. Die Reaktionslösung wird über eine Fritte abgesaugt, gewaschen und das Lösungsmittel abdestilliert. Es erfolgt säulenchromatographische Reinigung an Kieselgel (Essigsäureethylester → n-Heptan = 1/4 → 1/1).

Ausbeute: 910 mg (62 % d Theorie) Fp. 65-70°C

### Beispiel I-2-a-1

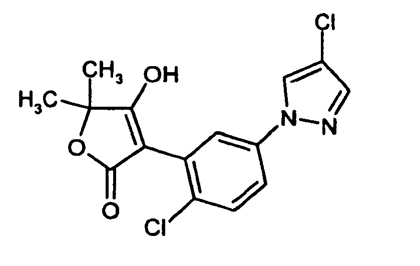

1 g KOtBu werden in 10 ml DMF vorgelegt und auf 0°C abgekühlt, 2,1 g der Verbindung gemäß Beispiel III-1 werden, in DMF gelöst und bei 0 - 10°C zugetropft. Man rührt 8 h bei Raumtemperatur. Das Lösungsmittel wird abdestilliert und der Rückstand in Wasser aufgenommen. Es erfolgt Extraktion mit Essigsäureethylester. Die wässrige Phase wird mit HCl angesäuert, der Niederschlag wird abgesaugt und das Filtrat getrocknet.

Ausbeute 0,25 g (10 % d. Theorie)
¹H-NMR (400 MHz, d₆-DMSO): δ = 1,50 (s, 6H, C(CH₃)₂), 7,5 -8,9 (m, 5H, Ar-H Pyrazol-1H) ppm

In Analogie zu Beispiel (I-2-a-1) und gemäß den allgemeinen Angaben zur Herstellung erhält man folgende Verbindungen der Formel (I-2-a)

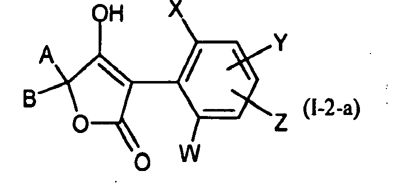

| **Bsp.-Nr.** | **W** | **X** | **Y** | **Z** | **A** | **B** | **Fp.°C** |
|---|---|---|---|---|---|---|---|
| I-2-a-2 | H | Cl | H | 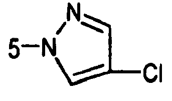 | -(CH₂)₂-CHOCH₃-(CH₂)₂- | | *3,3-3,5 (2 m, 1H, CHOCH₃) 3.3 (s, 3H, OCH₃) 7.73, 7.88 (2s, 2H, Ar-H) 7.90,8.76 (2 s, 2H, Pyr-H) |
| I-2-a-3 | C₂H₅ | Cl | H | 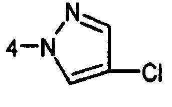 | CH₃ | CH₃ | *7.72, 7.82 (2 s, 2H, Ar-H) 7.90, 8.87 (2 s, 2H, Pyr-H) |
| I-2-a-4 | C₂H₅ | Cl | H | 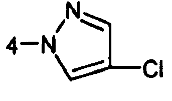 | -(CH2)4 | | *7.58, 7.67 (2 s, 2H, Ar-H) 7.68, 8.23 (2 s, 2H, Pyr-H) |
| I-2-a-5 | C₂H₅ | Cl | H | 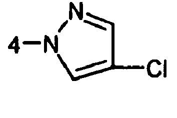 | -(CH₂)₂-CHOCH₃-(CH₂)₂- | | trans-Isomer¹⁾ *3.27 (s, 3H, 0-C^{H}3) 7.71, 7.82 (2 s, 2H, Ar-H) 7.90, 8.86 (2 s, 2H, Pyr-H) |
| I-2-a-6 | C₂H₅ | Cl | H | 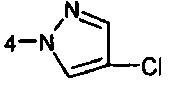 | -(CH₂)₂-CHOCH₃-(CH₂)₂- | | cis-Isomer¹⁾ *3.29 (s, 3H, O-CH₃) 7.72, 7.82 (2 s, 2H, Ar-H) 7.90, 8.87 (2 s, 2H, Pyr-H) |
| I-2-a-7 | CH₃ | C₂H₅ | H | 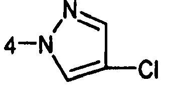 | -(CH₂)₂-O-(CH₂)₂- | | *7.48, 7.54 (2 s, 2H, Ar-H) 7.84, 7.95 (2 s, 2H, Pyr-H) |
| I-2-a-8 | CH₃ | C₂H₅ | H | 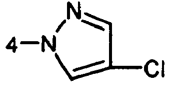 | -(CH₂)₂-CHOCH₃-(CH₂)₂- | | *7.53, 7.55 (2 s, 2H, Ar-H) 7.84, 7.95 (2 s, 2H, Pyr-H) |
| I-2-a-9 | CH₃ | C₂H₅ | H | 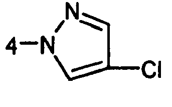 | -(CH₂)₅- | | *7.53, 7.55 (2 s, 2H, Ar-H) 7.83, 7.95 (2 s, 2H, Pyr-H) |
| I-2-a-10 | CH₃ | C₂H₅ | H | 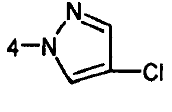 | CH₃ | CH₃ | *7.53, 7.55 (2 s, 2H, Ar-H) 7.84, 7.95 (2 s, 2H, Pyr-H) |

| | | | | | | | |
|---|---|---|---|---|---|---|---|
| *¹H-NMR (400 MHz, d₆-DMSO): δ in ppm ¹⁾ Trennung durch Säulenchromatographie an Kieselgel mit Methylenchlorid als Laufmittel | | | | | | | |

### Beispiel I-2-b-1

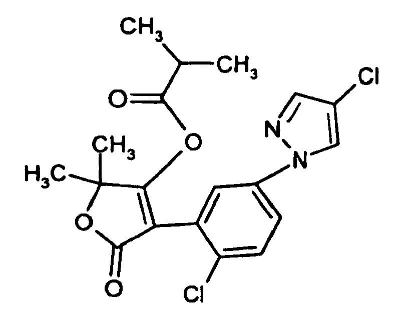

0,12 g der Verbindung gemäß Beispiel I-2-a-1 werden in 10 ml Dichlormethan und 0,05 ml Triethylamin vorgelegt und unter Eiskühlung mit 0,04 g Isobuttersäurechlorid versetzt. Man rührt 8 h bei Raumtemperatur. Dann wäscht man mit 10 % Citronensäure und trennt die Phasen. Es erfolgt Trocknung der organischen Phase und Abdestillation des Lösungsmittels.

Ausbeute: 0,04 g (28 % der Theorie) Fp. 150-152°C
¹H-NMR (CD₃CN): δ= 1.1 (d, 6H), 1.5 (s, 6H), 2.8 (m, 1H), 7.5 - 8.2 (m, 5H) ppm.

In Analogie zu Beispiel (I-2-b-1) und gemäß den allgemeinen Angaben zur Herstellung erhält man folgende Verbindungen der Formel (I-2-b)

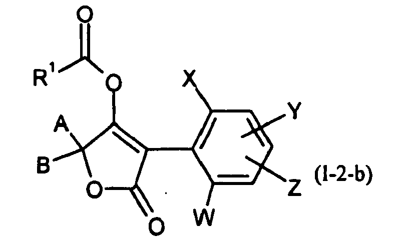

| **Bsp.- Nr.** | **W** | **X** | **Y** | **Z** | **A** | **B** | **R¹** | **Fp.°C** |
|---|---|---|---|---|---|---|---|---|
| I-2-b-2 | C₂H₅ | Cl | H | 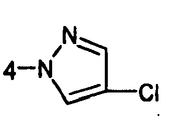 | CH₃ | CH₃ | i-C₃H₇ | *7.64, 7.70 (2s, 2H, Ar-H) 7.71, 8.27 (2 s, 2H, Pyr-H) |
| I-2-b-3 | C₂H₅ | Cl | H | 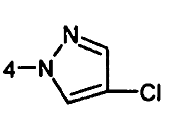 | -(CH₂)₄- | | i-C₃H₇ | *7.63, 7.70 (2 s, 2H, Ar-H) 7.71, 8.27 (2 s, 2H, Pyr-H) |
| I-2-b-4 | C₂H₅ | Cl | H | 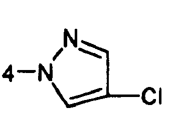 | -(CH₂)₂-CHOCH₃-(CH₂)_{2'} | | i-C₃H₇ | trans-Isomer ¹⁾ *3.31 (s, 3H, O-CH₃) 7.63, 7.70 (2 s, 2H, Ar-H) 7.71,8.27 (2 s, 2H, Pyr-H) |
| I-2-b-5 | C₂H₅ | Cl | H | 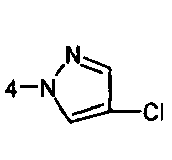 | -(CH₂)₂-CHOCH₃-(CH₂)₂- | | i-C₃H₇ | cis-Isomer¹⁾ *3.34 (s, 3H, O-CH₃) 7.63, 7.70 (2 s, 2H, Ar-H) 7.21, 8.27 (2 s, 2H, Pyr-H) |
| I-2-b-6 | CH₃ | C₂H₅ | H | 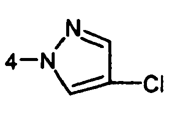 | CH₃ | CH₃ | i-C₃H₇ | *7.48, 7.49 (2 s, 2H, Ar-H) 7.67, 8.23 (2 s, 2H, Pyr-H) |
| I-2-b-7 | CH₃ | C₂H₅ | H | 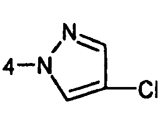 | -(CH₂)₂-O-CH₂)₂- | | i-C₃H₇ | *7.48, 7.50 (2 s, 2H, Ar-H) 7.67, 8.23 (2 s, 2H, Pyr-H) |
| I-2-b-8 | CH₃ | C₂H₅ | H | 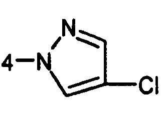 | -(CH₂)₅- | | i-C₃H₇ | *7.47, 7.48 (2 s, 2H, Ar-H) 7.67, 8.22 (2 s, 2H, Pyr-H) |
| I-2-b-9 | CH₃ | C₂H₅ | H | 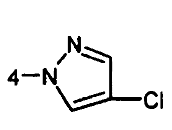 | -(CH₂)₂-CHOCH₃-(CH₂)₂- | | i-C₃H₇ | trans-Isomer ¹⁾ *3.30 (s, 3H, O-CH₃) 7.48, 7.50 (2 s, 2H, Ar-H) 7.67, 8.23 (2 s, 2H, Pyr-H) |
| I-2-b-10 | CH₃ | C₂H₅ | H | 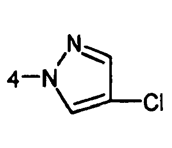 | -(CH₂)₂-CHOCH₃₄CH₂)₂- | | -C₃H₇ | cis-Isomer ¹⁾ *7.48, 7.49 (2 s, 2H, Ar-H) 7.67, 8.22 (2 s, 2H, Pyr-H) |

| | | | | | | | | |
|---|---|---|---|---|---|---|---|---|
| *¹H-NMR (400 MHz, d₆-DMSO): δ in ppm ¹⁾ Trennung durch Säulenchromatographie an Kieselgel mit Methylenchlorid als Laufmittel | | | | | | | | |

### Beispiel III-1

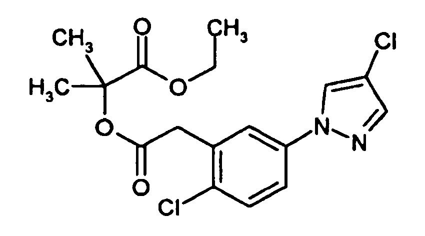

0,9 g Hydroxyisobuttersäureethylester und 2 g 2-Chlor-5-N-(4-chlorpyrazolyl)-phenylessigsäurechlorid werden in 20 ml Toluol 8 h unter Rückfluss gerührt. Das Lösungsmittel wird abdestilliert und der Rückstand getrocknet.

Ausbeute: 2,2 g (83 % d. Theorie)
¹H-NMR (400 MHz, d₆ DMSO): δ = 1.15 (t, 3H, CH₂-CH₃), 1.50 (s, 6H, C(CH₃)₂, 4.05 (q, 2H, O- CH₂-CH₃), 7.3 - 8.8 (m, 5H) ppm.

In Analogie zu Beispiel (III-1) und gemäß den allgemeinen Angaben zur Herstellung erhält man folgende Verbindungen der Formel (III)

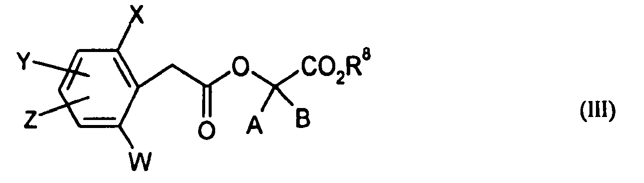

| **Bsp.- Nr.** | **W** | **X** | **Y** | **Z** | **A** | **B** | **R⁸** | **Fp.°C** |
|---|---|---|---|---|---|---|---|---|
| III-2 | H | Cl | H | 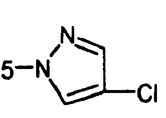 | -(CH₂)₂-CHOCH₃-(CH₂)_{2'} | | C₂H₂ | *7.18, 7.25 (2s, 2H, Ar-H) 7.88, 8.75 (2 s, 2H, Pyr-H) |
| III-3 | C₂H₅ | Cl | H | 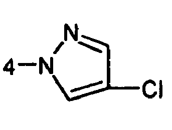 | CH₃ | CH₃ | C₂H₅ | *7.57, 7.67 (2 s, 2H, Ar-H) 7.69, 8.23 (2 s, 2H, Pyr-H) |
| III-4 | C₂H₅ | Cl | H | 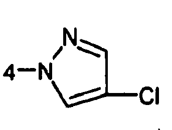 | -(CH₂)₄- | | C₂H₅ | *7.58, 7.67 (2 s, 2H, Ar-H) 7.69, 8.24 (2 s, 2H, Pyr-H) |
| III-5 | C₂H₅ | Cl | H | 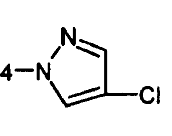 | -(CH₂)₂-CHOCH₃-(CH₂)₂- | | C₂H₅ | *7.58, 7.67 (2 s, 2H, Ar-H) 7.69, 8.24 (2 s, 2H, Pyr-H) |
| III-6 | CH₃ | C₂H₅ | H | 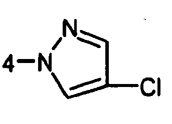 | -(CH₂)₂-O-(CH₂)₂- | | C₂H₅ | *7.44, 7.45 (2 s, 2H, Ar-H) 7.66,8.19(2s, 2H, Pyr-H) |
| III-7 | CH₃ | C₂H₅ | H | 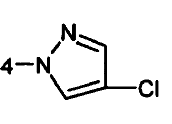 | -(CH₂)₂-CHOCH₃-(CH₂)₂- | | C₂H₅ | *7.43, 7.44 (2 s, 2H, Ar-H) 7.65,8.19(2 s, 2H, Pyr-H) |
| III-8 | CH₃ | C₂H₅ | H | 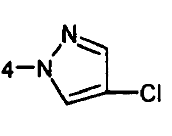 | -(CH₂)₅- | | C₂H₅ | *7.43, 7.45 (2 s, 2H, Ar-H) 7.65, 8.19 (2 s, 2H, Pyr-H) |
| III-9 | CH₃ | C₂H₅ | H | 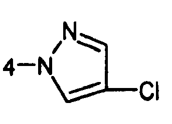 | CH₃ | CH₃ | C₂H₅ | *7.42, 7.43 (2 s, 2H, Ar-H) 7.65, 8.19 (2 s, 2H, Pyr-H) |

### Beispiel XXV-1

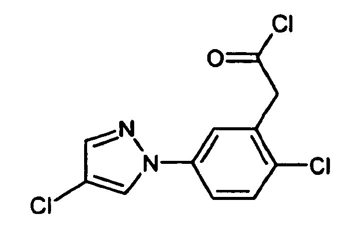

Zu 4 g 2-Chlor-5-N-(4-chlor-pyrazolyl)-phenylessigsäure in 100 ml Dichlormethan tropft man bei Raumtemperatur 2,3 g Oxalsäuredichlorid. Es wird 8 h bei Raumtemperatur gerührt. Anschließend wird bis zum Ende der Gasentwicklung unter Rückfluss gekocht. Das Lösungsmittel wird abdestilliert und der Rückstand entgast.

Ausbeute: 4,25 g (99 % d. Theorie), GC/MS: M⁺ 285 m/e.

### Beispiel I-6-a-1

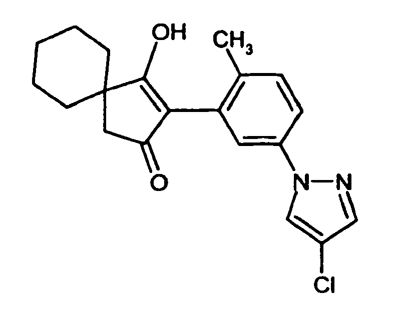

1,73 g (15,4 mmol) Kalium-tert.-buytlat werden in 20 ml DMF vorgelegt und mit 3,0 g (7,7 mmol) der Verbindung gemäß Beispiel VIII-1 in 10 ml DMF versetzt. Man rührt 3 Stunden bei 50°C. Die Reaktionslösung wird abgekühlt, auf 600 ml kalte 1 N HCl gegeben, der Niederschlag abgesaugt und getrocknet.

Ausbeute: 2,4 g (87 % der Theorie). Fp.: > 250°C

In Analogie zu Beispiel (I-6-a-1) und gemäß den allgemeinen Angaben zur Herstellung erhält man folgende Verbindungen der Formel (I-6-a)

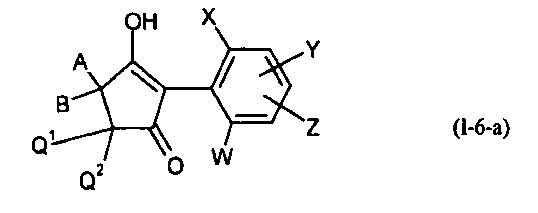

| **Bsp.-Nr.** | **W** | **X** | **Y** | **Z** | **A** | **B** | **Q¹** | **Q²** | **Fp.°C** |
|---|---|---|---|---|---|---|---|---|---|
| I-6-a-2 | CH₃ | CH₃ | H | 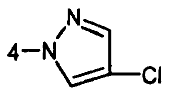 | -(CH₂)₅- | | H | H | 223-230 |

### Beispiel I-6-c-1

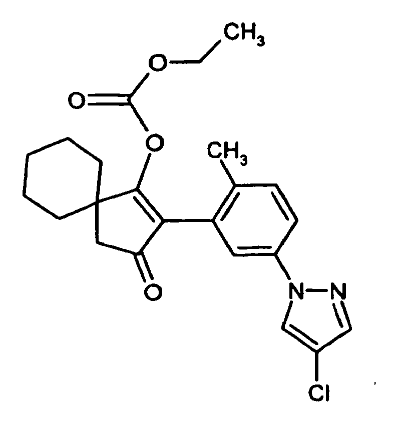

300 mg der Verbindung gemäß Beispiel I-6-a-1 werden in 5 ml wasserfreiem Aceton vorgelegt, mit 0,174 g Kaliumcarbonat versetzt. Dann werden 0,119 g Chlorameisensäureethylester zugegeben. Man rührt 3 Stunden bei 50°C.

Die Reaktionslösung wird eingeengt, in 10 ml CH₂Cl₂ aufgenommen und mit 10 ml H₂O gewaschen. Die organische Phase wird abgetrennt und das Lösungsmittel abdestilliert.

Ausbeute: 0,36 g (96 % der Theorie). Fp.: 132°C.

In Analogie zu Beispiel (I-6-c-1) und gemäß den allgemeinen Angaben zur Herstellung erhält man folgende Verbindungen der Formel (I-6-c)

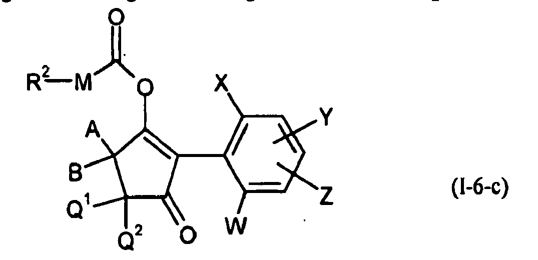

| **Bsp.- Nr.** | **W** | **X** | **Y** | **Z** | **A** | **B** | **Q¹** | **Q²** | **M** | **R²** | **Fp.°C** |
|---|---|---|---|---|---|---|---|---|---|---|---|
| I-6-c-2 | H | CH₃ | H | 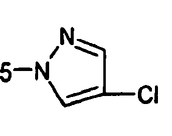 | -(CH₂)₅- | | H | H | O | i-C₃H₇ | Wachs * 1.19, (d, 6H, CH(CH₃)₂), 4.77 (m, 1H, O-CH) |
| I-6-c-3 | H | CH₃ | H | 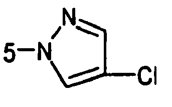 | -(CH₂)₅- | | H | H | S | i-C₄H₉ | Wachs *2.09, (s, 3H, Ar-CH₃) 8.75 (s, 1H, Pyr-H) |
| I-6-c-4 | H | CH₃ | H | 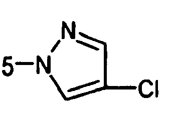 | -(CH₂)₅- | | H | H | O | C₆H₅-CH₂ | Wachs *2.05, (s, 3H, Ar-CH₃) 5.10 (s, 2H, O-CH₂) 7.29-7.37 (m, 6H, Ar-H) |

| | | | | | | | | | | | |
|---|---|---|---|---|---|---|---|---|---|---|---|
| *¹H-NMR (400 MHz, CDCl₃): δ in ppm | | | | | | | | | | | |

### Beispiel VIII-1

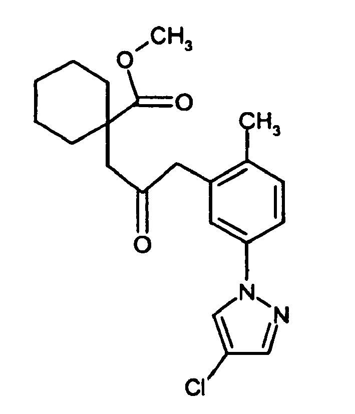

Zu 8,6 g (22,9 mmol) Rohprodukt gemäß Beispiel XXXV-1 in 100 ml wasserfreiem Aceton, gibt man 3,2 g (22,9 mmol) Kaliumcarbonat und 8,1 g (57,3 mmol) = 3,6 ml Methyliodid. Es wird 16 Stunden unter Rückfluss gerührt.

Die Reaktionslösung wird abgekühlt, der Niederschlag abgesaugt und mit Aceton gewaschen.

Es erfolgt säulenchromatographische Reinigung an Kieselgel (Dichlormethan:Petrolether, 2:1→ 4:1 → 8:1 → Dichlormethan).

Ausbeute: 3 g (34 % der Theorie).
1H-NMR (400 MHz , d₆-DMSO): δ = 1.30-1.78 (m,10H, Cyclohexyl-H), 2.10 (s, 3H, CH₃-Aryl), 3.51 (s, 3H, CO₂Me), 7.28 (d, 1H, Aryl-H), 7.54-7.60 (m, 2H, Aryl-H), 7.82, 8.72 (2s, je 1H, 2 Pyrazolyl-H) ppm

In Analogie zu Beispiel (VIII-1) und gemäß den allgemeinen Angaben zur Herstellung erhält man folgende Verbindungen der Formel (VIII)

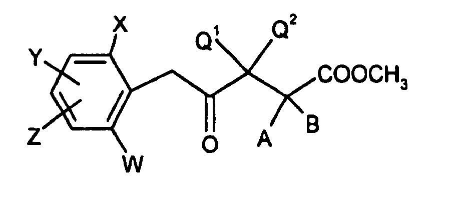

| **Bsp.-Nr.** | **W** | **X** | **Y** | **Z** | **A** | **B** | **Q¹** | **Q²** | **Fp.°C** |
|---|---|---|---|---|---|---|---|---|---|
| VIII-2 | CH₃ | CH₃ | H | 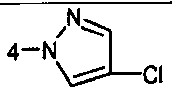 | -(CH₂)₅- | | H | H | |

### Beispiel XXXV-1

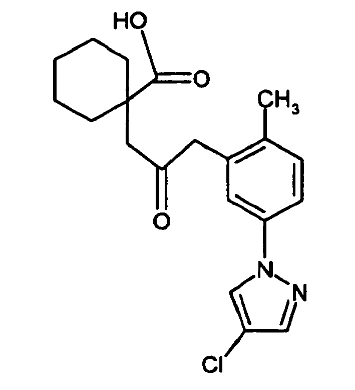

Zu einer Lösung von 9,44 ml LDA-Lösung (2 molar) in 30 ml wasserfreiem THF, wird eine Lösung von 5,0 g (18,9 mmol) 2-Methyl-5-[1-(4-chlorpyrazolyl)-phenyl]-essigsäure-methylester in 10 ml THF bei -15°C zugetropft und 60 min bei 0°C gerührt.

Dann wird bei -15°C eine Lösung von 4,13 g (18,9 mmol) 3,3-Pentamethylen-bernsteinsäuremethylester-chlorid in 10 ml wasserfreiem THF und gleichzeitig 14,2 ml LDA-Lösung (2,0 molar, 1,5 eq) zugetropft. Der Ansatz wird zwei Stunden bei Raumtemperatur gerührt und dann auf 150 ml eiskalte 10 %ige Ammoniumchlorid-Lösung gegeben.

Das Zwischenprodukt wird mit MTB-Ether extrahiert, die Lösungsmittel abdestilliert. Der Rückstand wird 3 Stunden mit 10 g KOH und 100 ml Wasser unter Rückfluss gekocht.

Die Reaktionslösung wird abgekühlt, mit konzentrierter HCl angesäuert und mit 200 ml CH₂Cl₂ extrahiert, getrocknet und das Lösungsmittel abdestilliert.

Ausbeute: 8,6 g (66,8 % der Theorie).

In Analogie zu Beispiel (XXXV-1) und gemäß den allgemeinen Angaben zur Herstellung erhält man folgende Verbindungen der Formel (XXXV)

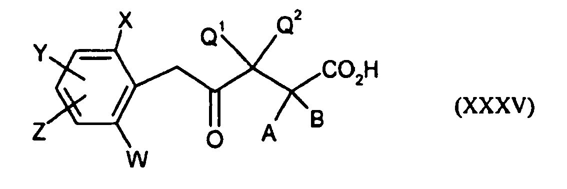

| **Bsp.-Nr.** | **W** | **X** | **Y** | **Z** | **A** | **B** | **Q¹** | **Q²** | **Fp.°C** |
|---|---|---|---|---|---|---|---|---|---|
| XXXV-2 | CH₃ | CH₃ | H | 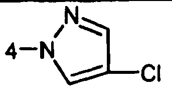 | -(CH₂)₅- | | H | H | * |

| | | | | | | | | | |
|---|---|---|---|---|---|---|---|---|---|
| * wurde als Rohprodukt direkt zu Verbindung der Formel (VIII-2) umgesetzt | | | | | | | | | |

### Beispiel I-7-a-1

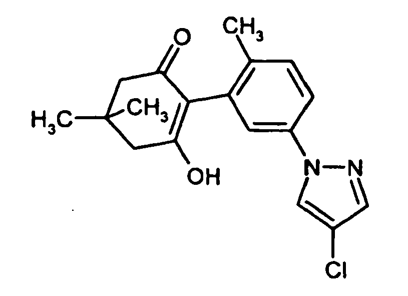

Zu 0,49 g (4,4 mmol, 2,0 eq) Kalium-tert.-butylat in 5 ml DMF gibt man 0,80 g (2,2 mmol) der Verbindung gemäß Beispiel IX-1 in 2 ml DMF.

Man rührt 3 Stunden bei 50°C. Man gibt 20 ml Eiswasser zu und füllt mit kalter 1 N HCl-Lösung auf 250 ml auf und extrahiert mit Dichlormethan. Die organische Phase wird getrocknet und eingeengt.

Es erfolgt säulenchromatographische Reinigung des Rückstandes an Kieselgel (Petrolether:Essigsäureethylester 2:1).

Ausbeute: 0,15 g (21 % der Theorie). Fp.: 172°C

In Analogie zu Beispiel (I-7-a-1) und gemäß den allgemeinen Angaben zur Herstellung erhält man folgende Verbindungen der Formel (I-7-a)

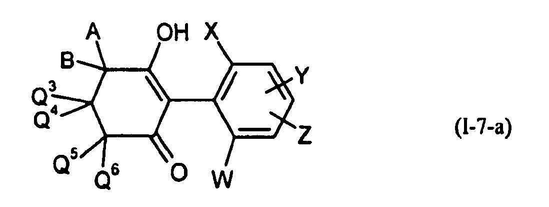

| **Bsp.-Nr.** | **W** | **X** | **Y** | **Z** | **A** | **B** | **Q³** | **Q⁴** | **Q⁵** | **Q⁶** | **Fp.°C** |
|---|---|---|---|---|---|---|---|---|---|---|---|
| I-7-a-2 | H | CH₃ | H | 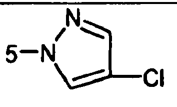 | H | H | -(CH₂)₄- | | H | H | 241 |

### Beispiel IX-1

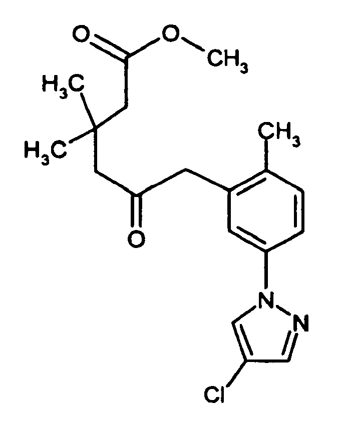

Zu 4,0 g Rohprodukt gemäß Beispiel XXXIX-1 in 50 ml wasserfreiem Aceton gibt man 1,58 g Kaliumcarbonat und 4,07 g (2,5 eq) = 1,79 ml Methyliodid. Es wird 16 Stunden unter Rückfluss gerührt. Die Reaktionslösung wird abgekühlt, der Niederschlag abgesaugt und mit Aceton gewaschen.

Es erfolgt säulenchromatographische Reinigung an Kieselgel mit einem Gradienten (Methylenchlorid:Essigsäureethylester 50:1 → 5:1).

Ausbeute: 0,8 g (15 % der Theorie).
1H-NMR (400 MHz, d₆-DMSO): δ = 1.03 (s, 6H, CH₃), 2.18 (s, 3H, CH₃-Aryl), 3.57 (s, 3H, CO₂Me) 7.30 (d, 1H, Aryl-H), 7.57-7.61 (m, 2H, Aryl-H), 7.83, 8.72 (2s, je 1H, 2 Pyrazolyl-H) ppm

In Analogie zu Beispiel (IX-1) und gemäß den allgemeinen Angaben zur Herstellung erhält man folgende Verbindungen der Formel (IX)

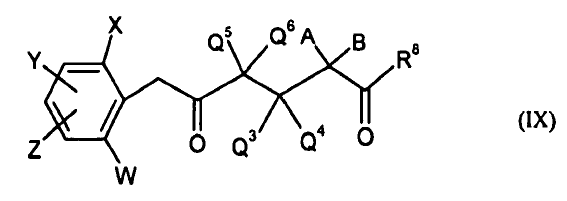

| **Bsp.-Nr.** | **W** | **X** | **Y** | **Z** | **A** | **B** | **Q³** | **Q⁴** | **Q⁵** | **Q⁶** | **R⁸** | **Fp.°C** |
|---|---|---|---|---|---|---|---|---|---|---|---|---|
| IX-2 | H | CH₃ | H | 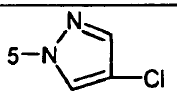 | H | H | -(CH₂)₄- | | H | H | CH₃ | * |

| | | | | | | | | | | | | |
|---|---|---|---|---|---|---|---|---|---|---|---|---|
| * 1H-NMR (δ₆-400 MHz DMSO): δ = 1.05-1.50 (m, 8H, Cyclopentyl-H), 2.13 (s, 3H, CH₃-Aryl), 3.52 (s, 3H, CO₂Me), 7.28 (d, 1H, Aryl-H), 7.82, 8.70 (2s, je 1H, 2 Pyrazolyl-H) ppm | | | | | | | | | | | | |

### Beispiel XXXIX-1

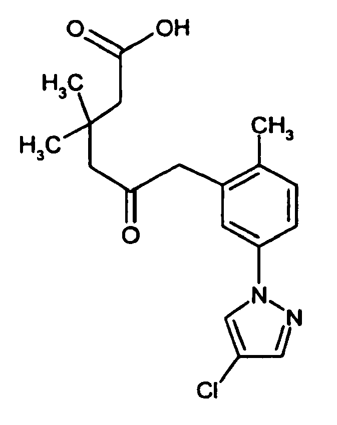

Zu einer Lösung von 5,7 ml LDA-Lösung (2 molar; 1,0 eq) in 20 ml wasserfreiem THF wird eine Lösung von 3,0 g (11,3 mmol; 1 eq) 2-Methyl-5-[1-(4-chlorpyrazolyl)-phenyl]-essigsäuremethylester in 5 ml wasserfreiem THF bei -15°C zugetropft und 60 min bei 0°C gerührt.

Dann wird bei -15°C eine Lösung von 1,61 g (11,3 mmol; 1,0 eq) 3,3-Dimethylglutarsäureanhydrid in 10 ml wasserfreiem THF und gleichzeitig 8,52 ml LDA-Lösung (2,0 molar; 1,5 eq) zugetropft. Der Ansatz wird 2 Stunden bei Raumtemperatur gerührt und dann auf 150 ml eiskalte 10 %ige Ammoniumchlorid-Lösung gegeben. Es wird mit konzentrierter HCl angesäuert.

Das Zwischenprodukt wird mit MTBE extrahiert und die Lösungsmittel abdestilliert. Der Rückstand wird 4 Stunden mit 7 g KOH und 70 ml Wasser unter Rückfluss gekocht.

Die Reaktionslösung wird abgekühlt, mit konzentrierter HCl angesäuert und mit 200 ml MTBE extrahiert, getrocknet und das Lösungsmittel abdestilliert.

Ausbeute: 4 g.

In Analogie zu Beispiel (XXXIX-1) und gemäß den allgemeinen Angaben zur Herstellung erhält man folgende Verbindungen der Formel (XXXIX)

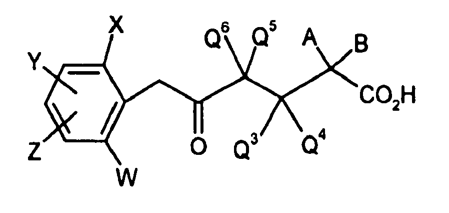

| **Bsp.-Nr.** | **W** | **X** | **Y** | **Z** | **A** | **B** | **Q³** | **Q⁴** | **Q⁵** | **Q⁶** | **Fp.°C** |
|---|---|---|---|---|---|---|---|---|---|---|---|
| XXXIX-2 | H | CH₃ | H | 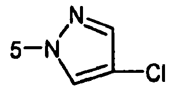 | H | H | -(CH₂)₄- | | H | H | * |

| | | | | | | | | | | | |
|---|---|---|---|---|---|---|---|---|---|---|---|
| * Die Verbindung wurde als Rohprodukt zur Herstellung von Bsp.-Nr.IX-2 eingesetzt | | | | | | | | | | | |

### Beispiel I-8a-1

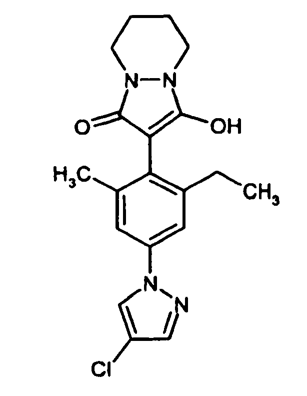

0,85 g Kalium-tert.-butylat werden in 36 ml N,N-Dimethylacetamid vorgelegt, 1,6 g der Verbindung gemäß Beispiel (XII-1) in N,N-Dimethylacetamid werden langsam bei 60°C zugetropft und 1 Stunde nachgerührt. Nach Abkühlen wird die Lösung in eisgekühlte Salzsäure eingetropft und der Niederschlag abgesaugt.

Ausbeute: 1,1 g (81 % d. Theorie). Fp. 258°C

In Analogie zu Beispiel (I-8-a-1) und gemäß den allgemeinen Angaben zur Herstellung erhält man folgende Verbindungen der Formel (I-8-a)

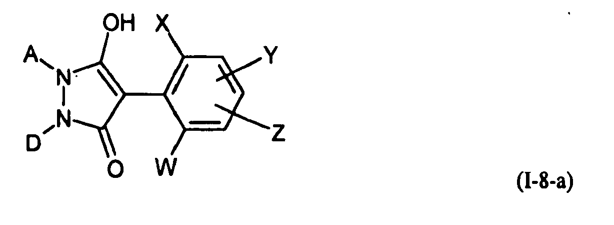

| **Bsp:-Nr.** | **W** | **X** | **Y** | **Z** | **A** | **D** | **Fp.°C** |
|---|---|---|---|---|---|---|---|
| I-8-a-2 | CH₃ | C₂H₅ | H | 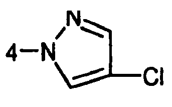 | -(CH₂)₂-O-(CH₂)₂- | | >300 |

### Beispiel I-8-b-7

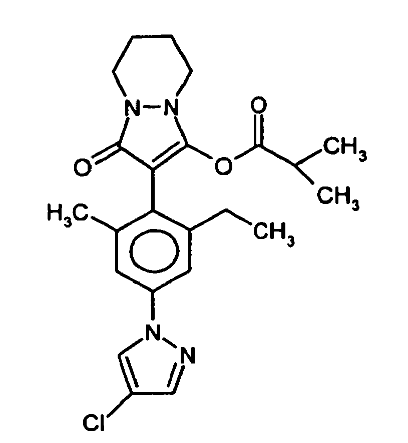

0,2 g der Verbindung gemäß Beispiel I-8-a-1 wird in 30 ml Dichlormethan vorgelegt mit 0,067 g Triethylamin versetzt und anschließend 0,054 g 2-Methylpropionylchlorid zugegeben und 3 h nachgerührt.

Man verdünnt mit Wasser und extrahiert. Die organische Phase wird getrocknet und das Lösungsmittel abdestilliert. Zum Rückstand gibt man n-Heptan und wenig Dichlormethan. Das Produkt kristallisiert aus und wird abgesaugt.

Ausbeute: 0,19 g (81% der Theorie), Fp: 161,4°C

In Analogie zu Beispiel (I-8-b-1) und gemäß den allgemeinen Angaben zur Herstellung erhält man folgende Verbindungen der Formel (1-8-b)

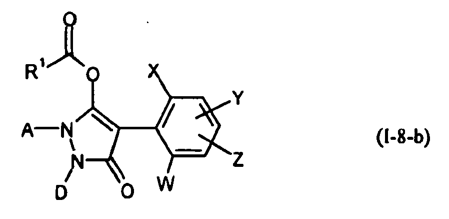

| **Bsp.- Nr.** | **W** | **X** | **Y** | **Z** | **A** | **D** | **R¹** | **Fp.°C** |
|---|---|---|---|---|---|---|---|---|
| I-8-b-2 | CH₃ | C₂H₅ | H | 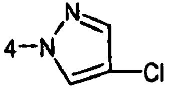 | -(CH₂)₂-O-(CH₂)₂- | | i-C₃H₇ | 138,4 |
| I-8-b-3 | CH₃ | C₂H₅ | H | 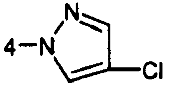 | -(CH₂)₂-O-(CH₂)₂ | | CH₂-OCH₃ | 252,6 |
| I-8-b-4 | CH₃ | C₂H₅ | H | 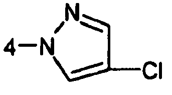 | -(CH₂)₂-O-(CH₂)₂ | | t-C₄H₉ | 119,1 |
| I-8-b-5 | CH₃ | C₂H₅ | H | 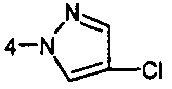 | -(CH₂)₄- | | CH₂-OCH₃ | 259 |
| I-8-b-6 | CH₃ | C₂H₅ | H | 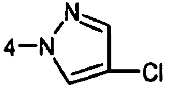 | -(CH₂)₄- | | t-Bu | 204,8 |

### Beispiel I-8-c-1

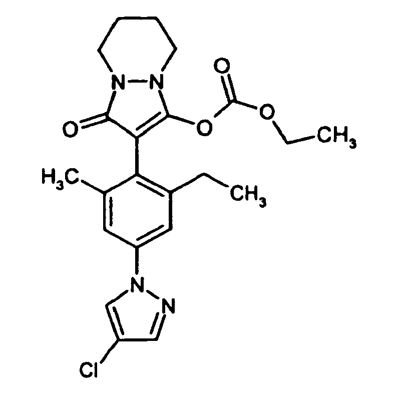

0,25 g der Verbindung gemäß Beispiel (I-8-a-1) werden in 36 ml Dichlormethan vorgelegt, mit 0,12 ml Triethylamin versetzt, bei Raumtemperatur 0,06 ml Chlorameisensäureethylester in Dichlormethan gelöst zugetropft und 1 h nachgerührt. Es wird anschließend mit Wasser verdünnt und extrahiert. Die organische Phase wird getrocknet und das Lösungsmittel abdestilliert. Zum Rückstand gibt man n-Heptan und wenig Dichlormethan. Das Produkt kristallisiert aus und wird abgesaugt.

Ausbeute: 0.2 g (71% d. Theorie). Fp. 172°C

In Analogie zu Beispiel (I-8-b-1) und gemäß den allgemeinen Angaben zur Herstellung erhält man folgende Verbindungen der Formel (I-8-c-1)

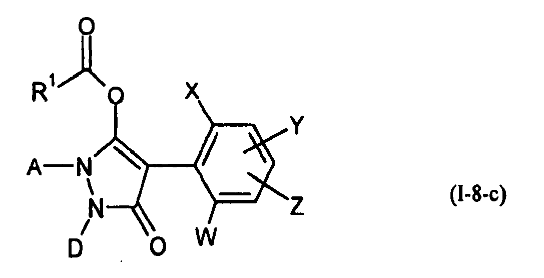

| **Bsp.- Nr.** | **W** | **X** | **Y** | **Z** | **A** | **D** | **M** | **R**² | **Fp.°C** |
|---|---|---|---|---|---|---|---|---|---|
| I-8-c-2 | CH₃ | C₂H₅ | H | 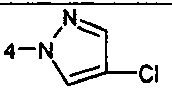 | -(CH₂)₂-O-(CH₂)₂- | | O | C₂H₅ | 230 |

### Beispiel (XII-1)

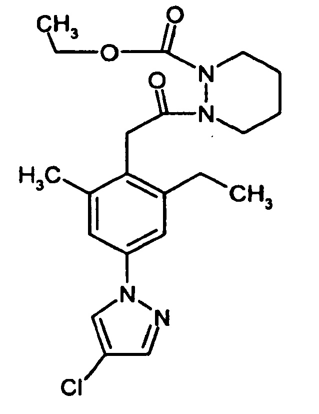

4-([4-Chlor-pyrazolyl)-2-ethyl-6-methyl-phenylessigsäure werden in 35 ml Dichlormethan vorgelegt und 1,28 g Oxalsäuredichlorid zugegeben. Man rührt unter Rückfluss und gibt 1 ml Dimethylformamid zu, wenn die Gasentwicklung abnimmt. Man rührt weiter unter Rückfluss und kühlt anschließend unter Schutzgasatmosphäre ab. Das Lösungsmittel wird abdestilliert. Der Rückstand wird in Dichlormethan aufgenommen und zu einer Lösung aus 1,2 g 1-Ethoxycarbonylhexahydropyridazin in 35 ml Dichlormethan und 1,6 ml Triethylamin getropft. Man rührt 3 h bei Raumtemperatur und extrahiert anschließend mit Wasser und Dichlormethan. Die organische Phase wird abgetrennt, getrocknet und das Lösungsmittel abdestilliert.

Ausbeute: 2 g (63 % d. Theorie).
¹H-NMR-Daten (300 MHz, CDCl₃): δ=2,6 (q, 2H, Ar-CH₂CH₃), 4,3 (q, 2H, O-CH₂CH₃), 7,3, 7,35 (2s, 2H, ArH), 7,6, 7,9 (2s, 2H, Pyr H) ppm

In Analogie zu Beispiel (XII-1) und gemäß den allgemeinen Angaben zur Herstellung erhält man folgende Verbindungen der Formel (XII)

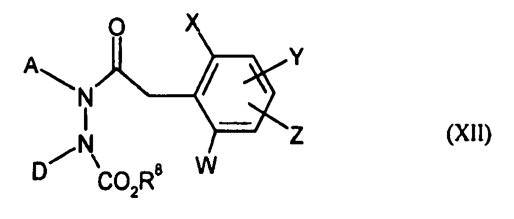

| **Bsp.- Nr.** | **W** | **X** | **Y** | **Z** | **A** | **D** | **R⁸** | **Fp.°C** |
|---|---|---|---|---|---|---|---|---|
| XII-2 | CH₃ | C₂H₅ | H | 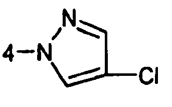 | -(CH₂)₂-O-(CH₂)₂- | | C₂H₅ | 87 |

### Beispiele nach Verfahren Q

### Beispiel XXXII-1

### (4-N-[4-Chlorpyrazolyl]-2,6-dimethyl)phenylessigsäuremethylester

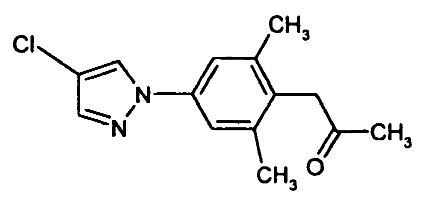

16,6 g (162 mmol) 4-Chlorpyrazol, 10,3 g (54 mmol) Kupfer(I)iodid und 56 g (405 mmol) Kaliumcarbonat (trocken) werden unter Argonatatmosphäre in 350 ml absolutem DMF vorgelegt und 5 min gerührt. Anschließend werden 34,7 g (135 mmol) (4-Brom-2,6-dimethyl)phenylessigsäuremethylester langsam zugetropft. Die Reaktionsmischung wird bei 105°C über vier Tage gerührt. Dabei wird der Reaktionsverlauf per GC kontrolliert und nach jeweils 24 Stunden (insgesamt dreimal) 2,6 g (13,5 mmol) Kupfer(I)iodid und 4,15 g (40,5 mmol) 4-Chlorpyrazol zugesetzt. Nach dem Abkühlen der Reaktionsmischung wird das Lösungsmittel im Vakuum entfernt, der Rückstand über eine kurze Fritte mit Kieselgel filtriert und anschließend chromatographisch aufgereinigt.

Ausbeute: 17,2 g (46 %).
¹H-NMR {400 MHz, CDCl₃}: 2.38 (s, 6H, CH₃); 3.70 (s, 3H, OCH₃); 3.71 (s, 2H, CH₂); 7.31 (s, 2H, Ph-H); 7.61 (s, 1H, Pyrazolyl-H); 7.88 (s, 1H, Pyrazolyl-H).

In Analogie zu Beispiel (XXXII-1) und gemäß den allgemeinen Angaben zur Herstellung erhält man folgende Verbindungen der Formel (XXXII).

### Beispiel XXXII-2

### (2-Ethyl-4-N-[4-methoxypyrazolyl]-6-methyl)phenylessigsäuremethylester

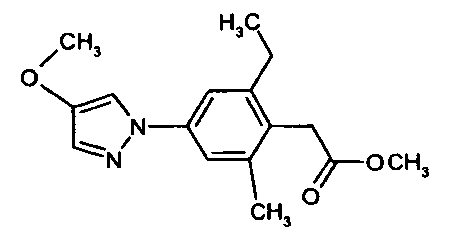

¹H-NMR {400 MHz, DMSO-d₆}: 1.15 (t,³J_{HH}= 7 Hz, 3H, CH₃); 2.28 (s, 3H, CH₃); 2.64 (q, ³J_{HH}= 7 Hz, 2H, CH₂); 3.62 (s, 3H, OCH₃); 3.73 (s, 2H, CH₂); 3.76 (s, 3H, OCH₃); 7.46 (m, 2H, Ph-H); 7.50 (s, 1H, Pyrazolyl-H); 8.24 (s, 1H, Pyrazolyl-H).
MS/CI: 289 (M+1).

### Beispiel XXXII-3

### (2,6-Dimethyl-4-N-[4-methoxypyrazolyl])phenylessigsäuremethylester

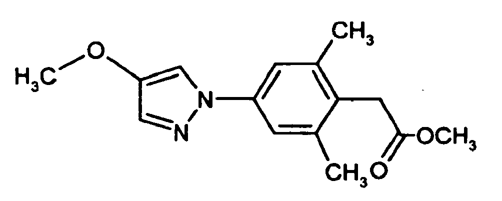

¹H-NMR {400 MHz, DMSO-d₆}: 2.30 (s, 6H, CH₃); 3.62 (s, 3H, OCH₃); 3.71 (s,2H, CH₂); 3.76 (s, 3H, OCH₃); 7.46 (s, 2H, Ph-H); 7.50 (s, 1H, Pyrazolyl-H); 8.22 (s, 1H, Pyrazolyl-H).
MS/CI: 275 (M+1).

### Beispiel XXXII-4

### (2,6-Diethyl-4-N-[4-chlorpyrazolyl])phenylessigsäuremethylester

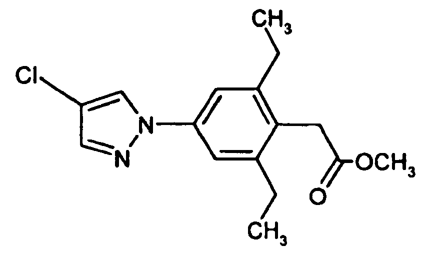

¹H-NMR {400 MHz, DMSO-d₆}: 1.18 (t, ³J_{HH}=7Hz, 6H, CH₃); 2.61 (q, ³J_{HH}= 7 Hz, 4H, CH₂); 3.57 (s, 3H, OCH₃); 3.79 (s, 2H, CH₂); 7.50 (m, 2H, Ph-H); 7.84 (s, 1H, Pyrazolyl-H); 8.79 (s,1H, Pyrazolyl-H).
MS/CI: 307(M+1).

### Beispiel XXXII-5

### (2,6-Dimethyl-4-N-[4-cyanopyrazolyl])phenylessigsäuremethylester

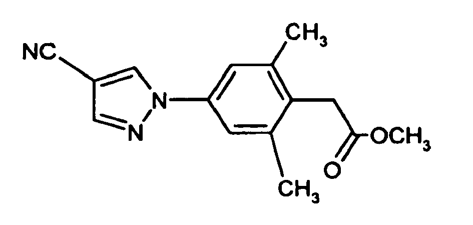

¹H-NMR {400 MHz, DMSO-d₆}: 2.31 (s, 6H, CH₃); 3.62 (s, 2H, CH₂); 3.74 (s, 3H, OCH₃); 7.52 (s, 2H, Ph-H); 8.05 (s, 1H, Pyrazolyl-H); 8.82 (s, 1H, Pyrazolyl-H).
MS/CI: 270 (M+1).

### Beispiel XXXII-6

### (2,6-Dimethyl-4-N-[3-chlortriazolyl])phenylessigsäuremethylester

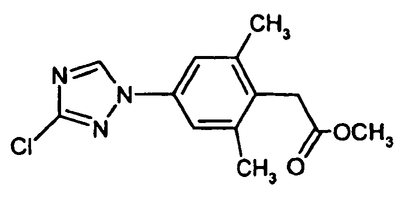

¹H-NMR {400 MHz, DMSO-d₆}: 2.26 (s, 6H, CH₃; 3.55 (s, 2H, CH₂); 3.79 (s, 3H, OCH₃); 7.44 (s, 2H, Ph-H); 9.22 (s, 1H, Triazolyl-H).
MS/CI: 280 (M+1).

### Beispiel XXXII-7

### (3-N-[4-Chlorpyrazolyl])-6-methyl]phenylessigsäuremethylester

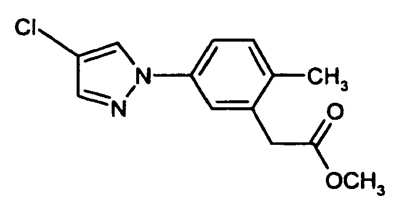

¹H-NMR {400 MHz, CDCl₃}: 2.32 (s, 3H, CH₃); 3.67 (s, 2H, CH₂); 3.70 (s, 3H, OCH₃); 7.22 (m, 1H, Ph-H); 7.40 (m, 1H, Ph-H); 7.50 (m, 1H, Ph-H); 7.60 (s, 1H, Pyrazolyl-H); 7.95 (s, 1H, Pyrazolyl-H).
GC-MS/CI: 265 (M+1).

### Beispiel XXXII-8

### (2-Chlor-6-ethyl-4-[4-chlorpyrazolyl])phenylessigsäuremethylester

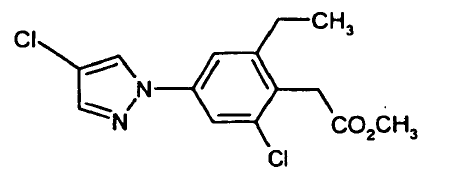

¹H-NMR {400 MHz, DMSO-d₆}: 1.18 (t, ³J_{HH} = 7 Hz, 3H, CH₃); 2.72 (q, ³J_{HH} = 7 Hz, 2H, CH₂); 3.64 (s, 3H, OCH₃); 3.89 (s, 2H, CH₂); 7.69 (m, 1H, Ph-H); 7.81 (m, 1H, Ph-H); 7.89 (s, 1H, Pyrazolyl-H); 8.88 (s, 1H, Pyrazolyl-H).
MS/CI: 313 (M+1).

### Beispiel XXXII-9

### (2-Chlor-6-ethyl-4-[4-chlorpyrazolyl)phenylessigsäuremethyl-ester

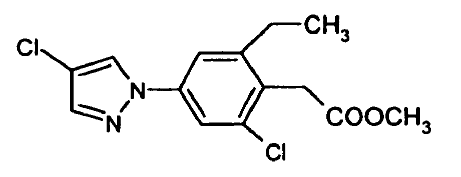

¹H-NMR{400 MHz, DMSO-d₆}: 1.18 (t, ³J_{HH} = 7 Hz, 3H, CH₃); 2,72 (q, ³J_{HH} = 7 Hz, 2H, CH₂); 3,64 (s, 3H, OCH₃): 3.89 (s, 2H, CH₂); 7,69 (m, 1H, Ph-H); 7,81 (m, 1H, Ph-H); 7,89 (s, 1H, Pyrazolyl-H); 8,88 (s, 1H, Pyrazolyl-H).
MS/CI: 313 (M+1).

### Beispiel XXXII-10

### (2-Ethyl-6-methyl-4-[4-chlorpyrazolyl])phenylessigsäuremethylester

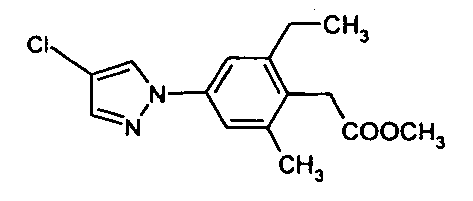

¹H-NMR {400 MHz, DMSO-d₆}: 1,18 (t, ³J_{HH} = 7 Hz, 3H, CH₃): 2,59 (q, ³J_{HH} = 7 Hz, 2H, CH₂); 3,59 (s, 3H, OCH₃); 3,72 (s, 2H, CH₂); 7,51 (m, 2H, Ph-H); 7,89 (s, 1H, Pyrazolyl-H); 8,78 (s, 1H, Pyrazolyl-H).
MS/CI: 313 (M+1).

### Beispiel XXVIII-1

### (4-N-[4-Chlorpyrazolyl])-2,6 dimethyl)phenylessigsäure

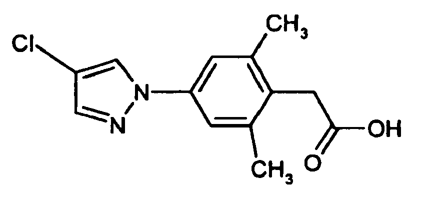

17,2 g (61,7 mmol) (4-[4-Chlorpyrazolyl]-2,6-dimethyl)phenylessigsäure werden in 160 ml Methanol gelöst und anschließend mit 4,2 g (74 mmol) Kaliumhydroxid in 160 ml Wasser für 12 Stunden auf 80°C erhitzt. Methanol wird am Rotationsverdampfer abgezogen, der Rückstand auf pH 3 gestellt und das ausgefallene Produkt abfiltriert und getrocknet.

Ausbeute: 16,2 g (99 %)
¹H-NMR {400 MHz, CDCl₃}: 2.38 (s, 6H, CH₃); 3.73 (s, 2H, CH₂); 7.32 (s, 2H, Ph-H); 7.61 (s, 1H, Pyrazolyl-H); 7.86 (s, 1H, Pyrazolyl-H); Säure-OH nicht detektiert.

In Analogie zu Beispiel (XXVIII-1) erhält man folgende Verbindungen der Formel (XXVIII).

### Beispiel XXVIII-2

### (2-Ethyl-4-N-[4-methoxypyrazolyl]-6-methyl)phenylessigsäure

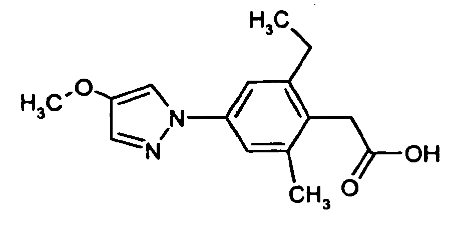

¹H-NMR {400 MHz, DMSO-d₆}: 1.17 (t,³J_{HH}= 7 Hz, 3H, CH₃); 2.30 (s, 3H, CH₃); 2.65 (q, ³J_{HH}= 7Hz, 2H, CH₂); 3.63 (s, 2H, CH₂); 3.77 (s, 3H, OCH₃); 7.45 (m, 2H, Ph-H); 7.50 (s, 1H, Pyrazolyl-H); 8.23 (s, 1H, Pyrazolyl-H); 12.5 (s, 1H, OH).
MS/CI: 275 (M+1).

### Beispiel XXVIII-3

### (2,6-Dimethyl-4-N-[4-methoxypyrazolyl])phenylessigsäure

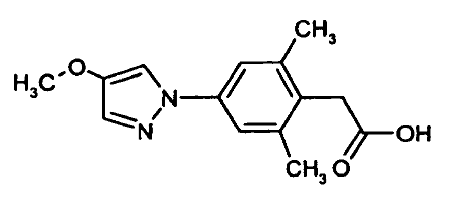

¹H-NMR {400 MHz, DMSO-d₆}: 2.30 (s, 6H, CH₃); 3.61 (s, 2H, CH₂); 3.82 (s, 3H, OCH₃); 7.44 (s, 2H, Ph-H); 7.50 (s, 1H, Pyrazolyl-H); 8.21 (s, 1H, Pyrazolyl-H); 12.4 (s, 1H, OH).
MS/CI: 261 (M+1).

### Beispiel XXVIII-4

### (2,6-Diethyl-4-N-[4-chlorpyrazolyl])phenylessigsäure

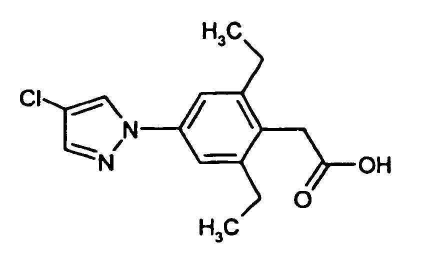

¹H-NMR {400 MHz, DMSO-d₆}: 1.18 (t, ³J_{HH}= 8 Hz, 6H, CH₃); 2.63 (q, ³J_{HH}= 8 Hz, 4H, CH₂); 3.67 (s, 2H, CH₂); 7.50 (s, 2H, Ph-H); 7.85 (s, 1H, Pyrazolyl-H); 8.79 (s, 1H, Pyrazolyl-H); 12.5 (s, 1H, OH).
MS/CI: 293 (M+1).

### Beispiel XXVIII-5

### (2,6-Dimethyl-4-N-[4-carboxylatopyrazolyl])phenylessigsäure

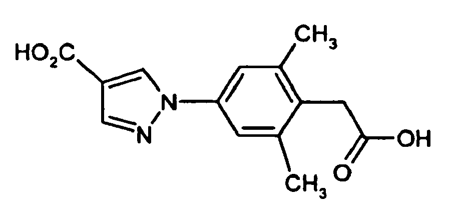

¹H-NMR {400 MHz, DMSO-d₆}: 2.32 (s, 6H, CH₃); 3.64 (s, 2H, CH₂); 7.52 (s, 2H, Ph-H); 8.09 (s, 1H, Pyrazolyl-H); 8.87 (s, 1H, Pyrazolyl-H); 12.3 (s, 2H, OH).
MS/CI: 275 (M+1).

### Beispiel XXVIII-6

### (2,6-Dimethyl-4-N-[3-chlortriazolyl])phenylessigsäure

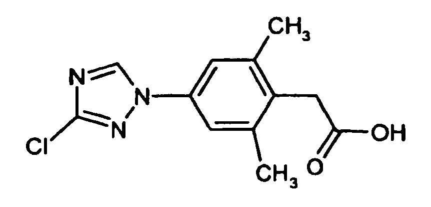

¹H-NMR {400 MHz, DMSO-d₆}: 2.26 (s, 6H, CH₃); 3.56 (s, 2H, CH₂); 7.44 (s, 2H, Ph-H); 9.22 (s, 1H, Triazolyl-H); 12.2 (s, 1H, OH).
MS/CI: 266 (M+1).

### Beispiel XXVIII-7

### (3-N-[4-Chlorpyrazolyl]-6-methyl)phenylessigsäure

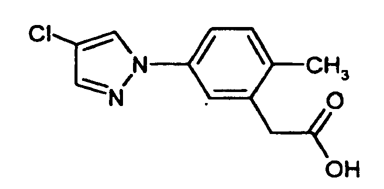

¹H-NMR {400 MHz, CDCl₃}: 2.34 (s, 3H, CH₃); 3.71 (s, 2H, CH₂); 7.26 (m, 1H, Ph-H); 7.40 (m, 1H, Ph-H); 7.52 (m, 1H, Ph-H); 7.62 (s, 1H, Pyrazolyl-H); 7.85 (s, 1H, Pyrazolyl-H); Säure-OH nicht detektiert.

### Beispiel XXVIII-8

### (2-Chlor-6-ethyl-4-[4-chlorpyrazolyl])phenylessigsäure

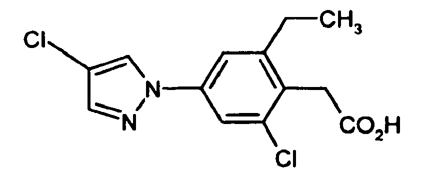

¹H-NMR {400 MHz, DMSO-d₆}: 1.17 (t, ³J_{HH} = 7 Hz, 3H, CH₃); 2.71 (q, ³J_{HH} = 7 Hz, 2H, CH₂); 3.79 (s, 2H, CH₂); 7.69 (m, 1H, Ph-H); 7.79 (m, 1H, Ph-H); 7.91 (s, 1H, Pyrazolyl-H); 8.88 (s, 1H, Pyrazolyl-H); 12,6 (s,1H, OH).
MS/CI: 299 (M+1).

### Beispiel XXXVIII-9

### (2-Chlor-6-ethyl-4-[4-chlorpyrazolyl])phenylessigsäure

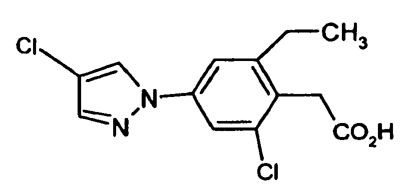

¹H-NMR {400 MHz, DMSO-d₆}: 1.17 (t,³J_{HH} = 7 Hz, 3H, CH₃); 2,71 (q, ³J_{HH}= 7 Hz, 2H, CH₂); 3,79 (s, 2H, CH₂); 7.69 (m, 1H, Ph-H); 7.79 (m, 1H, Ph-H); 7,91 (s, 1H, Pyrazolyl-H); 8.88 (s, 1H, Pyrazolyl-H); 12.6 (s, 1H, OH).
MS/CI: 299 (M+1).

### Beispiel XXVIII-10

### (2-Ethyl-6-methyl-4-[4-chlorpyrazolyl])phenylessigsäure

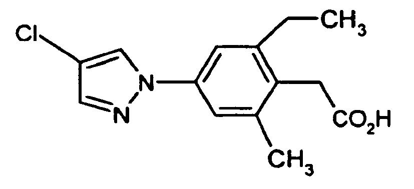

¹H-NMR {400 MHz, DMSO-d₆}: 1.19 (t,³J_{HH} = 7 Hz, 3H, CH₃); 2.60 (q, ³J_{HH} = 7 Hz, 2H, CH₂); 3,81 (s, 2H, CH₂); 7,52(m, 2H, Ph-H); 7.83 (s,1H, Pyrazolyl-H); 8.62 (s, 1H, Pyrazolyl-H); OH not detected.
MS/CI: 279 (M+1).

### Anwendungsbeispiele

### Beispiel A

### Meloidogyne-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z.B. die folgenden Verbindungen der Herstellungsbeispiele gute Wirksamkeit:

**Tabelle A**

| pflanzenschädigende Nematoden | | |
|---|---|---|
| **Meloidogyne-Test** | | |
| | | |
| Wirkstoffe | Wirkstoffkonzentration in ppm | Wirkung in % nach 14^{d} |
| Bsp.- I-1-a-2 | 20 | 100 |
| Bsp.- I-6-a-2 | 20 | 100 |

### Beispiel B

### Myzus-Test (Spritzbehandlung)

| | |
|---|---|
| Lösungsmittel: | 78 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (Brassica pekinensis), die von der Grünen Pfirsichblattlaus (Myzus persicae) befallen sind, werden mit einer Wirkstoffzubereitung der gewünschten Konzentration gespritzt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindung der Herstellungsbeispiele gute Wirksamkeit:

**Tabelle B**

| pflanzenschädigende Insekten | | |
|---|---|---|
| **Myzus-Test** | | |
| | | |
| Wirkstoffe | Wirkstoffkonzentration in g/ha | Abtötungsgrad in % nach 4^{d} |
| Bsp.- I-1-a-3 | 100 | 80 |

### Beispiel C

### Phaedon-Larven-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirktoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgenden Verbindungen der Herstellungsbeispiele gute Wirksamkeit:

**Tabelle C**

| pflanzenschädigende Insekten | | |
|---|---|---|
| **Phaedon-Larven-Test** | | |
| | | |
| Wirkstoffe | Wirkstoffkonzentration in ppm | Abtötungsgrad in % nach 7^{d} |
| Bsp.- I-6-a-1 | 500 | 90 |
| Bsp.- I-7-a-1 | 500 | 100 |
| Bsp.- I-7-a-2 | 500 | 100 |

### Beispiel D

### Spodoptera frugiperda-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt z. B. die folgende Verbindung der Herstellungsbeispiele gute Wirksamkeit:

**Tabelle D**

| pflanzenschädigende Insekten | | |
|---|---|---|
| **Spodoptera frugiperda -Test** | | |
| | | |
| Wirkstoffe | Wirkstoffkonzentration in ppm | Abtötungsgrad in % nach 7^{d} |
| Bsp.- I-1-a-1 | 100 | 85 |

### Beispiel E

### Tetranychus-Test (OP-resistent/Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (Phaseolus vulgaris), die von allen Stadien der Gemeinen Spinnmilbe (Tetranychus urticae) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigt z. B. die folgende Verbindung der Herstellungsbeispiele gute Wirksamkeit:

**Tabelle E**

| pflanzenschädigende Milben | | |
|---|---|---|
| **Tetranychus-Test** (OP-resistent/Spritzbehandlung) | | |
| | | |
| Wirkstoffe | Wirkstoffkonzentration in ppm | Abtötungsgrad in % nach 4^{d} |
| Bsp.- I-1-a-2 | 20 | 90 |

### Beispiel F

### Sphaerotheca-Test (Gurke) / protektiv

| | |
|---|---|
| Lösungsmittel : | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von ***Sphaerotheca fuliginea*** inokuliert. Die Pflanzen werden dann bei ca. 23°C und einer relativen Luftfeuchtigkeit von ca. 70% im Gewächshaus aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle F**

| **Sphaerotheca-Test (Gurke) / protektiv** | | |
|---|---|---|
| Wirkstoff | Aufwandmenge an Wirkstoff in g/ha | Wirkungsgrad in % |
| Bsp. I-6-a-2 | 100 | 97 |

### Beispiel G

In vitro-Test zur ED₅₀-Bestimmung bei Mikroorganismen

In die Kavitäten von Mikrotiterplatten wird eine methanolische Lösung des zu prüfenden Wirkstoffs, versetzt mit dem Emulgator PS16, pipettiert. Nachdem das Lösungsmittel abgedampft ist, werden je Kavität 200µl Potatoe-Dextrose-Medium hinzugefügt.

Das Medium wurde vorher mit einer geeigneten Konzentration von Sporen bzw. Mycel des zu prüfenden Pilzes versetzt.

Die resultierenden Konzentrationen des Wirkstoffs betragen 0.1, 1, 10 und 100 ppm. Die resultierende Konzentration des Emulgators beträgt 300 ppm.

Die Platten werden anschließend 3-5 Tage auf einem Schüttler bei einer Temperatur von 22°C inkubiert, bis in der unbehandelten Kontrolle ein ausreichendes Wachstum feststellbar ist.

Die Auswertung erfolgt photometrisch bei einer Wellenlänge von 620 nm. Aus den Messdaten der verschiedenen Konzentrationen wird die Wirkstoffdosis, die zu einer 50 %igen Hemmung des Pilzwachstums gegenüber der unbehandelten Kontrolle führt (ED₅₀), berechnet.

**Tabelle G**

| In vitro-Test zur ED₅₀-Bestimmung bei Mikroorganismen | | |
|---|---|---|
| Wirkstoff | Mikroorganismus | ED₅₀-Wert |
| Bsp. I-6-a-2 | Botrytis cinerea | < 0,1 |
| Bsp. I-6-c-1 | Botrytis cinerea | < 0,1 |

### Beispiel H

### Post-emergence-Test

| | |
|---|---|
| Lösungsmittel: | 5 Gewichtsteile Aceton |
| Emulgator: | 1 Gewichtsteil Alkyarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, dass die jeweils gewünschten Wirkstoffmengen pro Flächeneinheit ausgebracht werden. Die Konzentration der Spritzbrühe wird so gewählt, dass in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | | |
|---|---|---|
| 0 % | = | keine Wirkung (wie unbehandelte Kontrolle) |
| 100 % | = | totale Vernichtung |

### Beispiel I

### Pre-emergence-Test

| | |
|---|---|
| Lösungsmittel: | 5 Gewichtsteile Aceton |
| Emulgator: | 1 Gewichtsteil Alkyarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung bespritzt so, dass die jeweils gewünschten Wirkstoffmengen pro Flächeneinheit ausgebracht werden. Die Konzentration der Spritzbrühe wird so gewählt, dass in 1000 l Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | | |
|---|---|---|
| 0 % | = | keine Wirkung (wie unbehandelte Kontrolle) |
| 100 % | = | totale Vernichtung |

| Pre-emergence | Gewächshaus | g a.i./ha | Alopecurus | Avena fatua | Echinochloa | Setaria | Amaranthus | Sinapis |
|---|---|---|---|---|---|---|---|---|
| | Bsp.I-1-a-2 | 250 | 100 | 100 | 100 | 100 | 100 | 80 |

### J. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) bzw. emulgierbaren Konzentraten (EC) formulierten Testverbindungen werden dann als wässrige Suspension bzw. Emulsion mit einer Wasseraufwandmenge von umgerechnet 600 l/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

### K. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) bzw. emulgierbare Konzentrate (EC) formulierten Testverbindungen werden in verschiedenen Dosierungen mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

| post-emergence | Gewächshaus | g a.i./ha | Zuckerrüben | Echinochloa | Setaria | Sorghum |
|---|---|---|---|---|---|---|
| | Bsp.I-1-a-1 (EC) | 320 | 0 | 100 | 100 | 90 |

### Versuchsbeschreibung für Profiling-Versuche

### L. Herbizide Wirkung im Nachauflauf

Samen von mono- bzw. dikotylen Unkraut- bzw. Kulturpflanzen werden in Holzfasertöpfen oder in Plastiktöpfen in sandigem Lehmboden ausgelegt, mit Erde abgedeckt und im Gewächshaus, während der Vegetationsperiode auch im Freien außerhalb des Gewächshauses, unter guten Wachstumsbedingungen angezogen. 2 - 3 Wochen nach der Aussaat werden die Versuchspflanzen im Ein- bis Drei-Blattstadium behandelt. Die als Spritzpulver (WP) oder Flüssigkeit (EC) formulierten Testverbindungen werden in verschiedenen Dosierungen mit einer Wasseraufwandmenge von umgerechnet 300 l/ha unter Zusatz von Netzmittel (0,2 bis 0,3 %) auf die Pflanzen und die Bodenoberfläche gespritzt. 3 bis 4 Wochen nach Behandlung der Versuchspflanzen wird die Wirkung der Präparate visuell im Vergleich zu unbehandelten Kontrollen bonitiert (herbizide Wirkung in Prozent (%): 100 % Wirkung = Pflanzen sind abgestorben, 0 % Wirkung = wie Kontmllpflanzen).

### Verwendung von Safenern

Die Kulturpflanzen werden vor der Anwendung der Testsubstanzen mit dem Safener mit einer bestimmten Hektaraufwandmenge gespritzt
(üblicherweise 1 Tag vor der Anwendung der Prüfsubstanzen).

Durch Vergleich der Wirkung von Testsubstanzen auf Kulturpflanzen, welche ohne und mit Safener behandelt wurden, kann die Wirkung der Safenersubstanz beurteilt werden.

### GefäBversuche mit Getreide im Gewächshaus

**Tabelle**

| | **Aufwandmenge g a.i./ha** | **Sommerweizen beobachtet (%)** |
|---|---|---|
| I-1-a-2 | 25 | 50 |
| I-1-a-2 + Mefenpyr | 25 + 100 | 10 |

**Tabelle**

| | A**ufwandmenge g a.i./ha** | **Sommergerste beobachtet (%)** |
|---|---|---|
| I-1-a-6 | 100 | 20 |
| I-1-a-6 + Mefenpyr | 100 + 100 | 0 |

**Tabelle**

| | **Aufwandmenge g a.i./ha** | **Sommergerste beobachtet (%)** | **Sommerweizen Beobachtet (%)** |
|---|---|---|---|
| I-1-a-7 | 25 | 95 | 60 |
| I-1-a-7 + Mefenpyr | 25 +100 | 50 | 15 |

| | | | |
|---|---|---|---|
| Mefenpyr 1 Tag vor Herbizidapplikation | | | |

**Tabelle**

| | **Aufwandmenge g a.i./ha** | **Sommergerste beobachtet (%)** | **Sommerweizen beobachtet (%)** |
|---|---|---|---|
| Bsp. I-2-a-6 | 100 | 20 | 15 |
| | 50 | 10 | 15 |
| Bsp. I-2-a-6 | 100 + 100 | 5 | 10 |
| + Mefenpyr | 50 + 100 | 0 | 10 |

**Tabelle**

| | **Aufwandmenge g a.i./ha** | **Sommergerste beobachtet (%)** | **Sommerweizen beobachtet (%)** |
|---|---|---|---|
| Bsp. I-2-a-5 | 100 | 20 | 40 |
| | 50 | 15 | 20 |
| Bsp. I-2-a-5 | 100 + 100 | 0 | 10 |
| + Mefenpyr | 50 + 100 | 0 | 0 |

**Tabelle**

| | **Aufwandmenge g a.i./ha** | **Sommergerste beobachtet (%)** | **Sommerweizen beobachtet (%)** |
|---|---|---|---|
| Bsp. I-2-b-5 | 100 | 20 | 40 |
| | 50 | 15 | 40 |
| Bsp. I-2-b-5 | 100 + 100 | 0 | 20 |
| + Mefenpyr | 50+100 | 0 | 10 |

| | | | |
|---|---|---|---|
| Mefenpyr 1 Tag vor Herbizidapplikation | | | |

### Beispiel L

### Grenzkonzentrations- Test / Bodeninsekten-Behandlung transgener Pflanzen

| | |
|---|---|
| Testinsekt: | **Diabrotica balteata - Larven im Boden** |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 l Töpfe und lässt diese bei 20°C stehen.

Sofort nach dem Ansatz werden je Topf 5 vorgekeimte Maiskörner der Sorte YIELD GUARD (Warenzeichen von Monsanto Domp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1Pflanze = 20 % Wirkung).

### Beispiel M

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 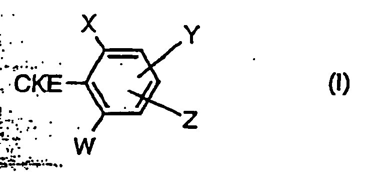 in welcher
W für Wasserstoff, Methyl oder Ethyl steht,
X für Chlor, Methyl oder Ethyl steht,
Y für Wasserstoff steht,
Z für den Rest in der 4- oder 5-Position 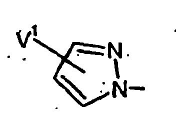 steht,
V¹ für Chlor oder Methoxy steht,
CKE für eine der Gruppen 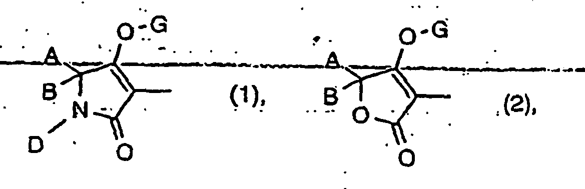 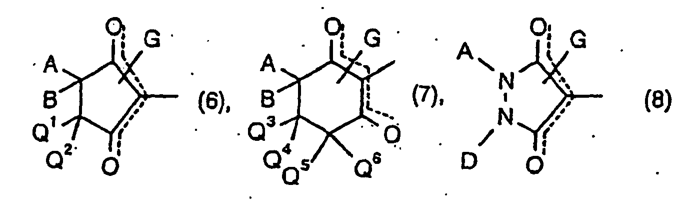 steht,
A für Wasserstoff, C₁-C₄₋Alkyl oder Cyclopropyl steht,
B für Wasserstoff oder Methyl steht, oder
A, B und das Kohlenstoffatom an das sie gebunden sind, für gesättigtes C₅-C₆- Cycloalkyl stehen, in welchem gegebenenfalls ein Ringglied durch Sauerstoff ersetzt ist und welches gegebenenfalls einfach durch Methyl oder Methoxy substituiert ist, mit der Maßgabe, dass dann Q³ für Wasserstoff steht,
D für Wasserstoff steht,
oder
A und D gemeinsam für C₃-C₅-Alkandiyl stehen, worin gegebenenfalls ein Kohlenstoffatom durch Sauerstoff ersetzt ist
Q¹ für Wasserstoff steht,
Q² für Wasserstoff steht,
Q³ für Methyl steht,
Q⁴ für Methyl steht, oder
Q³ und Q⁴ gemeinsam mit dem Kohlenstoff, an den sie gebunden sind, für einen gesättigten C₅-C₆-Ring stehen, mit der Maßgabe, dass dann A für Wasserstoff, steht,
Q⁵ für Wasserstoff steht,
Q⁶ für Wasserstoff steht,
G für Wasserstoff (a) oder für eine der Gruppen 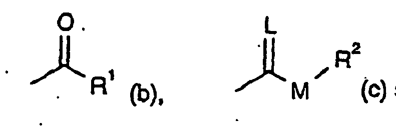 steht,
in welchen
L für Sauerstoff steht und
M für Sauerstoff oder Schwefel steht.
R¹ für C₁-C₆-Alkyl oder C₁-C₂-Alkoxy-C₁-alkyl steht,
R² für C₁-C₈-Alkyl oder Benzyl steht.

2. Verfahren zur Herstellung von Verbindungen der Formel (I) gemäß Anspruch 1, **dadurch gekennzeichnet, dass** man zum Erhalt von
(A) Verbindungen der Formel (I-1-a) 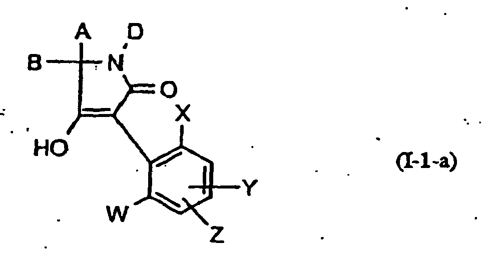 in welcher
A, B, D, W, X. Y und Z die oben angegebenen Bedeutungen haben,
Verbindungen der Formel (II) 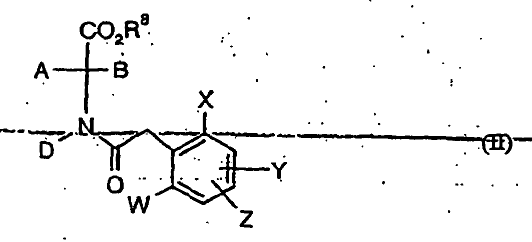 in welcher
A, B, D, W, X, Y und Z die oben angegebenen Bedeutungen haben, und
R⁸ für Alkyl steht,
in Gegenwart eines Verdünnungsmittels und in Gegenwart einer Base intramolekular kondensiert,
(B) Verbindungen der Formel (I-2-a) 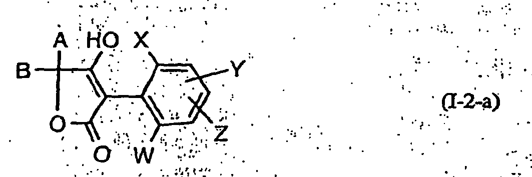 in welcher
A, B, W, X, Y und Z die oben angegebenen Bedeutungen haben,
Verbindungen der Formel (III) 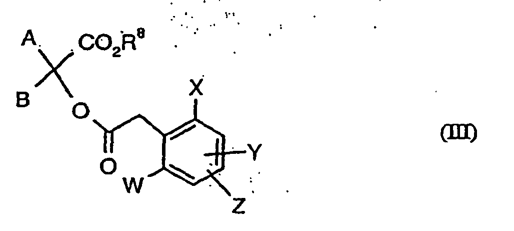 in welcher
A, B, W, X , Y, Z und R⁸ die oben angegebenen Bedeutungen haben,
in Gegenwart eines Verdünnungsmittels und in Gegenwart einer Base intramolekular kondensiert,
(F) Verbindungen der Formel (I-6-a) 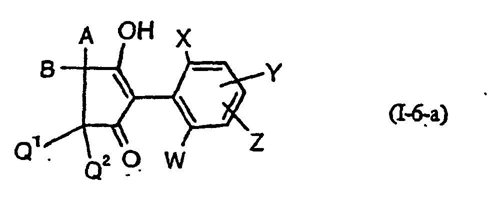 in welcher
A, B, Q¹, Q², W, X, Y und Z die oben angegebene Bedeutung haben,
Verbindungen der Formel (VIII) 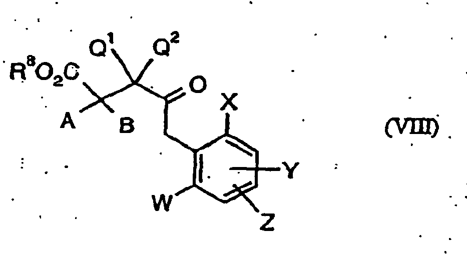 in welcher
A, B, Q¹, Q², W, X, Y und Z die oben angegebene Bedeutung haben, und R⁸ für Alkyl steht,
R⁸ für Alkyl steht,
gegebenenfalls in Gegenwart eines Verdünnungsmittels und in Gegenwart einer Base intramolekular cyclisiert,
(G) Verbindungen der Formel (I-7-a) 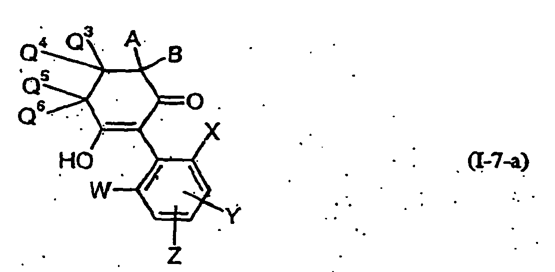 in welcher
A, B, Q³, Q⁴, Q⁵, Q⁶, W, X, Y und Z die oben angegebene Bedeutung haben,
Verbindungen der Formel (IX) 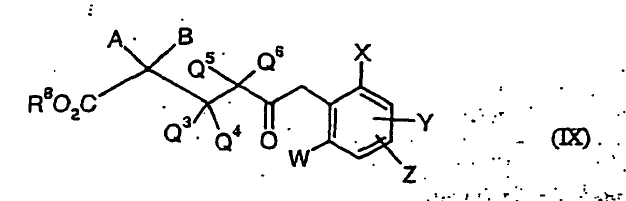 in welcher
A, B, Q³, Q⁴, Q⁵, Q⁶, W, X, Y und Z die oben angegebene Bedeutung haben und
R⁸ für Alkyl steht,
in Gegenwart eines Verdünnungsmittels und in Gegenwart einer Base intramolekular kondensiert,
(H) Verbindungen der Formel (I-8-a) 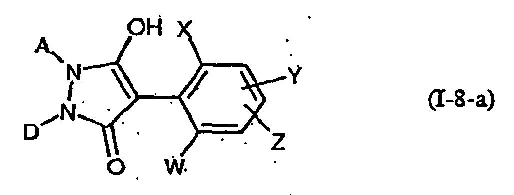 in welcher
A, D, W, X, Y und Z die oben angegebenen Bedeutungen haben,
Verbindungen der Formel (X) 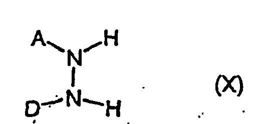 in welcher
A und D die oben angegebene Bedeutung haben,
α) mit Verbindungen der Formel (VI) 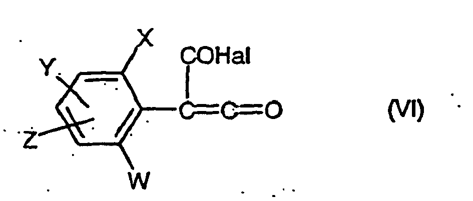 in welcher
Hal, W, X, Y und Z die oben angegebenen Bedeutungen haben,
gegebenenfalls in Gegenwart eines Verdünnungsmittels und gegebenenfalls in Gegenwart eines Säureakzeptors umsetz, oder
β) mit Verbindungen der Formel (XI) 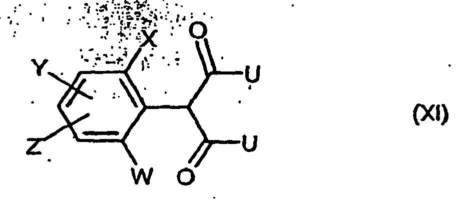 in welcher
W, X, Y und Z die oben angegebene Bedeutung haben,
und U für NH₂ oder O-R⁸ steht,
gegebenenfalls in Gegenwart eines Vendünnungsmittels und gegebenenfalls in Gegenwart einer Base umsetzt, oder
γ) Verbindungen der Formel (XII) 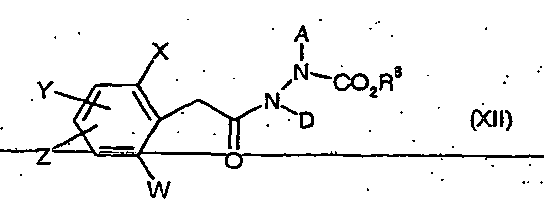 in welcher
A, D, W, X, Y, Z und R⁸ die oben angegebene Bedeutung haben,
gegebenenfalls in Gegenwart eines Verdünnungsmittels und gegebenenfalls in Gegenwart einer Base umsetzt,
(I) Verbindungen der oben gezeigten Formeln (I-1-b) bis (I-8-b), in welchen A, B, D, Q¹, Q², Q¹, Q⁴, Q⁵, Q⁶, R¹, W, X, Y und Z die oben angegebenen Bedeutungen haben, Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen" haben, jeweils
(α) mit Säurehalogeniden der Formel (XIII) 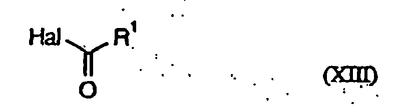 in welcher
R¹ die oben angegebene Bedeutung hat und
Hal für Halogen steht
oder
(β) mit Carbonsäureanhydriden der Formel (XIV)
R¹-CO-O-CO-R¹ (XIV)
in welcher
R¹ die oben angegebene Bedeutung hat,
gegebenenfalls in Gegenwart eines Verdünnnungsmittels und gegebenenfalls in Gegenwart eines Säurebindemittels umsetzt,
(J) Verbindungen der oben gezeigten Formeln (I-1-c) bis (I-8-c), in welchen A, B, D, Q¹, Q², Q³, Q⁴, Q⁵, Q⁶, R², M, W, X, Y und Z die oben angegebenen Bedeutungen haben und L für Sauerstoff steht, Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
mit Chlorameisensäureestern oder Chlorameisensäurethioestern der Formel (XV)
R²-M-CO-Cl (XV)
in welcher
R² and M die oben angegeben Bedeutungen haben,
gegebenenfalls in Gegenwart eines Verdünnungsmittels und gegebenenfalls in Gegenwart eines Säurebindemittels umsetzt,
(K) Verbindungen der oben gezeigten Formeln (I-1-c) bis (I-8-c), in welchen A, B, D, Q¹, Q², Q³ Q⁴, Q⁵, Q⁶, R², M, W, X, Y und Z die oben angegebenen Bedeutungen haben und L für Schwefel steht, Verbindungen der oben gezeigten Formeln (I-1-a) bis (I-8-a), in welchen A, B, D, Q¹, Q², Q³, Q⁴, Q⁵, Q⁶, W, X, Y und Z die oben angegebenen Bedeutungen haben, jeweils
mit Chlormononothioameisensäureestern oder Chlordithioameisensäureestern der Formel (XVI) 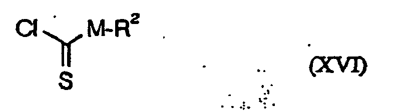 in welcher
M und R² die oben angegebenen Bedeutungen haben,
gegebenenfalls in Gegenwart eines Verdünnungsmittels und gegebenenfalls in Gegenwart eines Säurebindemittels umsetzt
(P) Verbindungen der oben gezeigten Formeln (I-1) bis (I-8), in welchen A, B, D, Q¹, Q², Q³, Q⁴, Q⁵, Q⁶, W, X, Y und Z die oben angegebene Bedeutung haben, Verbindungen der Formel (I-1'bis (I-8'), 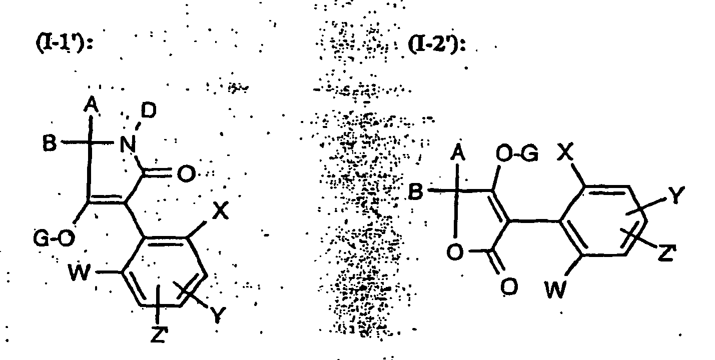 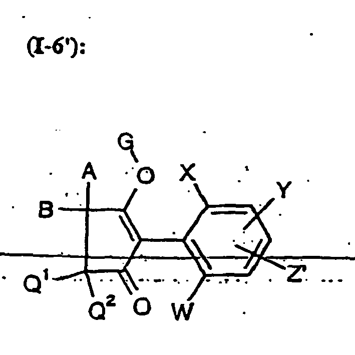 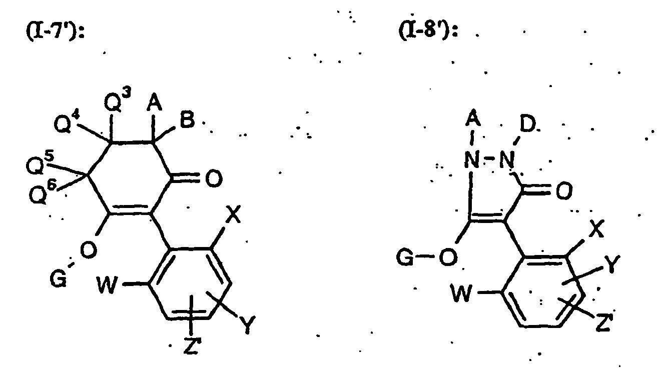 in welchen
A, B, D, G, Q¹, Q², Q³, Q⁴, Q⁵, Q⁶, W, X und Y die oben angegebene Bedeutung haben und
Z' für Chlor, Brom, Jod steht,
mit NH-Heterocyclen der Formel (XXIII)
H-Z (XXIII)
in welcher
Z die oben angegebene Bedeutung hat und
in Gegenwart eines Lösungsmittels, einer Base und eines Katalysators umsetzt, wobei als Katalysator insbesondere Kupfer-I-Salze in Frage kommen.

3. Verbindungen der Formel (II) 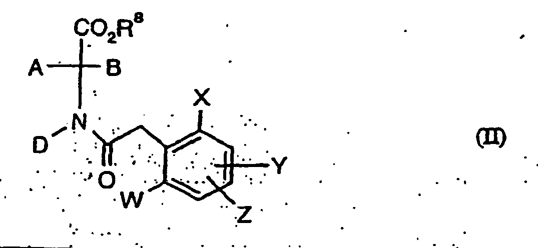 in welcher
A, B, D, W, X, Y , Z und R⁸ die oben angegebenen Bedeutungen haben.

4. Verbindungen der Formel (III) 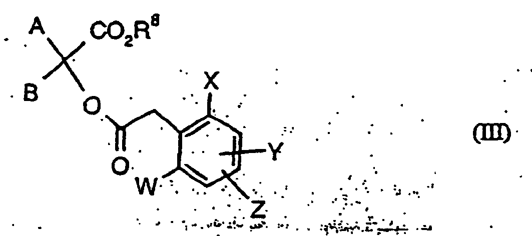 in welcher
A, B, W, X, Y, Z und R⁸ die oben angegebenen Bedeutungen haben.

5. Verbindungen der Formel (VI) 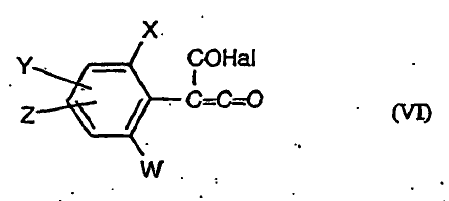 in welcher
W, X, Y, Z und Hal die oben angegebenen Bedeutungen haben.

6. Verbindungen der Formel (VIII) 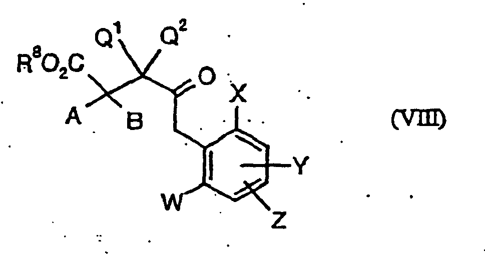 in welcher
A, B, Q¹, Q², W, X, Y, Z und R⁸ die oben angegebene Bedeutung hat.

7. Verbindungen der Formel (IX) 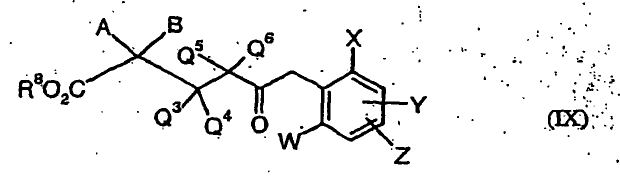 in welche
A, B, R⁸ ,Q³, Q⁴, Q⁵, Q⁶, W, X, Y und Z die oben angegebene Bedeutung haben.

8. Verbindungen der Formel (XI) 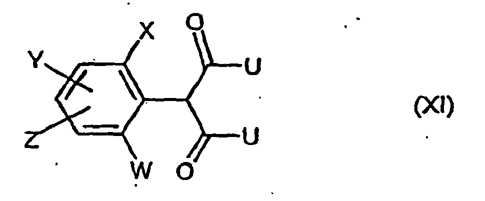 in welcher
U, W, X, Y und Z die oben angegebene Bedeutung haben.

9. Verbindungen der Formel (XII) 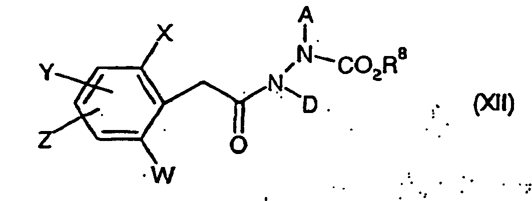 in welcher
A, D, W, X, Y, Z und R⁸ die oben angegebene Bedeutung haben.

10. Verbindungen der Formel (XXV) 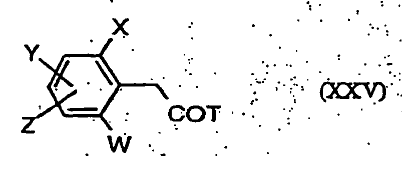 in welcher
T, W, X, Y und Z die oben angegebenen Bedeutungen haben.

11. Verbindungen der Formel (XXVI) 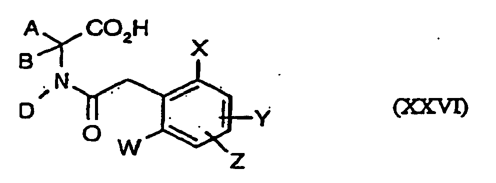 in welcher
A, B, D, W, X, Y und Z die oben angegebenen Bedeutungen haben.

12. Verbindungen der Formel (XXX) 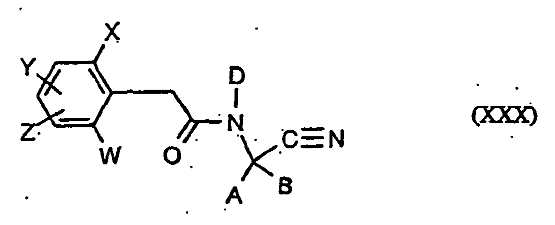 in welcher
A, B, D, W, Y, Y und Z die oben angegebenen Bedeutungen haben.

13. Verbindungen der Formel (XXVIII) 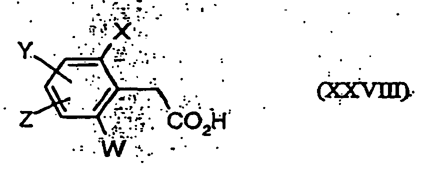 in welcher
W, X, Y und Z die oben angegebene Bedeutung haben.

14. Verbindungen der Formel (XXXII) 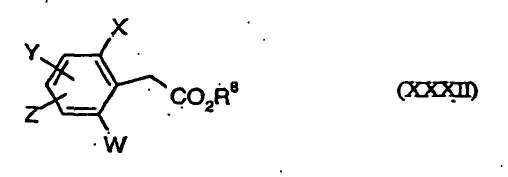 in welcher
W, X, Y, Z und R⁸ Y, Z und R⁸ die oben angegebene Bedeutung haben.

15. Verbindungen der Formel (XXXIV) 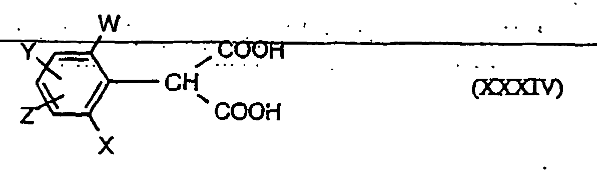 in welcher
W, X, Y und Z die oben angegebenen Bedeutungen haben.

16. Verbindungen der Formel (XXXV) 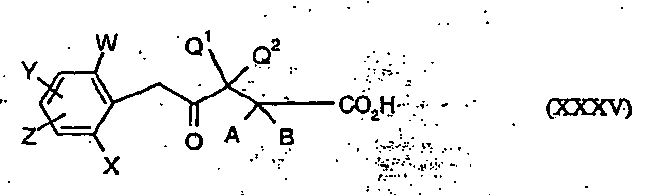 in welcher
A, B, Q¹, Q², W, X, Y und Z die oben angegebene Bedeutung haben.

17. Verbindungen der Formel (XXXVI) 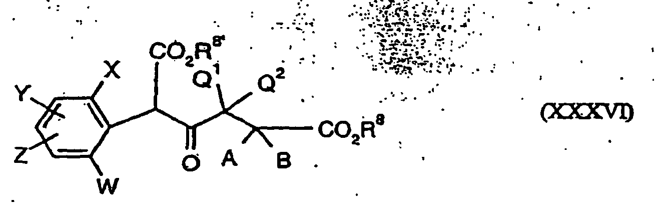 in welcher
A, B, Q¹, Q², W, X, Y und Z die oben angegebene Bedeutung haben
und
R⁸ und R^{8'} für Alkyl stehen.

18. Verbindungen der Formel (XXXIX) 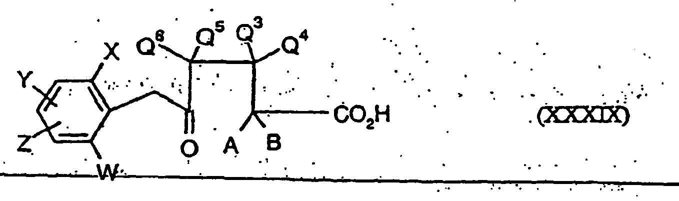 in welcher
A, B, Q³, Q⁴, Q⁵, Q⁶, W, X, Y und Z die oben angegebene Bedeutung haben.

19. Verbindungen der Formel (XL) 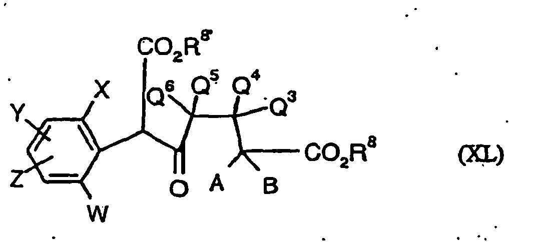 in welcher
Q³, Q⁴, Q⁵, Q⁶, W, X, Y und Z die oben angegebene Bedeutung haben und
R⁸ und R^{8'} für Alkyl stehen.

20. Schädlingsbekämpfungsmittel und/oder Herbizide und/oder Fungizide, **gekennzeichnet durch** einen Gehalt an mindestens einer Verbindung der Formel (I) gemäß Anspruch 1.

21. Verfahren zur nicht-therapeutischen Bekämpfung von tierischen Schädlingen und/oder unerwünschtem Pflanzenbewuchs und/oder Pilzen, **dadurch gekennzeichnet, dass** man Verbindungen der Formel (I) gemäß Anspruch 1 auf Schädlinge und/oder ihren Lebensraum ein wirken lässt.

22. Verwendung von Verbindungen der Formel (I) gemäß Anspruch 1 zur nicht-therapeutischen Bekämpfung von tierischen Schädlingen und/oder unerwünschtem Pflanzenbewuchs, und/oder Pilzen.

23. Verfahren zur Herstellung von Schädlingsbekämpfungsmitteln und/oder Herbiziden und/oder Fungiziden, **dadurch gekennzeichnet, dass** man Verbindungen der Formel (I) gemäß Anspruch 1 mit Streckmitteln and/oder oberflächenaktiven Stoffen vermischt.

24. Verwendung von Verbindungen der Formel (I) gemäß Anspruch 1 zur Herstellung von Schädlingsbekämpfungsmitteln und/oder Herbiziden und/oder Fungiziden.

25. Mittel enthaltend einen wirksamen Gehalt an einer Wirkstoffkombination umfassend als Komponenten
(a') mindestens ein substituiertes, cyclisches Ketoenol der Formel (I), in welcher CKE, W, X, Y und Z die oben angegebene Bedeutung haben
und
(b') zumindest eine die Kulturpflanzen-Verträglichkeit verbessernde Verbindung aus der folgenden Gruppe von Verbindungen:

26. Verfahren zum Bekämpfen von unerwünschtem Pflanzenwuchs, **dadurch gekennzeichnet, dass** man ein Mittel gemäß Anspruch 25 auf die Pflanzen oder ihre Umgebung einwirken lässt.

27. Verwendung eines Mittels gemäß Anspruch 25 zum Bekämpfen von unerwünschten Pflanzenwuchs.

28. Verfahren zum Bekämpfen von unerwünschtem Pflanzenwuchs, **dadurch gekennzeichnet, dass** man eine Verbindung der Formel (I) gemäß Anspruch 25 und die die Kulturpflanzenverträglichkeit verbessernde Verbindung gemäß Anspruch 1 in zeitlich naher Abfolge getrennt auf die Pflanzen oder ihre Umgebung einwirken lässt.

## Claims

1. Compounds of the formula (I) 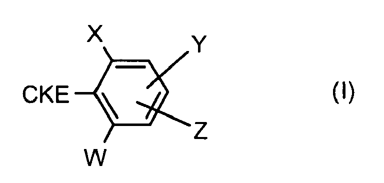 in which
W represents hydrogen, methyl or ethyl,
X represents chlorine, methyl or ethyl,
Y represents hydrogen,
Z represents, in the 4- or 5-position, the radical 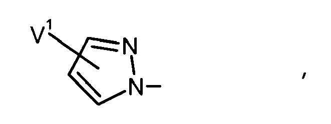
V¹ represents chlorine or methoxy, CKErepresents one of the groups 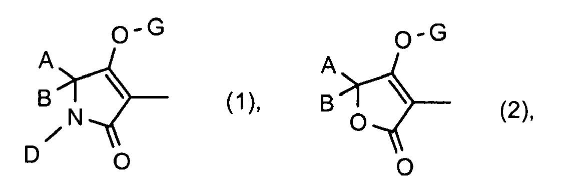 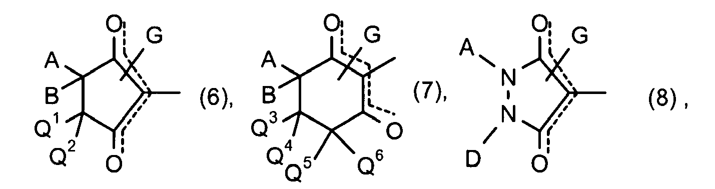
A represents hydrogen, C₁-C₄-alkyl or cyclopropyl,
B represents hydrogen or methyl, or
A, B and the carbon atom to which they are attached represent saturated C₅-C₆-cycloalkyl in which optionally one ring member is replaced by oxygen and which is optionally monosubstituted by methyl or methoxy, with the proviso that in this case Q³ represents hydrogen,
D represents hydrogen,
or
A and D together represent C₃-C₅-alkanediyl in which optionally one carbon atom is replaced by oxygen,
Q¹ represents hydrogen,
Q² represents hydrogen,
Q³ represents methyl,
Q⁴ represents methyl, or
Q³ and Q⁴ together with the carbon to which they are attached represent a saturated C₅-C₆- ring, with the proviso that in this case A represents hydrogen,
Q⁵ represents hydrogen,
Q⁶ represents hydrogen,
G represents hydrogen (a) or represents one of the groups 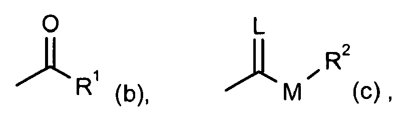
in which
L represents oxygen and
M represents oxygen or sulphur,
R¹ represents C₁-C₆-alkyl or C₁-C₂-alkoxy-C₁- alkyl,
R² represents C₁-C₈-alkyl or benzyl.

2. Process for preparing compounds of the formula (I) according to Claim 1, **characterized in that**, to obtain
(A) compounds of the formula (I-1-a) 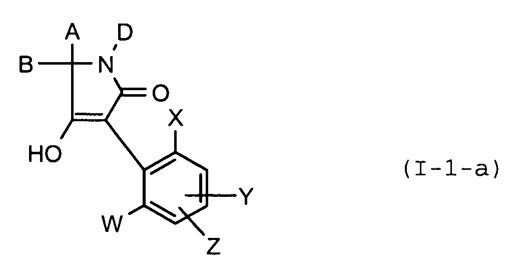 in which
A, B, D, W, X, Y and Z are as defined above, compounds of the formula (II) 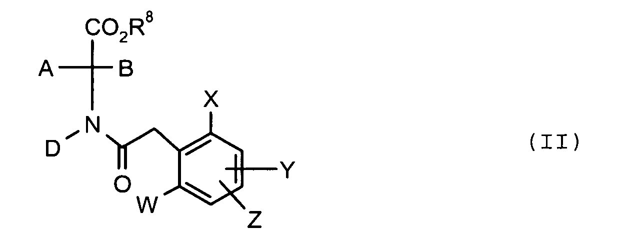 in which
A, B, D, W, X, Y and Z are as defined above, and
R⁸ represents alkyl,
are condensed intramolecularly in the presence of a diluent and in the presence of a base,
(B) compounds of the formula (I-2-a) 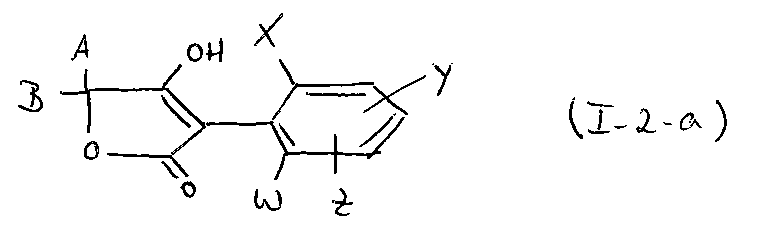 in which
A, B, W, X, Y and Z are as defined above compounds of the formula (III) 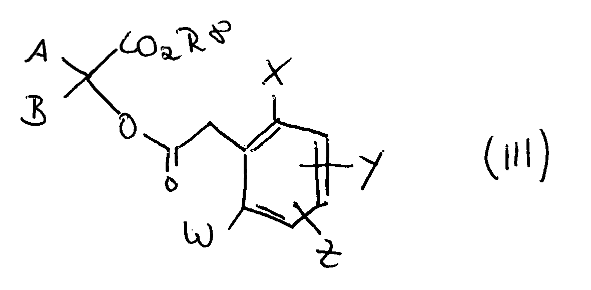 in which
A, B, W, X, Y and Z are as defined above are condensed nitramolecularly in the presence of a diluent and in the presence of a base,
(F) compounds of the formula (I-6-a) 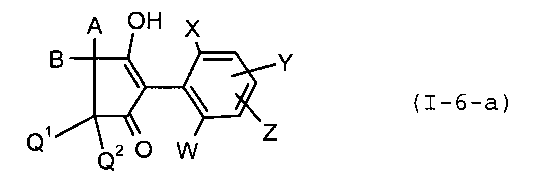 in which
A, B, Q¹, Q², W, X, Y and Z are as defined above,
compounds of the formula (VIII) 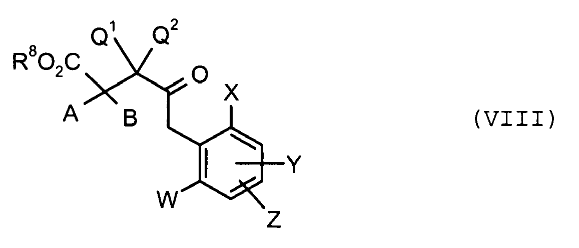 in which
A, B, Q¹, Q², W, X, Y and Z are as defined above, and
R⁸ represents alkyl,
are cyclized intramolecularly, if appropriate in the presence of a diluent and in the presence of a base,
(G) compounds of the formula (I-7-a) 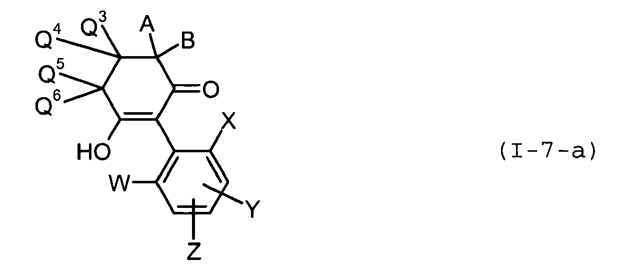 in which
A, B, Q³, Q⁴, Q⁵, Q⁶, W, X, Y and Z are as defined above,
compounds of the formula (IX) 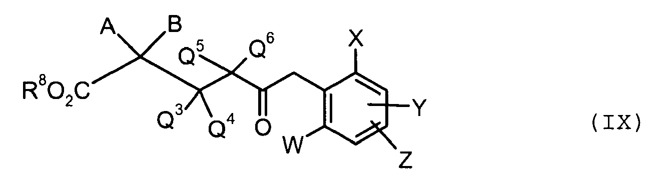 in which
A, B, Q³, Q⁴, Q⁵, Q⁶, W, X, Y and Z are as defined above
and
R⁸ represents alkyl
are condensed intramolecularly in the presence of a diluent and in the presence of a base,
(H) compounds of the formula (I-8-a) 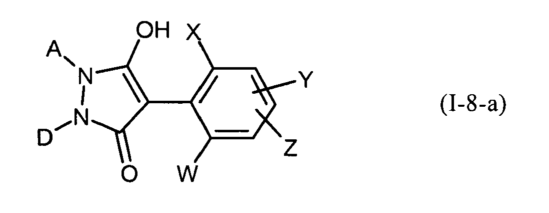 in which
A, D, W, X, Y and Z are as defined above, compounds of the formula (X) 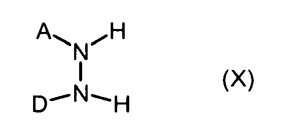 in which
A and D are as defined above
α) are reacted with compounds of the formula (VI) 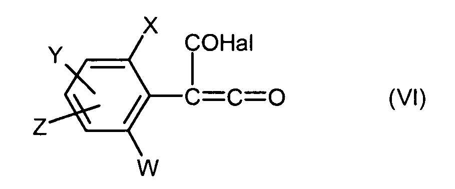 in which
Hal, W, X, Y and Z are as defined above, if appropriate in the presence of a diluent and if appropriate in the presence of an acid acceptor, or
β) are reacted with compounds of the formula (XI) 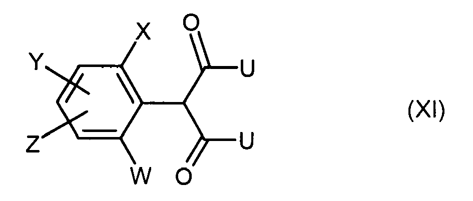 in which
W, X, Y and Z are as defined above and U represents NH₂ or O-R⁸,
if appropriate in the presence of a diluent and if appropriate in the presence of a base, or
γ) are reacted with compounds of the formula (XII) 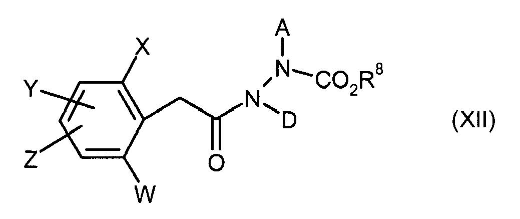 in which
A, D, W, X, Y, Z and R⁸ are as defined above,
if appropriate in the presence of a diluent and if appropriate in the presence of a base,
(I)compounds of the formulae (I-1-b) to (I-8-b) shown above in which A, B, D, Q¹, Q², Q³, Q⁴, Q⁵, Q⁶, R¹, W, X, Y and Z are as defined above, compounds of the formulae (I-1-a) to (I-8-a) shown above in which A, B, D, Q¹, Q², Q³, Q⁴, Q⁵, Q⁶, W, X, Y and Z are as defined above are in each case
(α) reacted with acid halides of the formula (XIII) 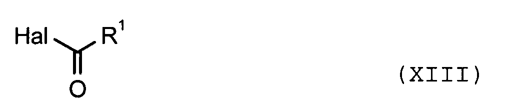 in which
R¹ is as defined above and
Hal represents halogen,
or
(β) reacted with carboxylic anhydrides of the formula (XIV)
R¹-CO-O-CO-R¹ (XIV)
in which
R¹ is as defined above, if appropriate in the presence of a diluent and if appropriate in the presence of an acid binder,
(J)compounds of the formulae (I-1-c) to (I-8-c) shown above in which A, B, D, Q¹, Q², Q³, Q⁴, Q⁵, Q⁶, R², M, W, X, Y and Z are as defined above and L represents oxygen, compounds of the formulae (I-1-a) to (I-8-a) shown above in which A, B, D, Q¹, Q², Q³, Q⁴, Q⁵, Q⁶, W, X, Y and Z are as defined above are in each case
reacted with chloroformic esters or chloroformic thioesters of the formula (XV)
R²-M-CO-Cl (XV)
in which
R² and M are as defined above,
if appropriate in the presence of a diluent and if appropriate in the presence of an acid binder,
(K) compounds of the formulae (I-1-c) to (I-8-c) shown above in which A, B, D, Q¹, Q², Q³, Q⁴, Q⁵, Q⁶, R², M, W, X, Y and Z are as defined above and L represents sulphur, compounds of the formulae (I-1-a) to (I-8-a) shown above in which h A, B, D, Q¹, Q², Q³, Q⁴, Q⁵, Q⁶, W, X, Y and Z are as defined above are in each case
reacted with chloromonothioformic esters or chlorodithioformic esters of the formula (XVI) 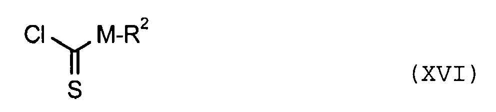 in which
M and R² are as defined above,
if appropriate in the presence of a diluent and if appropriate in the presence of an acid binder,
(P) compounds of the formulae (I-1) to (1-8) shown above in which h A, B, D, Q¹, Q², Q³, Q⁴, Q⁵, Q⁶, W, X, Y and Z are as defined above, compounds of the formulae (I-1') to (I-8') 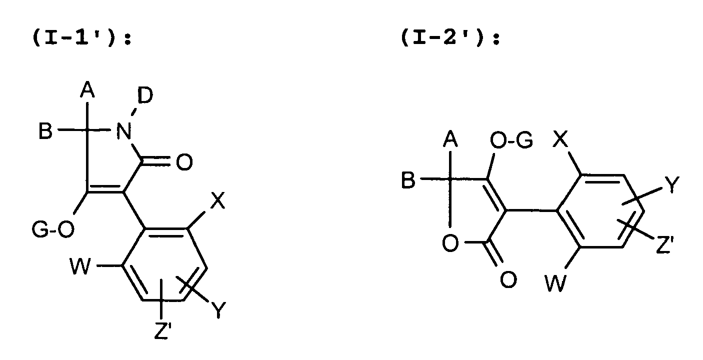 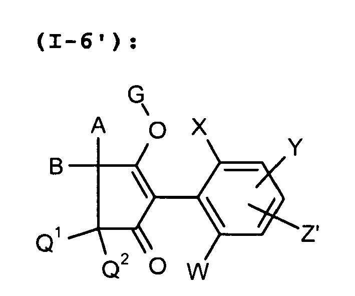 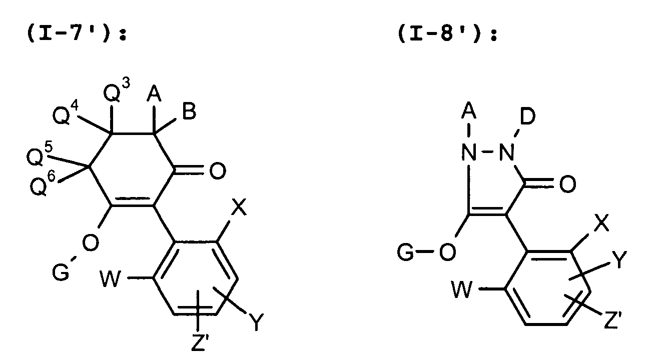 in which
A, B, D, G, Q¹, Q², Q³, Q⁴, Q⁵, Q⁶, W, X and Y are as defined above and
Z' represents chlorine, bromine, iodine,
are reacted with NH heterocycles of the formula (XXIII)
H - Z (XXIII)
in which
Z is as defined above,
in the presence of a solvent, a base and a catalyst, suitable catalysts being, in particular, copper(I) salts.

3. Compounds of the formula (II) 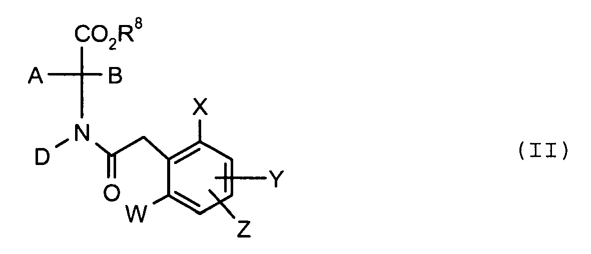 in which
A, B, D, W, X, Y, Z and R⁸ are as defined above.

4. Compounds of the formula (III) 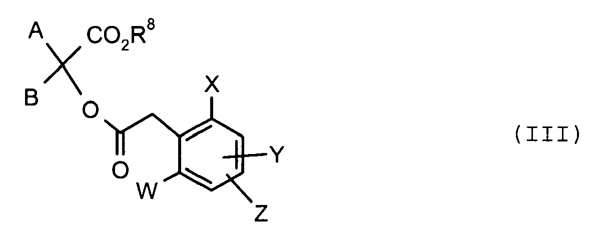 in which
A, B, W, X, Y, Z and R⁸ are as defined above.

5. Compounds of the formula (VI) 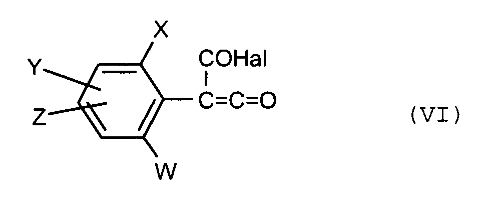 in which
W, X, Y, Z and Hal are as defined above.

6. Compounds of the formula (VIII) 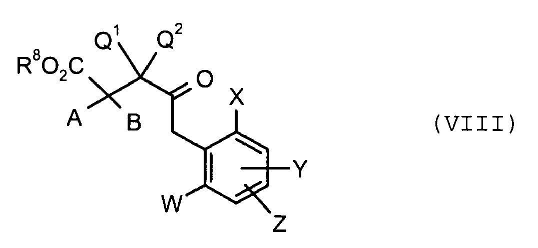 in which
A, B, Q¹, Q², W, X, Y, Z and R⁸ are as defined above.

7. Compounds of the formula (IX) 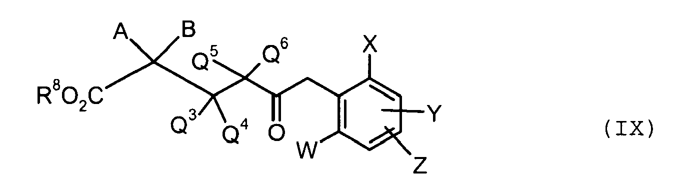 in which
A, B, R⁸, Q³, Q⁴, Q⁵, Q⁶, W, X, Y and Z are as defined above.

8. Compounds of the formula (XI) 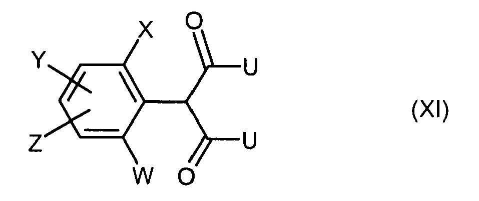 in which
U, W, X, Y and Z are as defined above.

9. Compounds of the formula (XII) 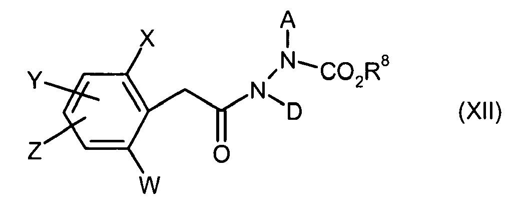 in which
A, D, W, X, Y, Z and R⁸ are as defined above.

10. Compounds of the formula (XXV) 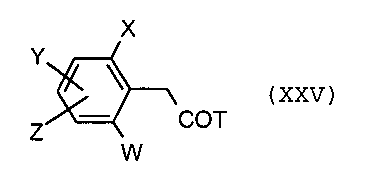 in which
T, W, X, Y and Z are as defined above.

11. Compounds of the formula (XXVI) 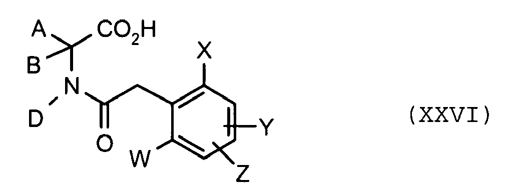 in which
A, B, D, W, X, Y and Z are as defined above.

12. Compounds of the formula (XXX) 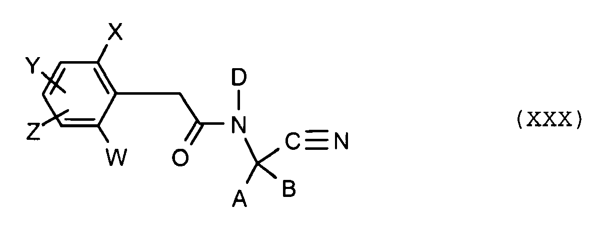 in which
A, B, D, W, X, Y and Z are as defined above.

13. Compounds of the formula (XXVIII) 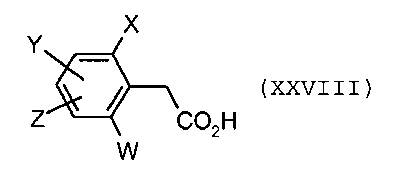 in which
W, X, Y and Z are as defined above.

14. Compounds of the formula (XXXII) 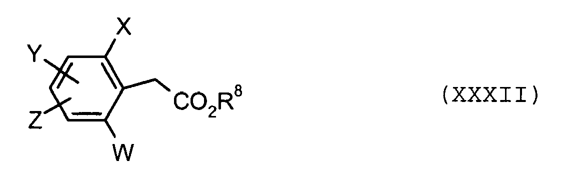 in which
W, X, Y, Z and R⁸ are as defined above.

15. Compounds of the formula (XXXIV) 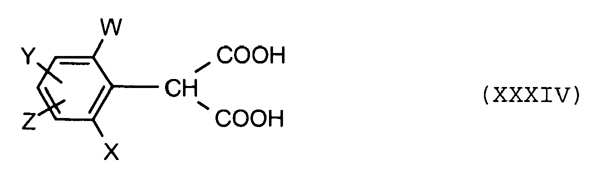 in which
W, X, Y and Z are as defined above.

16. Compounds of the formula (XXXV) 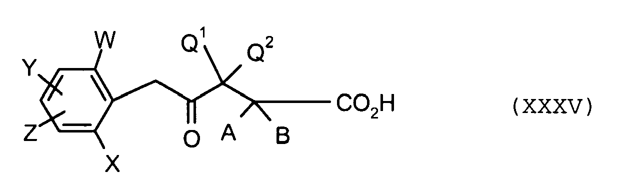 in which
A, B, Q¹, Q², W, X, Y and Z are as defined above.

17. Compounds of the formula (XXXVI) 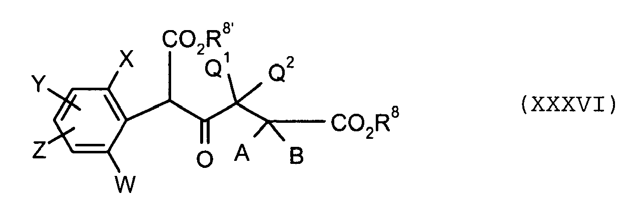 in which
A, B, Q¹, Q², W, X, Y and Z are as defined above and
R⁸ and R^{8'} represent alkyl.

18. Compounds of the formula (XXXIX) 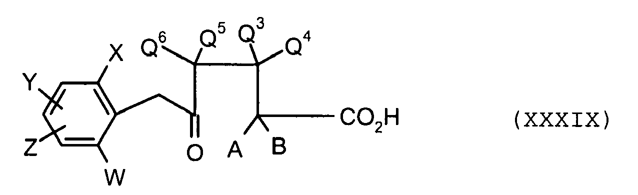 in which
A, B, Q³, Q⁴, Q⁵, Q⁶, W, X, Y and Z are as defined above.

19. Compounds of the formula (XL) 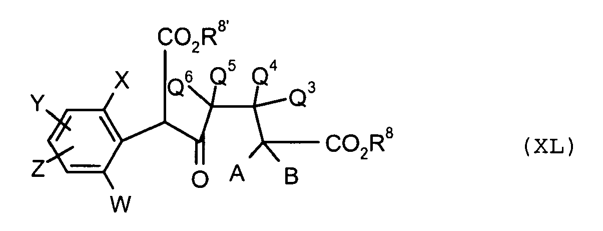 in which
A, B, Q³, Q⁴, Q⁵, Q⁶, W, X, Y and Z are as defined above
and
R⁸ and R^{8'} represent alkyl.

20. Pesticides and/or herbicides and/or fungicides, **characterized in that** they comprise at least one compound of the formula (I) according to Claim 1.

21. Method for the non-therapeutic control of animal pests and/or unwanted vegetation and/or fungi, **characterized in that** compounds of the formula (I) according to Claim 1 are allowed to act on pests and/or their habitat.

22. Use of compounds of the formula (I) according to Claim 1 for the non-therapeutic control of animal pests and/or unwanted vegetation and/or fungi.

23. Process for preparing pesticides and/or herbicides and/or fungicides, **characterized in that** compounds of the formula (I) according to Claim 1 are mixed with extenders and/or surfactants.

24. Use of compounds of the formula (I) according to Claim 1 for preparing pesticides and/or herbicides and/or fungicides.

25. Compositions, comprising an effective amount of an active compound combination comprising, as components,
(a') at least one substituted, cyclic ketoenol of the formula (I), in which CKE, W, X, Y and Z are as defined above
and
(b') at least one compound which improves crop plant tolerance and which is selected from the following group of compounds: cloquintocet-mexyl or mefenpyr-diethyl.

26. Method for controlling unwanted vegetation, **characterized in that** a composition according to Claim 25 is allowed to act on the plants or their habitat.

27. Use of a composition according to Claim 25 for controlling unwanted vegetation.

28. Method for controlling unwanted vegetation, **characterized in that** a compound of the formula (I) according to Claim 25 and the compound which improves crop plant tolerance according to Claim 1 are allowed to act separately within a short interval on the plants or their habitat.

## Revendications

1. Composés de la formule (I) 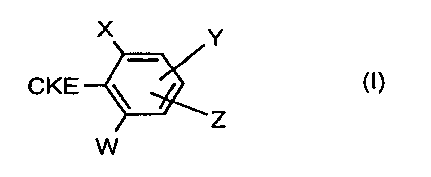 dans laquelle
W représente l'hydrogène, un groupe méthyle ou éthyle,
X représente le chlore, un groupe méthyle ou éthyle,
Y représente l'hydrogène,
Z représente le reste dans la position 4 ou 5 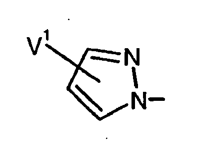
V¹ représente le chlore ou un groupe méthoxy,
CKE représente un des groupes 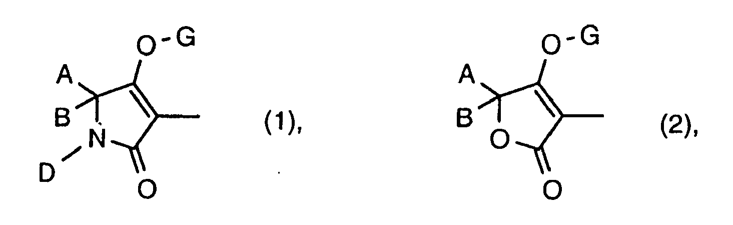 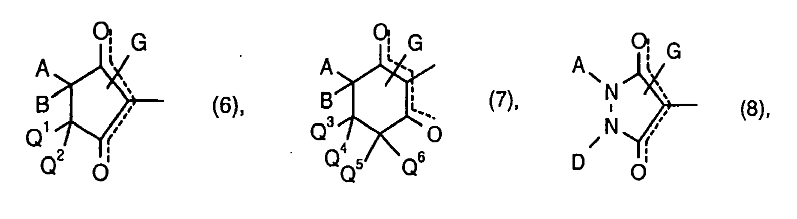
A représente l'hydrogène, un groupe alkyle en C₁-C₄ ou cyclopropyle,
B représente l'hydrogène ou un groupe méthyle, ou
A, B et l'atome de carbone auxquels ils sont liés représentent un groupe cycloalkyle en C₅-C₆ saturé, dans lequel un élément de noyau est éventuellement remplacé par de l'oxygène et lequel est éventuellement substitué une fois par un groupe méthyle ou méthoxy, à condition que Q³ représente alors l'hydrogène,
D représente l'hydrogène,
ou
A et D représentent ensemble un groupe alcanediyle en C₃-C₅, dans lequel un atome de carbone est éventuellement remplacé par de l'oxygène,
Q¹ représente l'hydrogène,
Q² représente l'hydrogène,
Q³ représente le groupe méthyle,
Q⁴ représente le groupe méthyle
ou
Q³ et Q⁴ avec l'atome de carbone auquel ils sont liés, représentent un noyau en C₅-C₆ saturé, à condition que A représente alors l'hydrogène,
Q⁵ représente l'hydrogène,
Q⁶ représente l'hydrogène,
G représente l'hydrogène (a) ou un des groupes 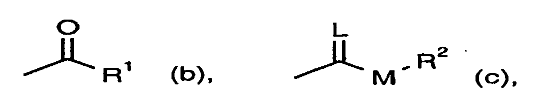 dans lesquels
L représente l'oxygène et
M représente l'oxygène ou le soufre.
R¹ représente un groupe alkyle en C₁-C₆ ou (alcoxy en C₁-C₂)-(alkyle en C₁),
R² représente un groupe alkyle en C₁-C₈ ou benzyle.

2. Procédé pour la préparation de composés de la formule (I) selon la revendication 1, **caractérisée en ce que**
pour l'obtention
(A) de composés de la formule (I-1-a) 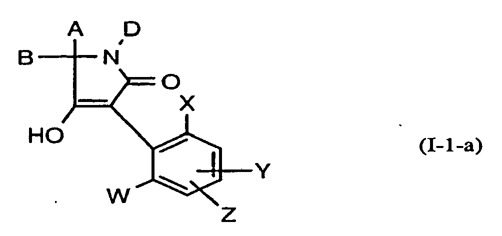 dans laquelle
A, B, D, W, X, Y et Z ont les significations indiquées ci-dessus, des composés de la formule (II) 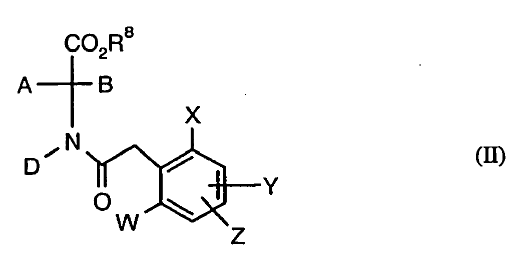 dans laquelle
A, B, D, W, X, Y et Z ont les significations indiquées ci-dessus,
et
R⁸ représente un groupe alkyle, sont condensés par voi intramoléculaire en présence d'un diluant et en présence d'une base,
(B) des composés de la formule (I-2-a) 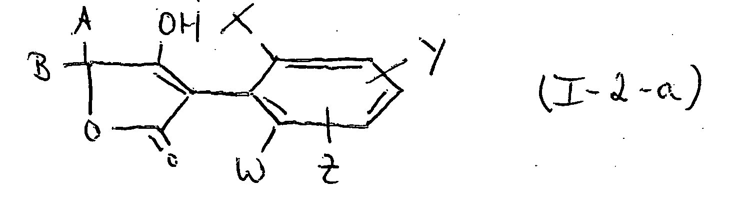 dans laquelle
A, B, W, X, Y et Z ont les significations indiquées ci-dessus,
des composés de la formule (III) 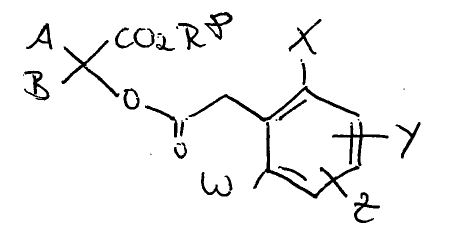 dans laquelle
A, B, W, X, Y, Z et R⁸ ont les significations indiquées ci-dessus,
sont condensés par voi intramoléculaire en présence d'un diluant et en présence d'une base,
pour l'obtention
(F) de composés de la formule (I-6-a) 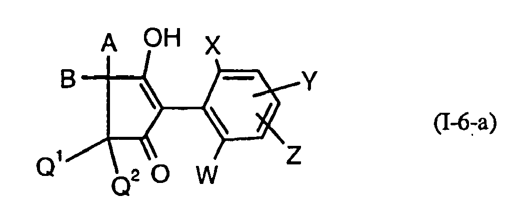 dans laquelle
A, B, Q¹, Q², W, X, Y et Z ont la signification indiquée ci-dessus, des composés de la formule (VIII) 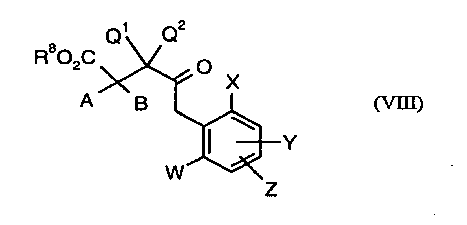 dans laquelle
A, B, Q¹, Q², W, X, Y et Z ont la signification indiquée ci-dessus, et
R⁸ représente un groupe alkyle,
sont cyclisés par voie intramoléculaire éventuellement en présence d'un diluant et en présence d'une base,
pour l'obtention
(G) de composés de la formule (I-7-a) 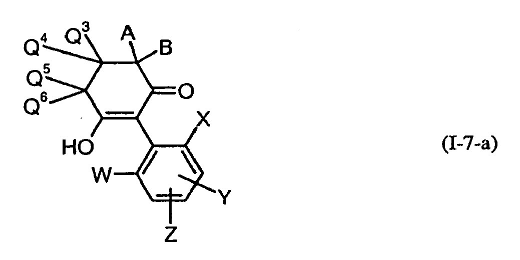 dans laquelle
A, D, Q³, Q⁴, Q⁵, Q⁶, W, X, Y et Z ont la signification indiquée ci-dessus,
des composés de la formule (IX) 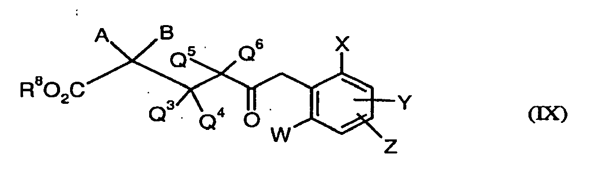 dans laquelle
A, B, Q³, Q⁴, Q⁵, Q⁶, W, X, Y et Z ont la signification indiquée ci-dessus,
et
R⁸ représente un groupe alkyle,
sont condensés par voie intramoléculaire en présence d'un diluant et en présence d'une base,
pour l'obtention
(H) de composés de la formule (I-8-a) 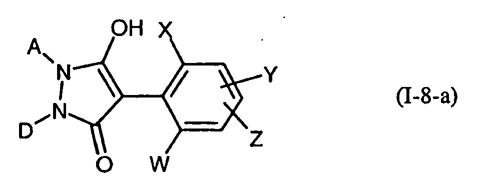 dans laquelle
A, D, W, X, Y et Z ont les significations indiquées ci-dessus, des composés de la formule (X) 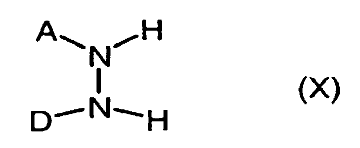 dans laquelle
A et D ont la signification indiquée ci-dessus, réagissent avec
α) des composés de la formule (VI) 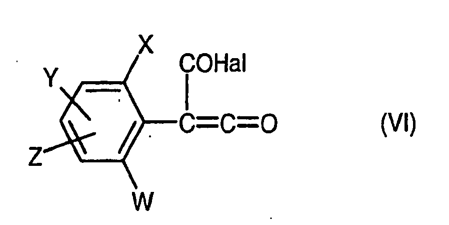 dans laquelle
Hal, W, X, Y et Z ont les significations indiquées ci-dessus,
éventuellement en présence d'un diluant et éventuellement en présence d'un accepteur d'acide, ou
β) avec des composés de la formule (XI) 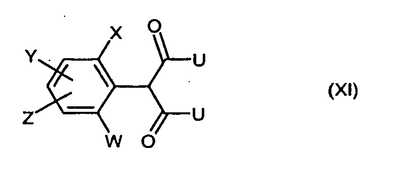 dans laquelle
W, X, Y et Z ont la signification indiquée ci-dessus, et U représente NH₂ ou O-R⁸,
éventuellement en présence d'un diluant et éventuellement en présence d'une base, ou
γ) avec des composés de la formule (XII) 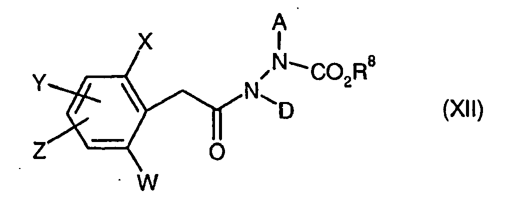 dans laquelle
A, D, W, X, Y, Z et R⁸ ont la signification indiquée ci-dessus, éventuellement en présence d'un diluant et éventuellement en présence d'une base,
pour l'obtention
(I) de composés des formules (I-1-b) à (I-8-b) représentées ci-dessus, dans lesquelles A, B, D, Q¹, Q², Q³, Q⁴, Q⁵, Q⁶, R¹, W, X, Y et Z ont les significations indiquées ci-dessus, des composés des formules (I-1-a) à (I-8-a) représentées ci-dessus, dans lesquelles A, B, D, Q¹, Q², Q³, Q⁴, Q⁵, Q⁶, W, X, Y et Z ont les significations indiquées ci-dessus, réagissent à chaque fois
(α) avec des halogénures d'acides de la formule (XIII) 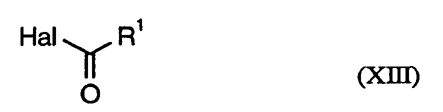 dans laquelle
R¹ a la signification indiquée ci-dessus et
Hal représente un halogène
ou
(β) avec des anhydrides d'acides carboxyliques de la formule (XIV)
R¹-CO-O-CO-R¹ (XIV)
dans laquelle
R¹ a la signification indiquée ci-dessus,
éventuellement en présence d'un diluant et éventuellement en présence d'un liant d'acide,
pour l'obtention
(J) de composés des formules (I-1-c) à (I-8-c) représentées ci-dessus, dans lesquelles A, B, D, Q¹, Q², Q³, Q⁴, Q⁵, Q⁶, R², M, W, X, Y et Z ont les significations indiquées ci-dessus et L représente l'oxygène, des composés des formules (I-1-a) à (I-8-a) représentées ci-dessus, dans lesquelles A, B, D, Q¹, Q², Q³, Q⁴, Q⁵, Q⁶, W, X, Y et Z ont les significations indiquées ci-dessus, réagissent à chaque fois
avec des esters d'acide chloroformique ou des thioesters d'acide chloroformique de la formule (XV)
R²-M-CO-Cl (XV)
dans laquelle
R² et M ont les significations indiquées ci-dessus, éventuellement en présence d'un diluant et éventuellement en présence d'un liant d'acide,
pour l'obtention
(K) de composés des formules (I-1-c) à (I-8-c) représentées ci-dessus, dans lesquelles A, B, D, Q¹, Q², Q³, Q⁴, Q⁵, Q⁶, R², M, W, X, Y et Z ont les significations indiquées ci-dessus et L représente le soufre, des composés des formules (I-1-a) à (I-8-a) représentées ci-dessus, dans lesquelles A, B, D, Q¹, Q², Q³, Q⁴, Q⁵, Q⁶, W, X, Y et Z ont les significations indiquées ci-dessus, réagissent à chaque fois
avec des esters d'acide chloromonothioformique ou avec des esters d'acide chlorodithioformique de la formule (XVI) 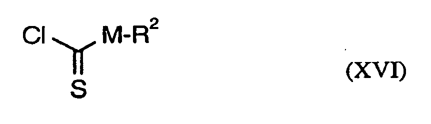 dans laquelle
M et R² ont les significations indiquées ci-dessus, éventuellement en présence d'un diluant et éventuellement en présence d'un liant d'acide,
pour l'obtention
(P) de composés des formules (I-1) à (I-8) représentées ci-dessus, dans lesquelles A, B, D, Q¹, Q², Q³, Q⁴, Q⁵, Q⁶, W, X, Y et Z ont la signification indiquée ci-dessus, des composés des formules (I-1') à (I-8') 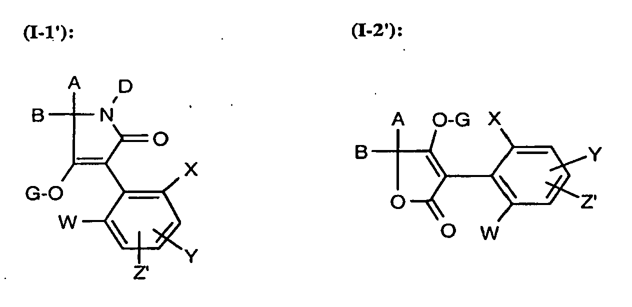 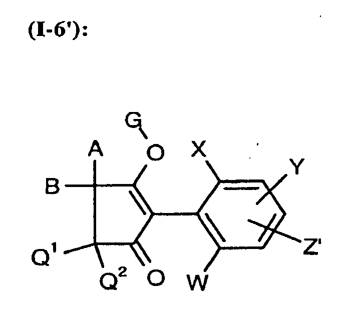 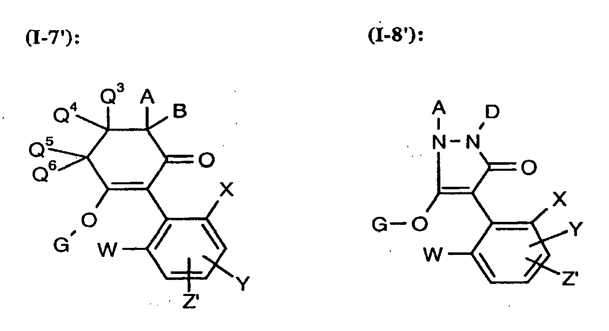 dans lesquelles
A, B, D, G, Q¹, Q², Q³, Q⁴, Q⁵, Q⁶, W, X et Y ont la signification indiquée ci-dessus et
Z' représente le chlore, le brome, l'iode,
réagissent
avec des NH-hétérocycles de la formule (XXIII)
H-Z (XXIII)
dans laquelle
Z a la signification indiquée ci-dessus et
en présence d'un solvant, d'une base et d'un catalyseur, des sels du cuivre 1 intervenant en particulier comme catalyseur.

3. Composés de la formule (II) 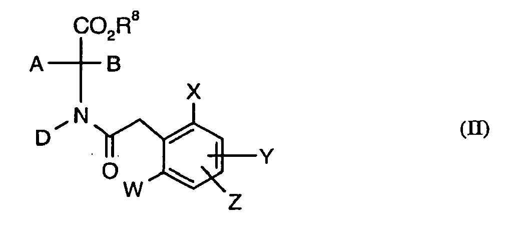 dans laquelle
A, B, D, W, X, Y, Z et R⁸ ont les significations indiquées ci-dessus.

4. Composés de la formule (III) 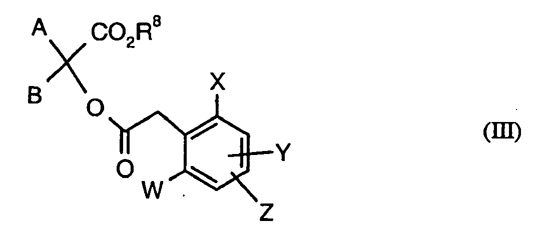 dans laquelle
A, B, W, X, Y, Z et R⁸ ont les significations indiquées ci-dessus.

5. Composés de la formule (VI) 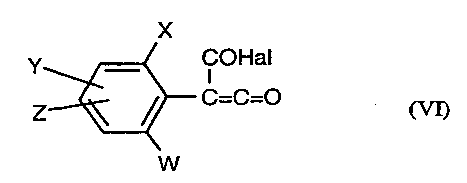 dans laquelle
W, X, Y, Z et Hal ont les significations indiquées ci-dessus.

6. Composés de la formule (VIII) 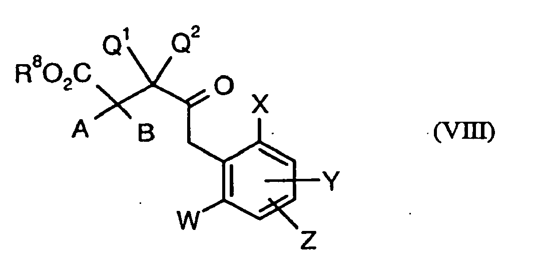 dans laquelle
A, B, Q¹, Q², W, X, Y, Z et R⁸ ont la signification indiquée ci-dessus.

7. Composés de la formule (IX) 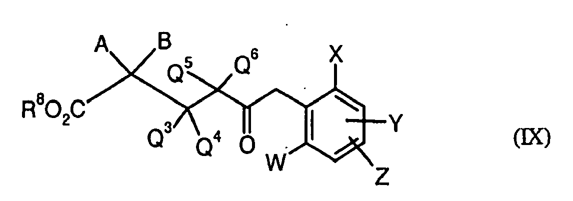 dans laquelle
A, B, R⁸, Q³, Q⁴, Q⁵, Q⁶, W, X, Y et Z ont la signification indiquée ci-dessus.

8. Composés de la formule (XI) 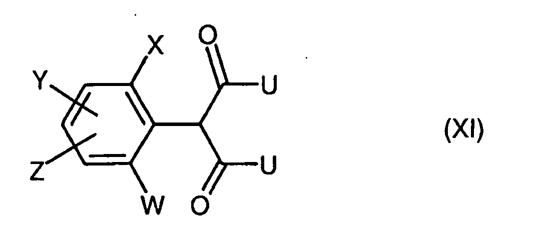 dans laquelle
U, W, X, Y et Z ont la signification indiquée ci-dessus.

9. Composés de la formule (XII) 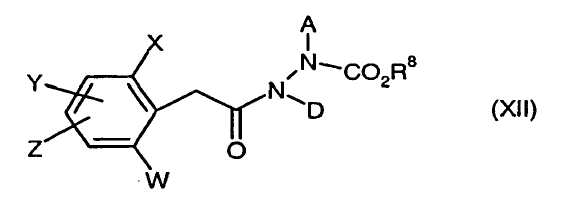 dans laquelle
A, D, W, X, Y, Z et R⁸ ont la signification indiquée ci-dessus.

10. Composés de la formule (XXV) 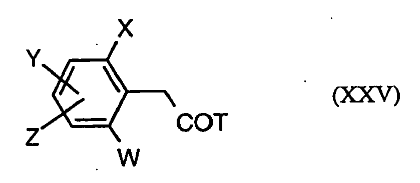 dans laquelle
T, W, X, Y et Z ont les significations indiquées ci-dessus.

11. Composés de la formule (XXVI) 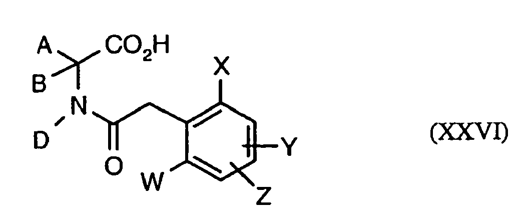 dans laquelle
A, B, D, W, X, Y et Z ont les significations indiquées ci-dessus.

12. Composés de la formule (XXX) 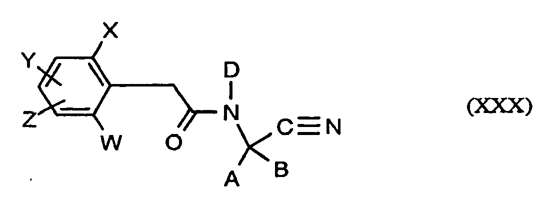 dans laquelle
A, B, D, W, X, Y et Z ont les significations indiquées ci-dessus.

13. Composés de la formule (XXVIII) 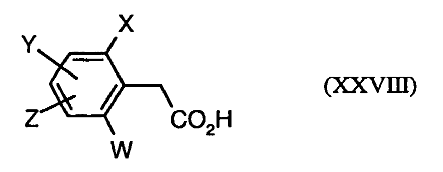 dans laquelle
W, X, Y et Z ont la signification indiquée ci-dessus.

14. Composés de la formule (XXXII) 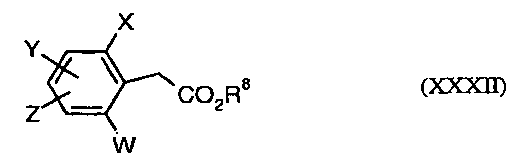 dans laquelle
W, X, Y, Z et R⁸ ont la signification indiquée ci-dessus.

15. Composés de la formule (XXXIV) 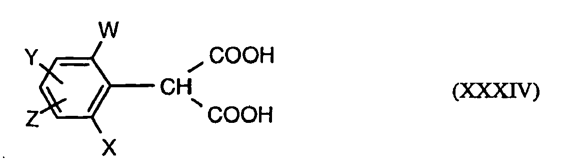 dans laquelle
W, X, Y et Z ont les significations indiquées ci-dessus.

16. Composés de la formule (XXXV) 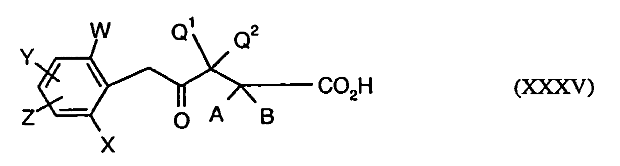 dans laquelle
A, B, Q¹, Q², W, X, Y et Z ont la signification indiquée ci-dessus.

17. Composés de la formule (XXXVI) 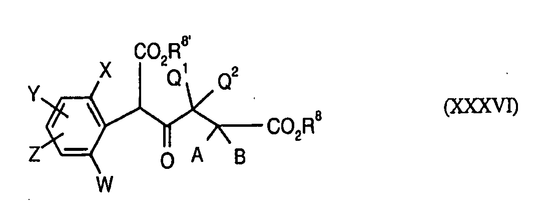 dans laquelle
A, B, Q¹, Q², W, X, Y et Z ont la signification indiquée ci-dessus et
R⁸ et R^{8'} représentent un groupe alkyle.

18. Composés de la formule (XXXIX) 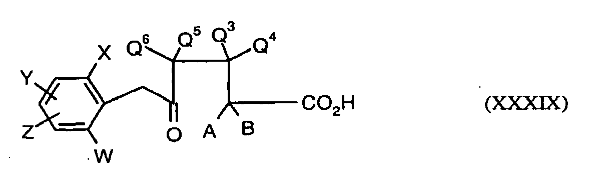 dans laquelle
A, B, Q³, Q⁴, Q⁵, Q⁶, W, X, Y et Z ont la signification indiquée ci-dessus.

19. Composés de la formule (XL) 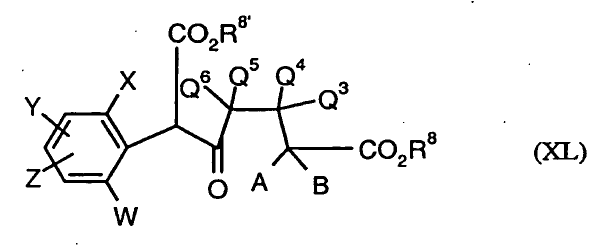 dans laquelle
A, B, Q³, Q⁴, Q⁵, Q⁶, W, X, Y et Z ont la signification indiquée ci-dessus
et
R⁸ et R^{8'} représentent un groupe alkyle.

20. Insecticides et/ou herbicides et/ou fongicides, **caractérisés par** une teneur en au moins un composé de la formule (I) selon la revendication 1.

21. Procédé pour une lutte non thérapeutique contre des parasites animaux et/ou une végétation non souhaitée et/ou des champignons, **caractérisé en ce que** l'on laisse agir des composés de la formule (I) selon la revendication 1 sur des parasites et/ou leur biotope.

22. Utilisation de composés de la formule (I) selon la revendication 1 pour une lutte non thérapeutique contre des parasites animaux et/ou une végétation non souhaitée et/ou des champignons.

23. Procédé pour la préparation d'insecticides et/ou d'herbicides et/ou de fongicides, **caractérisé en ce que** l'on mélange des composés de la formule (I) selon la revendication 1 avec des diluants et/ou des tensioactifs.

24. Utilisation de composés de la formule (I) selon la revendication 1 pour la préparation d'insecticides et/ou d'herbicides et/ou de fongicides.

25. Agent contenant une quantité active d'une combinaison de principes actifs comprenant comme constituants
(a') au moins un cétoénol cyclique substitué de la formule (I), dans laquelle CKE, W, X, Y et Z ont la signification indiquée ci-dessus
et
(b') au moins un composé améliorant la compatibilité aux plantes cultivées du groupe suivant des composés :
cloquintocet-mexyl ou mefenpyr-diéthyle.

26. Procédé pour la lutte contre une végétation non souhaitée, **caractérisé en ce que** l'on laisse agir un agent selon la revendication 25 sur les plantes ou leur environnement.

27. Utilisation d'un agent selon la revendication 25 pour la lutte contre une végétation non souhaitée.

28. Procédé pour lutter contre une végétation non souhaitée, **caractérisé en ce que** l'on laisse agir séparément un composé de la formule (I) selon la revendication 25 et le composé améliorant la compatibilité aux plantes cultivées selon la revendication 1 successivement de manière rapprochée dans le temps sur les plantes ou leur environnement.
